# EUROPEAN PATENT SPECIFICATION

(11) **EP 3 555 068 B1**
(45) Date of publication and mention of the grant of the patent: **02.12.2020**
(21) Application number: 17818632.6
(22) Date of filing: 11.12.2017
(51) Int. Cl.: C07D 403/12, C07D 403/10, A61P 11/00, A61P 9/04, A61K 31/551

(54) **3-OXO-1,4-DIAZEPINYLE COMPOUNDS AS NRF2 ACTIVATORS**
3-OXO-1,4-DIAZEPINYL-VERBINDUNGEN ALS NRF2 AKTIVATOREN
COMPOSÉS DE 3-OXO-1,4-DIAZEPINYLE COMME ACTIVATEURS DE NRF2

(30) Priority: 14.12.2016 US 201662433965 P
(43) Date of publication of application: 23.10.2019
(73) Proprietor: GlaxoSmithKline Intellectual Property Development Limited, Brentford Middlesex TW8 9GS (GB)
(72) Inventor: BOEHM, Jeffrey Charles, King Of Prussia, Pennsylvania 19406 (US); DAVIS, Roderick S., King Of Prussia, Pennsylvania 19406 (US); GOODWIN, Nicole Cathleen, King Of Prussia, Pennsylvania 19406 (US); KERNS, Jeffrey K., King Of Prussia, Pennsylvania 19406 (US); LI, Tindy, King Of Prussia, Pennsylvania 19406 (US); YAN, Hongxing, King Of Prussia, Pennsylvania 19406 (US)
(74) Representative: Learoyd, Stephanie Anne
(86) International application number: PCT/IB2017/057806
(87) International publication number: WO 2018/109648

(56) References cited:
- WO-A1-2015/092713
- WO-A1-2016/202253

## Description

### FIELD OF THE INVENTION

The present invention relates to bisaryl lactam compounds, methods of making them, pharmaceutical compositions containing them and their use as NRF2 activators.

### BACKGROUND OF THE INVENTION

NRF2 (NF-E2 related factor 2) is a member of the cap-n-collar family of transcription factors containing a characteristic basic-leucine zipper motif. Under basal conditions, NRF2 levels are tightly controlled by the cytosolic actin-bound repressor, KEAP1 (Kelch-like ECH associating protein 1), which binds to NRF2 and targets it for ubiquitylation and proteasomal degradation via the Cul3-based E3-ubiquitin ligase complex. Under conditions of oxidative stress, DJ1 (PARK7) is activated and stabilizes NRF2 protein by preventing NRF2 from interacting with KEAP1. Also, modification of reactive cysteines on KEAP1 can cause a conformational change in KEAP1 that alters NRF2 binding and promotes NRF2 stabilization. Thus, the levels of NRF2 in the cell are usually kept low in normal conditions but the system is designed to respond quickly to environmental stress by increasing NRF2 levels and thus downstream NRF2 activity.

Inappropriately low NRF2 activity in the face of on-going oxidative stress appears to be a pathological mechanism underlying chronic obstructive pulmonary disease (COPD). Yamada, K., et al. BMC Pulmonary Medicine, 2016, 16: 27. This may be a result of an altered equilibrium between NRF2 activators with both inappropriate lack of positive activators such as DJ1, and overabundance of negative activators such as Keap1 and Bach1. Therefore, restoration of NRF2 activity in the lungs of COPD patients should result in repair of the imbalance and mitigation of deleterious processes such as apoptosis of structural cells (including alveolar epithelial and endothelial cells) and inflammation. The results of these effects would be enhanced cytoprotection, preservation of lung structure, and structural repair in the COPD lung, thus slowing disease progression. Therefore, NRF2 activators may treat COPD (Boutten, A., et al. 2011. Trends Mol. Med. 17:363-371) and other respiratory diseases, including asthma, Acute Lung Injury (ALI) (Cho, H.Y., and Kleeberger, S.R., 2015, Arch Toxicol. 89:1931-1957; Zhao, H. et al., 2017, Am J Physiol Lung Clee MolPhysiol 312:L155-L162, first published November 18, 2016; doi:10.1152/ajplung.00449.2016), Acute Respiratory Distress Syndrome (ARDS) and pulmonary fibrosis (Cho, H.Y., and Kleeberger, S.R. 2010. Toxicol. Appl. Pharmacol. 244:43-56).

The therapeutic potential of an NRF2 activator is exemplified in pulmonary macrophages from COPD patients where NRF2 pathway appears maladaptive. These cells have impaired bacterial phagocytosis compared with similar cells from control patients, and this effect is reversed by the addition of NRF2 activators in vitro. Therefore, in addition to the effects mentioned above, restoration of appropriate NRF2 activity could also rescue COPD exacerbations by reducing lung infection.

This is demonstrated by the NRF2 activator, Sulforaphane, which increases the expression of Macrophage Receptor with Collagenous structure (MARCO) by COPD macrophages and alveolar macrophages from cigarette smoke-exposed mice, thereby improving in these cells bacterial phagocytosis (*Pseudomonas aeruginosa,* non-typable *Haemophilus influenzae*) and bacterial clearance both ex *vivo* and *in vivo.* (Harvey, C. J., et al. 2011. Sci. Transl. Med. 3:78ra32).

The therapeutic potential of targeting NRF2 in the lung is not limited to COPD. Rather, targeting the NRF2 pathway could provide treatments for other human lung and respiratory diseases that exhibit oxidative stress components such as chronic asthma and acute asthma, lung disease secondary to environmental exposures including but not limited to ozone, diesel exhaust and occupational exposures, fibrosis, acute lung infection (e.g., viral (Noah, T.L. et al. 2014. PLoS ONE 9(6): e98671), bacterial or fungal), chronic lung infection, α1 antitrypsin disease, ALI, ARDS and cystic fibrosis (CF, Chen, J. et al. 2008. PLoS One. 2008;3(10):e3367).

A therapy that targets the NRF2 pathway also has many potential uses outside the lung and respiratory system. Many of the diseases for which an NRF2 activator may be useful are autoimmune diseases (psoriasis, IBD, MS), suggesting that an NRF2 activator may be useful in autoimmune diseases in general.

In the clinic, a drug targeting the NRF2 pathway (bardoxolone methyl) has shown efficacy in diabetic patients with diabetic nephropathy / chronic kidney disease (CKD) (Aleksunes, L.M., et al. 2010. J. Pharmacol. Exp. Ther. 335:2-12), though phase III trials with this drug in patients with the most severe stage of CKD were terminated. Furthermore, there is evidence to suspect that such a therapy would be effective in sepsis-induced acute kidney injury, other acute kidney injury (AKI) (Shelton, L.M., et al. 2013. Kidney International. Jun 19. doi: 10.1038/ki.2013.248.), and kidney disease or malfunction seen during kidney transplantation.

In the cardiac area, bardoxolone methyl is currently under investigation in patients 30 with Pulmonary Arterial Hypertension and so a drug targeting NRF2 by other mechanisms may also be useful in this disease area. Oxidative stress is increased in the diseased myocardium, resulting in accumulation of reactive oxygen species (ROS) which impairs cardiac function [Circ (1987) 76(2); 458-468] and increases susceptibility to arrhythmia [J of Mol & Cell Cardio (1991) 23(8); 899-918] by a direct toxic effect of increased necrosis and apoptosis [Circ Res (2000) 87(12); 1172-1179]. In a mouse model of pressure overload (TAC), NRF2 gene and protein expression is increased during the early stage of cardiac adaptive hypertrophy, but decreased in the later stage of maladaptive cardiac remodeling associated with systolic dysfunction [Arterioscler Thromb Vasc Biol (2009) 29(11); 1843- 5 1850; PLOS ONE (2012) 7(9); e44899]. In addition, NRF2 activation has been shown to suppress myocardial oxidative stress as well as cardiac apoptosis, ALI, ARDS, fibrosis, hypertrophy, and dysfunction in mouse models of pressure overload [Arterioscler Thromb Vasc Biol (2009) 29(11**)**; J of Mol & Cell Cardio (2014) 72; 305-315; and 1843-1850; PLOS ONE (2012) 7(9); e44899]. NRF2 activation has also been shown to protect against cardiac I/R injury in mice 10 [Circ Res (2009) 105(4); 365-374; J of Mol & Cell Cardio (2010) 49(4); 576-586] and reduce myocardial oxidative damage following cardiac I/R injury in rat. Therefore, a drug targeting NRF2 by other mechanisms may be useful in a variety of cardiovascular diseases including but not limited to atherosclerosis, hypertension, and heart failure (Oxidative Medicine and Cellular Longevity Volume 2013 (2013), Article ID 104308, 10 pages), acute coronary 15 syndrome, myocardial infarction, myocardial repair, cardiac remodeling, cardiac arrhythmias, heart failure with preserved ejection fraction, heart failure with reduced ejection fraction and diabetic cardiomyopathy.

A drug activating the NRF2 pathway could also be useful for treatment of several neurodegenerative diseases including Parkinson's disease (PD), Alzheimer's disease (AD), amyotrophic lateral sclerosis (ALS) (Brain Res. 2012 Mar 29;1446:109-18. 2011.12.064. Epub 2012 Jan 12.) and multiple sclerosis (MS). Multiple *in vivo* models have shown that NRF2 KO mice are more sensitive to neurotoxic insults than their wild-type counterparts. Treatment of rats with the NRF2 activator tert-butylhydroquinone (tBHQ) reduced cortical damage in rats in a cerebral ischemia-reperfusion model, and cortical glutathione levels were increased in NRF2 wild-type but not KO mice after administration of tBHQ (Shih, A.Y.,et al. 2005. J. Neurosci. 25: 10321-10335). Tecfidera™ (dimethyl fumarate), which activates NRF2 among other targets, is approved in the U.S. to treat relapsing-remitting multiple sclerosis (MS). Activation of NRF2 may also help treat cases of Friedreich's Ataxia, where increased sensitivity to oxidative stress and impaired NRF2 activation has been reported (Paupe V., et al, 2009. PLoS One; 4(1):e4253. Omaveloxolone (RTA-408) is also in clinical trials for Friedreich's Ataxia.

There is preclinical evidence of the specific protective role of the NRF2 pathway in models of inflammatory bowel disease (IBD, Crohn's Disease and Ulcerative Colitis) and/or colon cancer (Khor, T.O., et al 2008. Cancer Prev. Res. (Phila) 1:187-191).

Age-related macular degeneration (AMD) is a common cause of vision loss in people over the age of 50. Cigarette smoking is a major risk factor for the development of non-neovascular (dry) AMD and perhaps also neovascular (wet) AMD. Findings *in vitro* and in preclinical species support the notion that the NRF2 pathway is involved in the anti-oxidant response of retinal epithelial cells and modulation of inflammation in pre-clinical models of eye injury (Schimel, et al. 2011. Am. J. Pathol. 178:2032-2043). Fuchs Endothelial Corneal Dystrophy (FECD) is a progressive, blinding disease characterized by corneal endothelial cells apoptosis. It is a disease of aging and increased oxidative stress related to low levels of NRF2 expression and/or function (Bitar, M.S., et al. 2012. Invest Ophthalmol. Vis. Sci. August 24, 2012 vol. 53 no. 9 5806-5813). In addition, an NRF2 activator may be useful in uveitis or other inflammatory eye condtions.

Non-alcoholic steatohepatitis (NASH) is a disease of fat deposition, inflammation, and damage in the liver that occurs in patients who drink little or no alcohol. In pre-clinical models, development of NASH is greatly accelerated in KO mice lacking NRF2 when challenged with a methionine- and choline-deficient diet (Chowdhry S., et al. 2010. Free Rad. Biol. & Med. 48:357-371). Administration of the NRF2 activators oltipraz and NK-252 in rats on a choline-deficient L-amino acid-defined diet significantly attenuated progression of histologic abnormalities, especially hepatic fibrosis (Shimozono R. et al. 2012. Molecular Pharmacology. 84:62-70). Other liver diseases that may be amenable to NRF2 modulation are toxin-induced liver disease (e.g., acetaminophen-induced hepatic disease), viral hepatitis, and cirrhosis (Oxidative Medicine and Cellular Longevity Volume 2013 (2013), Article ID 763257, 9 page).

Recent studies have also begun to elucidate the role of ROS in skin diseases such as psoriasis. A study in psoriasis patients showed an increase in serum malondialdehyde and nitric oxide end products and a decrease in erythrocyte-superoxide dismutase activity, catalase activity, and total antioxidant status that correlated in each case with disease severity index (Dipali P.K., et al. Indian J Clin Biochem. 2010 October; 25(4): 388-392). Also, an NRF2 activator may be useful in treating the dermatitis/topical effects of radiation (Schäfer, M. et al. 2010. Genes & Devl. 24:1045-1058), and the immunosuppression due to radiation exposure (Kim, J.H. et al., J. Clin. Invest. 2014 Feb 3; 124(2):730-41).

There are also data suggesting that an NRF2 activator may be beneficial in preeclampsia, a disease that occurs in 2-5% of pregnancies and involves hypertension and proteinuria (Annals of Anatomy - Anatomischer Anzeiger Volume 196, Issue 5, September 2014, Pages 268-277).

Preclinical data has shown that compounds with NRF2 activating activity are better at reversing high altitude-induced damage than compounds without NRF2 activity, using animal and cellular models of Acute Mountain Sickness (Lisk C. et al, 2013, Free Radic Biol Med. Oct 2013; 63: 264-273.) WO2015092713 discloses NRF2 regulators.

### SUMMARY OF THE INVENTION

In one aspect, this invention provides for bisaryl lactam analogs, or a salt, particularly a pharmaceutically acceptable salt thereof, and pharmaceutical compositions containing them. In particular, the compounds of this invention include a compound of Formula (I): 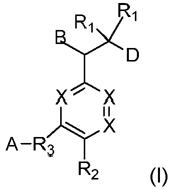 wherein:
B is benzotriazolyl, phenyl, triazolopyridinyl, -O(CH₂)-triazolyl or -(CH₂)₂-triazolyl, wherein each of the benzotriazolyl, phenyl, triazolopyridinyl, -O(CH₂)-triazolyl or -(CH₂)₂-triazolyl is unsubstituted or substituted by 1, 2, or 3 substituents independently selected from -C₁₋₃alkyl, -O-C₁₋₃alkyl, CN, -(CH₂)₂-O-(CH₂)₂-OR₄ and halo;
D is -C(O)OH, -C(O)NR₆R₇, -C(O)NHSO₂CH₃, -SO₂NHC(O)CH₃, 5-(trifluoromethyl)-4H-1,2,4-triazol-2-yl, -NR₆-C(O)-R₇, -NR₆-C(O)-NR₆R₇; -NR₆-C(O)-O-R₇ or tetrazolyl;
R₁ is independently hydrogen, -OH, -C₁₋₃alkyl, - C₁₋₃alkylOR', F, -C₃₋₆spirocycloalkyl, oxetane, or the two R₁ groups together with the carbon to which they are attached form a cyclopropyl group;
R' is hydrogen or -C₁₋₃alkyl;
R₂ is hydrogen, -C₁₋₄alkyl, -CF₃, or halo;
R₃ is -(CH₂)ₘ;
R₄ is hydrogen or -C₁₋₃alkyl;
or, when R₂ is -C₁₋₄alkyl, R₃ is -(CH₂)ₘ-, and m is 2 or 3, R₂ and R₃ together form a cycloalkyl ring fused to the phenyl ring to which they are attached;
A is 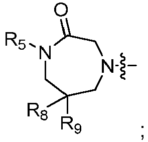
R₅ is hydrogen, -C₁₋₅alkyl or -(CH₂)ₙ-C₃₋₅cycloalkyl;
R₆ is hydrogen or -C₁₋₄alkyl;
R₇ is hydrogen, -C₁₋₅alkyl, -C₃₋₇cycloalkyl, -C₄₋₈heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-SO₂C₁₋₃alkyl, -C₁₋₃alkyl-C₄₋₈heterocycloalkyl, -C₁₋₃alkyl-C(O)NR₄R₆, or heteroaryl, wherein each of -C₁₋₅ alkyl, -C₃₋₇ cycloalkyl, -C₄₋₇ heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-C(O)NR₄R₆, or heteroaryl is unsubstituted or substituted by one or two substituents selected from -OH, -CO₂H,
-C(O)NR₄R₆,-C(O)OR₄, -N-C(O)-C₁₋₃alkyl, F, -CN, -CH-F₂, -CF₃, -(CH₂)ₙ-O-(CH₂)ₘ-CH₃, and-C₃₋₇cycloalkyl, a 5-6-membered heteroaryl ring containing 1, 2 or 3 heteroatoms selected from O, N and S;
or R₆ and R₇ together with the nitrogen atom to which they are attached form a 5-8-membered heterocyclic ring, an 8-11-membered bicyclic heterocyclic ring or a 9- 10-membered bridged bicyclic heterocyclic ring, wherein each 5-8-membered ring, 8-11-membered bicyclic ring, or 9-10-membered bridged bicyclic ring optionally includes one - C(O) or one -S(O)₂, and wherein each 5-8-membered ring, 8-11 -membered bicyclic ring, or 9-10-membered bridged bicyclic ring optionally contains one, two or three oxygen ring atoms, one, two or three sulfur ring atoms or one, two or three nitrogen ring atoms, and wherein each 5-8-membered ring, 8-11-membered bicyclic ring, or 9-10-membered bridged bicyclic ring is unsubstituted or substituted by one, two or three substituents independently selected from -C₁₋₅alkyl, -C₃₋₇cycloalkyl, -C₄₋₇heterocycloalkyl, -(CH₂)phenyl, halogen,-NR₃R₄, -CHF₂, -CF₃, and -(CH₂)ₙ-O-(CH₂)ₘ-CH₃;
R₈ is hydrogen or -C₁₋₄alkyl;
R₉ is hydrogen or -C₁₋₄alkyl;
m is 1, 2 or 3;
n is 0, 1, 2 or 3; and
X is independently CH or N;
or a pharmaceutically acceptable salt thereof.

In a second aspect, this invention provides for the use of the compounds of Formula (I) as NRF2 activators.

Accordingly, the present invention is also directed to a method of regulating NRF2 which method comprises contacting a cell with a compound according to Formula (I), or a salt, particularly a pharmaceutically acceptable salt, thereof.

In another aspect, this invention provides for the use of the compounds of Formula (I) for treating and preventing conditions associated with NRF2 imbalance.

In one aspect, the invention is provides a pharmaceutical composition comprising a compound of the invention according to Formula (I), or a salt, particularly a pharmaceutically acceptable salt thereof, and a pharmaceutically acceptable excipient. Particularly, this invention is directed to a pharmaceutical composition for the treatment of an NRF2 regulated disease or disorder, wherein the composition comprises a compound according to Formula (I), or a salt, particularly a pharmaceutically acceptable salt thereof, and a pharmaceutically acceptable excipient.

In a further aspect, this invention provides for a method of treating a respiratory or non-respiratory disorder, including COPD, asthma, ALI, ARDS, fibrosis, chronic asthma and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness, which comprises administering to a human in need thereof, a compound of Formula (I).

In one aspect, this invention relates to a method of treating COPD, which comprises administering to a human in need thereof, a compound of Formula (I), or a salt, particularly a pharmaceutically acceptable salt thereof.

In one aspect, this invention relates to a method of treating heart failure, which comprises administering to a human in need thereof, a compound of Formula (I), or a salt, particularly a pharmaceutically acceptable salt thereof.

In yet another aspect, this invention provides for the use of a compound of Formula (I), or a salt, particularly a pharmaceutically acceptable salt thereof, for the treatment of a respiratory or non-respiratory disorder, including COPD, asthma, ALI, ARDS, fibrosis, chronic asthma and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness.

In one aspect, this invention relates to the use of a compound of Formula (I), or a salt, particularly a pharmaceutically acceptable salt thereof, for the treatment of COPD.

In one aspect, this invention relates to the use of a compound of Formula (I), or a salt, particularly a pharmaceutically acceptable salt thereof, for the treatment of heart failure.

In a further aspect, this invention relates to use of a compound of Formula (I), or a salt, particularly a pharmaceutically acceptable salt thereof, in the manufacture of a medicament for use in the treatment of a respiratory or non-respiratory disorder, including COPD, asthma, ALI, ARDS, fibrosis, chronic asthma and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness.

In one aspect, this invention relates to use of a compound of Formula (I), or a salt, particularly a pharmaceutically acceptable salt thereof, in the manufacture of a medicament for the treatment of COPD.

In one aspect, this invention relates to use of a compound of Formula (I), or a salt, particularly a pharmaceutically acceptable salt thereof, in the manufacture of a medicament for the treatment of heart failure.

In a further aspect, this invention relates to a compound of Formula (I), or a salt, particularly a pharmaceutically acceptable salt thereof, for use in medical therapy.This invention relates to a compound of Formula (I), or a salt, particularly a pharmaceutically acceptable salt thereof, for use in therapy, specifically for use in the treatment of a respiratory or non-respiratory disorder, including COPD, asthma, ALI, ARDS, fibrosis, chronic asthma and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness.

In one aspect, this invention relates to a compound of Formula (I), or a salt, particularly a pharmaceutically acceptable salt thereof, for use in the treatment of COPD.

In one aspect, this invention relates to a compound of Formula (I), or a salt, particularly a pharmaceutically acceptable salt thereof, for use in the treatment of heart failure.

Other aspects and advantages of the present invention are described further in the following detailed description of the embodiments thereof.

### DETAILED DESCRIPTION OF THE INVENTION

The present invention provides for compounds of Formula (I): 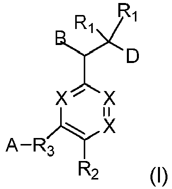 wherein:
B is benzotriazolyl, phenyl, triazolopyridinyl, -O(CH₂)-triazolyl or -(CH₂)₂-triazolyl, wherein each of the benzotriazolyl, phenyl, triazolopyridinyl, -O(CH₂)-triazolyl or -(CH₂)₂-triazolyl is unsubstituted or substituted by 1, 2, or 3 substituents independently selected from -C₁₋₃alkyl, -O-C₁₋₃alkyl, CN, -(CH₂)₂-O-(CH₂)₂-OR₄ and halo;
D is -C(O)OH, -C(O)NR₆R₇, -C(O)NHSO₂CH₃, -SO₂NHC(O)CH₃, 5-(trifluoromethyl)-4H-1,2,4-triazol-2-yl, -NR₆-C(O)-R₇, -NR₆-C(O)-NR₆R₇; -NR₆-C(O)-O-R₇ or tetrazolyl;
R₁ is independently hydrogen, -OH, -C₁₋₃alkyl, - C₁₋₃alkylOR', F, -C₃₋₆spirocycloalkyl, oxetane, or the two R₁ groups together with the carbon to which they are attached form a cyclopropyl group;
R' is hydrogen or -C₁₋₃alkyl;
R₂ is hydrogen, -C₁₋₄alkyl, -CF₃, or halo;
R₃ is -(CH₂)ₘ;
R₄ is hydrogen or -C₁₋₃alkyl;
or, when R₂ is -C₁₋₄alkyl, R₃ is -(CH₂)ₘ-, and m is 2 or 3, R₂ and R₃ together form a cycloalkyl ring fused to the phenyl ring to which they are attached;
A is 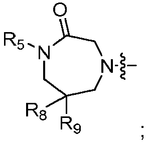
R₅ is hydrogen, -C₁₋₅alkyl or -(CH₂)ₙ-C₃₋₅cycloalkyl;
R₆ is hydrogen or -C₁₋₄alkyl;
R₇ is hydrogen, -C₁₋₅alkyl, -C₃₋₇cycloalkyl, -C₄₋₈heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-SO₂C₁₋₃alkyl, -C₁₋₃alkyl-C₄₋₈heterocycloalkyl, -C₁₋₃alkyl-C(O)NR₄R₆, or heteroaryl, wherein each of -C₁₋₅ alkyl, -C₃₋₇ cycloalkyl, -C₄₋₇ heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-C(O)NR₄R₆, or heteroaryl is unsubstituted or substituted by one or two substituents selected from -OH, -CO₂H,
-C(O)NR₄R₆,-C(O)OR₄, -N-C(O)-C₁₋₃alkyl, F, -CN, -CH-F₂, -CF₃, -(CH₂)ₙ-O-(CH₂)ₘ-CH₃, and-C₃₋₇cycloalkyl, a 5-6-membered heteroaryl ring containing 1, 2 or 3 heteroatoms selected from O, N and S;
or R₆ and R₇ together with the nitrogen atom to which they are attached form a 5-8-membered heterocyclic ring, an 8-11-membered bicyclic heterocyclic ring or a 9- 10-membered bridged bicyclic heterocyclic ring, wherein each 5-8-membered ring, 8-11-membered bicyclic ring, or 9-10-membered bridged bicyclic ring optionally includes one-C(O) or one -S(O)₂, and wherein each 5-8-membered ring, 8-11 -membered bicyclic ring, or 9-10-membered bridged bicyclic ring optionally contains one, two or three oxygen ring atoms, one, two or three sulfur ring atoms or one, two or three nitrogen ring atoms, and wherein each 5-8-membered ring, 8-11-membered bicyclic ring, or 9-10-membered bridged bicyclic ring is unsubstituted or substituted by one, two or three substituents independently selected from -C₁₋₅alkyl, -C₃₋₇cycloalkyl, -C₄₋₇heterocycloalkyl, -(CH₂)phenyl, halogen,-NR₃R₄, -CHF₂, -CF₃, and -(CH₂)ₙ-O-(CH₂)ₘ-CH₃;
R₈ is hydrogen or -C₁₋₄alkyl;
R₉ is hydrogen or -C₁₋₄alkyl;
m is 1, 2 or 3;
n is 0, 1, 2 or 3; and
X is independently CH or N;
or a pharmaceutically acceptable salt thereof.

"Alkyl" refers to a monovalent saturated hydrocarbon chain having the specified number of carbon member atoms. For example, C₁₋₄ alkyl refers to an alkyl group having from 1 to 4 carbon member atoms. Alkyl groups may be straight or branched. Representative branched alkyl groups have one, two, or three branches. Alkyl includes methyl, ethyl, propyl, (n-propyl and isopropyl), butyl (n-butyl, isobutyl, s-butyl, and t-butyl), pentyl (n-pentyl, tert-pentyl, iso-pentyl), and hexyl (n-hexyl, isohexyl, ter-hexyl).

"Cycloalkyl" refers to a monovalent saturated or unsaturated hydrocarbon ring having the specified number of carbon member atoms. For example, C₃₋₆cycloalkyl refers to a cycloalkyl group having from 3 to 6 carbon member atoms. Unsaturated cycloalkyl groups have one or more carbon-carbon double bonds within the ring. Cycloalkyl groups are not aromatic. Cycloalkyl includes cyclopropyl, cyclopropenyl, cyclobutyl, cyclobutenyl, cyclopentyl, cyclopentenyl, cyclohexyl, and cyclohexenyl.

"C₄₋₇heterocycloalkyl" refers to a 4- to 7-membered ring that contains up to 4 hetero atoms, including, nitrogen, oxygen, and sulfur. Examples are azetidine, thietane, thietane 1-oxide, thietane 1,1-dioxide, tetrahydrofuran, pyrrolidine, tetrahydrothiophene, tetrahydrothiophene 1-oxide, tetrahydrothiophene 1, 2-dioxide, piperidine, morpholine, thiomorpholine, thiomorpholine 1-oxide, thiomorpholine 1,1-dioxide, tetrahydropyran, tetrahydrothiopyran, tetrahydrothiopyran 1-oxide, tetrahydrothiopyran 1-1 dioxide, piperidine-2-one, azepan-2-one, pyrrolidin-2-one, azepane, oxepane, oxazepane, thiepane, thiepane 1-oxide, thiepane 1,1-dioxide, and thiazepane.

"Heteroaryl" represents a group or moiety comprising an aromatic monocyclic radical, containing 5 to 6 ring atoms unless otherwise limited in size, including 1 to 4 heteroatoms independently selected from nitrogen, oxygen and sulfur. This term also encompasses 9- to 10-membered bicyclic heterocyclic-aryl groups containing either an aryl ring moiety fused to a heterocycloalkyl ring moiety or a heteroaryl ring moiety fused to a cycloalkyl ring moiety.

Illustrative examples of heteroaryls include, but are not limited to, furanyl, thienyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl (pyridyl), oxo-pyridyl (pyridyl-N-oxide), pyridazinyl, pyrazinyl, pyrimidinyl, triazinyl, benzofuranyl, isobenzofuryl, 2,3-dihydrobenzofuryl, 1,3-benzodioxolyl, dihydrobenzodioxinyl, benzothienyl, indolizinyl, indolyl, isoindolyl, dihydroindolyl, benzimidazolyl, dihydrobenzimidazolyl, benzoxazolyl, dihydrobenzoxazolyl, benzothiazolyl, benzoisothiazolyl, dihydrobenzoisothiazolyl, indazolyl, imidazopyridinyl, pyrazolopyridinyl, benzotriazolyl, triazolopyridinyl, purinyl, quinolinyl, tetrahydroquinolinyl, isoquinolinyl, tetrahydroisoquinolinyl, quinoxalinyl, cinnolinyl, phthalazinyl, quinazolinyl, 1,5-naphthyridinyl, 1,6-naphthyridinyl, 1,7-naphthyridinyl, 1,8-naphthyridinyl, and pteridinyl.

Bicyclic heteroaryl groups include 6,5-fused heteroaryl (9-membered heteroaryl) and 6,6-fused heteroaryl (10-membered heteroaryl) groups. Examples of 6,5-fused heteroaryl (9-membered heteroaryl) groups include benzothienyl, benzofuranyl, indolyl, indolinyl, isoindolyl, isoindolinyl, indazolyl, indolizinyl, isobenzofuryl, 2,3-dihydrobenzofuryl, benzoxazolyl, benzthiazolyl, benzimidazolyl, benzoxadiazolyl, benzthiadiazolyl, benzotriazolyl, 1,3-benzoxathiol-2-on-yl (2-oxo-1,3-benzoxathiolyl), purinyl and imidazopyridinyl.

Examples of 6,6-fused heteroaryl (10-membered heteroaryl) groups include quinolyl, isoquinolyl, phthalazinyl, naphthridinyl (1,5-naphthyridinyl, 1,6-naphthyridinyl, 1,7-naphthyridinyl, 1,8-naphthyridinyl), quinazolinyl, quinoxalinyl, 4H-quinolizinyl, tetrahydroquinolinyl, cinnolinyl, and pteridinyl.

"Oxo" represents a double-bonded oxygen moiety; for example, if attached directly to a carbon atom forms a carbonyl moiety (C = O). "Hydroxy" or "hydroxyl" is intended to mean the radical -OH. As used herein, the term "cyano" refers to the group -CN.

When used herein, the terms 'halogen' and 'halo' include fluorine, chlorine, bromine and iodine, and fluoro, chloro, bromo, and iodo, respectively.

"Substituted" in reference to a group indicates that one or more hydrogen atom attached to a member atom within the group is replaced with a substituent selected from the group of defined substituents. It should be understood that the term "substituted" includes the implicit provision that such substitution be in accordance with the permitted valence of the substituted atom and the substituent and that the substitution results in a stable compound (i.e., one that does not spontaneously undergo transformation such as by rearrangement, cyclization, or elimination and that is sufficiently robust to survive isolation from a reaction mixture). When it is stated that a group may contain one or more substituents, one or more (as appropriate) member atoms within the group may be substituted. In addition, a single member atom within the group may be substituted with more than one substituent as long as such substitution is in accordance with the permitted valence of the atom. Suitable substituents are defined herein for each substituted or optionally substituted group.

The term "independently" means that where more than one substituent is selected from a number of possible substituents, those substituents may be the same or different. That is, each substituent is separately selected from the entire group of recited possible substituents.

The invention also includes various isomers of the compounds of Formula (I) and mixtures thereof. "Isomer" refers to compounds that have the same composition and molecular weight but differ in physical and/or chemical properties. The structural difference may be in constitution (geometric isomers) or in the ability to rotate the plane of polarized light (stereoisomers). The compounds according to Formula (I) contain one or more asymmetric centers, also referred to as chiral centers, and may, therefore, exist as individual enantiomers, diastereomers, or other stereoisomeric forms, or as mixtures thereof. All such isomeric forms are included within the present invention, including mixtures thereof.

Chiral centers may also be present in a substituent such as an alkyl group. Where the stereochemistry of a chiral center present in Formula (I), or in any chemical structure illustrated herein, is not specified the structure is intended to encompass any stereoisomer and all mixtures thereof. Thus, compounds according to Formula (I) containing one or more chiral centers may be used as racemic mixtures, enantiomerically enriched mixtures, or as enantiomerically pure individual stereoisomers.

Individual stereoisomers of a compound according to Formula (I) which contain one or more asymmetric centers may be resolved by methods known to those skilled in the art. For example, such resolution may be carried out (1) by formation of diastereoisomeric salts, complexes or other derivatives; (2) by selective reaction with a stereoisomer-specific reagent, for example by enzymatic oxidation or reduction; or (3) by gas-liquid or liquid chromatography in a chiral environment, for example, on a chiral support such as silica with a bound chiral ligand or in the presence of a chiral solvent. The skilled artisan will appreciate that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation.

For compounds falling within the scope of the invention, the structural conventions used in the Examples are as follows: (a) absolute stereochemistry is defined by the structure; (b) when annotated by "or", then stereochemistry is unknown but resolved; and (c) when annotated by "&" or "and", then stereochemistry is relative, but racemic.

It is to be understood that the references herein to a compound of Formula (I) or a salt thereof includes a compound of Formula (I) as a free base [or acid, as appropriate], or as a salt thereof, for example as a pharmaceutically acceptable salt thereof. Thus, in one embodiment, the invention is directed to a compound of Formula (I). In another embodiment, the invention is directed to a salt of a compound of Formula (I). In a further embodiment, the invention is directed to a pharmaceutically acceptable salt of a compound of Formula (I). In another embodiment, the invention is directed to a compound of Formula (I) or a salt thereof. In a further embodiment, the invention is directed to a compound of Formula (I) or a pharmaceutically acceptable salt thereof.

Compounds of Formula (I) have both a basic amine group and a carboxylic acid group and can consequently be in the form of a zwitterion, also known as an inner salt. Therefore, in an embodiment the compound of Formula (I) is in a zwitterion form.

As used herein, "pharmaceutically acceptable" refers to those compounds (including salts), materials, compositions, and dosage forms which are, within the scope of sound medical judgment, suitable for use in contact with the tissues of human beings and animals without excessive toxicity, irritation, or other problem or complication, commensurate with a reasonable benefit/risk ratio.

Pharmaceutically acceptable salts include, amongst others, those described in Berge, J. Pharm. Sci., 1977, 66, 1-19, or those listed in PH Stahl and C G Wermuth, editors, Handbook of Pharmaceutical Salts; Properties, Selection and Use, Second Edition Stahl/Wermuth: Wiley- VCH/VHCA, 2011 (see http://www.wiley.com/WileyCDA/WileyTitle/productCd-3906390519.html).

The skilled artisan will appreciate that pharmaceutically acceptable salts of the compounds according to Formula (I) may be prepared. These pharmaceutically acceptable salts may be prepared *in situ* during the final isolation and purification of the compound, or by separately treating the purified compound in its free acid or free base form with a suitable base or acid, respectively.

It will be understood that if a compound of Formula (I) contains two or more basic moieties, the stoichiometry of salt formation may include 1, 2 or more equivalents of acid. Such salts would contain 1, 2 or more acid counterions, for example, a dihydrochloride salt.

Stoichiometric and non-stoichiometric forms of a pharmaceutically acceptable salt of a compound of Formula (I) are included within the scope of the invention, including sub-stoichiometric salts, for example where a counterion contains more than one acidic proton.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexylresorcinate, hippurate, hydrabamine (*N,N*'-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, *p*-aminobenzenesulfonate, *p-*aminosalicyclate, pamoate (embonate), pantothenate, pectinate, persulfate, phenylacetate, phenylethylbarbiturate, phosphate, polygalacturonate, propionate, *p*-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (*N*-benzylphenethylamine), benzathine (*N,N*'-dibenzylethylenediamine), *bis*-(2-hydroxyethyl)amine, bismuth, calcium, chloroprocaine, choline, clemizole (1-*p* chlorobenzyl-2-pyrrolildine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N*-methylglucamine), piperazine, piperidine, potassium, procaine, quinine, quinoline, sodium, strontium, *t-*butylamine, and zinc.

As used herein, the term "a compound of Formula (I)" or "the compound of Formula (I)" refers to one or more compounds according to Formula (I). The compound of Formula (I) may exist in solid or liquid form. In the solid state, it may exist in crystalline or noncrystalline form, or as a mixture thereof. The skilled artisan will appreciate that pharmaceutically acceptable solvates may be formed from crystalline compounds wherein solvent molecules are incorporated into the crystalline lattice during crystallization. Solvates may involve non-aqueous solvents such as, but not limited to, ethanol, isopropanol, DMSO, acetic acid, ethanolamine, or ethyl acetate, or they may involve water as the solvent that is incorporated into the crystalline lattice. Solvates wherein water is the solvent incorporated into the crystalline lattice are typically referred to as "hydrates." Hydrates include stoichiometric hydrates as well as compositions containing variable amounts of water. The invention includes all such solvates.

The skilled artisan will further appreciate that certain compounds of the invention that exist in crystalline form, including the various solvates thereof, may exhibit polymorphism (i.e., the capacity to occur in different crystalline structures). These different crystalline forms are typically known as "polymorphs." The invention includes all such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions.

The subject invention also includes isotopically-labelled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

Compounds of the present invention and pharmaceutically acceptable salts of said compounds that contain the aforementioned isotopes and/or other isotopes of other atoms are within the scope of the present invention. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds of Formula (I) and following of this invention can generally be prepared by carrying out the procedures disclosed in the Schemes and/or in the Examples below, by substituting a readily available isotopically labeled reagent for a non-isotopically labeled reagent.

### Representative Embodiments

In one embodiment, the compound of Formula (I) is: 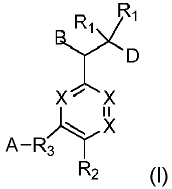 wherein:
B is benzotriazolyl, phenyl, triazolopyridinyl, -O(CH₂)-triazolyl or -(CH₂)₂-triazolyl, wherein each of the benzotriazolyl, phenyl, triazolopyridinyl, -O(CH₂)-triazolyl or -(CH₂)₂-triazolyl is unsubstituted or substituted by 1, 2, or 3 substituents independently selected from -C₁₋₃alkyl, -O-C₁₋₃alkyl, CN, -(CH₂)₂-O-(CH₂)₂-OR₄ and halo;
D is -C(O)OH, -C(O)NR₆R₇, -C(O)NHSO₂CH₃, -SO₂NHC(O)CH₃, 5-(trifluoromethyl)-4H-1,2,4-triazol-2-yl, -NR₆-C(O)-R₇, -NR₆-C(O)-NR₆R₇; -NR₆-C(O)-O-R₇ or tetrazolyl;
R₁ is independently hydrogen, -OH, -C₁₋₃alkyl, - C₁₋₃alkylOR', F, -C₃₋₆spirocycloalkyl, oxetane, or the two R₁ groups together with the carbon to which they are attached form a cyclopropyl group;
R' is hydrogen or -C₁₋₃alkyl;
R₂ is hydrogen, -C₁₋₄alkyl, -CF₃, or halo;
R₃ is -(CH₂)ₘ;
R₄ is hydrogen or -C₁₋₃alkyl;
or, when R₂ is -C₁₋₄alkyl, R₃ is -(CH₂)ₘ-, and m is 2 or 3, R₂ and R₃ together form a cycloalkyl ring fused to the phenyl ring to which they are attached;
A is 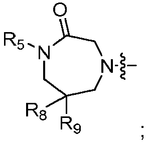
R₅ is hydrogen, -C₁₋₅alkyl or -(CH₂)ₙ-C₃₋₅cycloalkyl;
R₆ is hydrogen or -C₁₋₄alkyl;
R₇ is hydrogen, -C₁₋₅alkyl, -C₃₋₇cycloalkyl, -C₄₋₈heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-SO₂C₁₋₃alkyl, -C₁₋₃alkyl-C₄₋₈heterocycloalkyl, -C₁₋₃alkyl-C(O)NR₄R₆, or heteroaryl, wherein each of -C₁₋₅ alkyl, -C₃₋₇ cycloalkyl, -C₄₋₇ heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-C(O)NR₄R₆, or heteroaryl is unsubstituted or substituted by one or two substituents selected from -OH, -CO₂H,
-C(O)NR₄R₆, -C(O)OR₄, -N-C(O)-C₁₋₃alkyl, F, -CN, -CH-F₂, -CF₃, -(CH₂)ₙ-O-(CH₂)ₘ-CH₃, and-C₃₋₇cycloalkyl, a 5-6-membered heteroaryl ring containing 1, 2 or 3 heteroatoms selected from O, N and S;
or R₆ and R₇ together with the nitrogen atom to which they are attached form a 5-8-membered heterocyclic ring, an 8-11-membered bicyclic heterocyclic ring or a 9- 10-membered bridged bicyclic heterocyclic ring, wherein each 5-8-membered ring, 8-11-membered bicyclic ring, or 9-10-membered bridged bicyclic ring optionally includes one - C(O) or one -S(O)₂, and wherein each 5-8-membered ring, 8-11 -membered bicyclic ring, or 9-10-membered bridged bicyclic ring optionally contains one, two or three oxygen ring atoms, one, two or three sulfur ring atoms or one, two or three nitrogen ring atoms, and wherein each 5-8-membered ring, 8-11-membered bicyclic ring, or 9-10-membered bridged bicyclic ring is unsubstituted or substituted by one, two or three substituents independently selected from -C₁₋₅alkyl, -C₃₋₇cycloalkyl, -C₄₋₇heterocycloalkyl, -(CH₂)phenyl, halogen,-NR₃R₄, -CHF₂, -CF₃, and -(CH₂)ₙ-O-(CH₂)ₘ-CH₃;
R₈ is hydrogen or -C₁₋₄alkyl;
R₉ is hydrogen or -C₁₋₄alkyl;
m is 1, 2 or 3;
n is 0, 1, 2 or 3; and
X is independently CH or N;
or a pharmaceutically acceptable salt thereof.

In one embodiment, the compound of Formula (I) is substituted as follows:
B is benzotriazolyl, phenyl, triazolopyridinyl, -O(CH₂)-triazolyl or -(CH₂)₂-triazolyl, wherein each of the benzotriazolyl, phenyl, triazolopyridinyl, -O(CH₂)-triazolyl or -(CH₂)₂-triazolyl is unsubstituted or substituted by 1, 2, or 3 substituents independently selected from -C₁₋₃alkyl, -O-C₁₋₃alkyl, CN, -(CH₂)₂-O-(CH₂)₂-OR₄ and halo;
D is -C(O)OH, -C(O)NR₆R₇, -C(O)NHSO₂CH₃, -SO₂NHC(O)CH₃, 5-(trifluoromethyl)-4H-1,2,4-triazol-2-yl, or tetrazolyl;
R₁ is independently hydrogen, -OH, -C₁₋₃alkyl, -C₁₋₃alkylOR', F, -C₃₋₆spirocycloalkyl, oxetane, or the two R₁ groups together with the carbon to which they are attached form a cyclopropyl group;
R' is hydrogen or -C₁₋₃alkyl;
R₂ is hydrogen, -C₁₋₄alkyl, -CF₃, or halo;
R₃ is -(CH₂)ₘ;
R₄ is hydrogen or -C₁₋₃alkyl;
or, when R₂ is -C₃₋₄alkyl, R₃ is -(CH₂)ₘ-, and m is 2 or 3, R₂ and R₃ together form a cycloalkyl ring fused to the phenyl ring to which they are attached;
A is 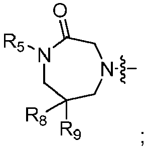
R₅ is hydrogen, C₁₋₅alkyl or -(CH₂)ₙ-C₃₋₅cycloalkyl;
R₆ is hydrogen or -C₁₋₄alkyl;
R₇ is hydrogen, -C₁₋₅alkyl, -C₃₋₇cycloalkyl, -C₄₋₈heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-SO₂C₁₋₃alkyl, -C₁₋₃alkyl-C₄₋₈heterocycloalkyl, -C₁₋₃alkyl-C(O)NR₃R₄, or heteroaryl, wherein each of -C₁₋₅ alkyl, -C₃₋₇ cycloalkyl, -C₄₋₇ heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-C(O)NR₃R₄, or heteroaryl is unsubstituted or substituted by one or two substituents selected from -OH, -C(O), -N-C(O)-C₁₋₃alkyl, F, CN, -CH-F₂, -CF₃, -(CH₂)ₙ-O-(CH₂)ₘ-CH₃, and -C₃₋₇cycloalkyl;
or R₆ and R₇ together with the nitrogen atom to which they are attached form a 5-8-membered heterocyclic ring, an 8-11-membered bicyclic heterocyclic ring or a 9- 10-membered bridged bicyclic heterocyclic ring, wherein each 5-8-membered ring, 8-11-membered bicyclic ring, or 9-10-membered bridged bicyclic ring optionally includes one - C(O) or one -S(O)₂, and wherein each 5-8-membered ring, 8-11 -membered bicyclic ring, or 9-10-membered bridged bicyclic ring optionally contains one, two or three oxygen ring atoms, one, two or three sulfur ring atoms or one, two or three nitrogen ring atoms, and wherein each 5-8-membered ring, 8-11-membered bicyclic ring, or 9-10-membered bridged bicyclic ring is unsubstituted or substituted by one, two or three substituents independently selected from -C₁₋₅alkyl, -C₃₋₇cycloalkyl, -C₄₋₇heterocycloalkyl, -(CH₂)phenyl, halogen,-NR₃R₄, -CHF₂, -CF₃, and -(CH₂)ₙ-O-(CH₂)ₘ-CH₃;
R₈ is hydrogen or -C₁₋₄alkyl;
R₉ is hydrogen or -C₁₋₄alkyl;
m is 1, 2 or 3;
n is 0, 1, 2 or 3; and
X is independently CH or N;
or a pharmaceutically acceptable salt thereof.

In one embodiment, the compound of Formula (I) is substituted as follows:
B is benzotriazolyl which is unsubstituted or substituted by 1, 2, or 3 substituents independently selected from -C₁₋₃alkyl;
D is -C(O)OH;
R₁ is independently hydrogen or -C₁₋₃alkyl;
R₂ is methyl or chloro;
R₃ is -(CH₂)ₘ;
A is 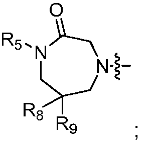
R₅ is -C₁₋₅alkyl or -(CH₂)ₙ-C₃₋₅cycloalkyl;
R₈ is hydrogen or methyl;
R₉ is hydrogen or methyl;
m is 1;
n is 0 or 1; and
X is CH;
or a pharmaceutically acceptable salt thereof.

It is to be understood that the present invention covers all combinations of the embodiments and particular groups described hereinabove.

Specific examples of compounds of the present invention include the following:
(S)-3-(1,4-dimethyl-1H-benzo[d][1,2,3]triazol-5-yl)-2,2-dimethyl-3-(4-methyl-3-((4-methyl-3-oxo-1,4-(S)-3-(3-((4-cyclopropyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((6,6-dimethyl-3-oxo-4-propyl-1,4-diazepan-1-yl)methyl)-4-methylphenyl)-2,2-dimethylpropanoic acid;
(S)-3-(1,4-Dimethyl-1H-benzo[d][1,2,3]triazol-5-yl)-2,2-dimethyl-3-(4-methyl-3-((4-methyl-3-oxo-1,4-diazepan-1-yl)methyl)phenyl)propanoic acid;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((4-ethyl-6,6-dimethyl-3-oxo-1,4-diazepan-1-yl)methyl)-4-methylphenyl)-2,2-dimethylpropanoic acid;
3-(1,4-Dimethyl-1H-benzo[d][1,2,3]triazol-5-yl)-3-(3-((6-ethyl-4-methyl-3-oxo-1,4-diazepan-1-yl)methyl)-4-methylphenyl)-2,2-dimethylpropanoic acid;
(S)-3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid, 0.3 Formic acid salt;
3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid;
3-(3-((4-Cyclobutyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid;
3-(3-((4-(Cyclopropylmethyl)-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid;
rel-3S-(1,4-Dimethyl-1H-benzo[d][1,2,3]triazol-5-yl)-2,2-dimethyl-3-(rel-(R)-3-(4,6,6-trimethyl-3-oxo-1,4-diazepan-1-yl)-2,3-dihydro-1H-inden-5-yl)propanoic acid;
rel-3S-(1,4-Dimethyl-1H-benzo[d][1,2,3]triazol-5-yl)-2,2-dimethyl-3-(rel-(S)-3-(4,6,6-trimethyl-3-oxo-1,4-diazepan-1-yl)-2,3-dihydro-1H-inden-5-yl)propanoic acid;
rel-(3R)-3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid;
3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid;
3-((1-Ethyl-1H-1,2,3-triazol-4-yl)methoxy)-2,2-dimethyl-3-(4-methyl-3-((4,6,6-trimethyl-3-oxo-1,4-diazepan-1-yl)methyl)phenyl)propanoic acid;
3-(4-Chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoic acid; and
3-(4-Chloro-3-((4,6,6-trimethyl-3-oxo-1,4-diazepan-1-yl)methyl)phenyl)-5-(1-ethyl-1H-1,2,3-triazol-4-yl)-2,2-dimethylpentanoic acid;
or a pharmaceutically acceptable salt thereof.

### Compound Preparation

The skilled artisan will appreciate that if a substituent described herein is not compatible with the synthetic methods described herein, the substituent may be protected with a suitable protecting group that is stable to the reaction conditions. The protecting group may be removed at a suitable point in the reaction sequence to provide a desired intermediate or target compound. Suitable protecting groups and the methods for protecting and de-protecting different substituents using such suitable protecting groups are well known to those skilled in the art; examples of which may be found in T. Greene and P. Wuts, Protecting Groups in Chemical Synthesis (3rd ed.), John Wiley & Sons, NY (1999). In some instances, a substituent may be specifically selected to be reactive under the reaction conditions used. Under these circumstances, the reaction conditions convert the selected substituent into another substituent that is either useful as an intermediate compound or is a desired substituent in a target compound.

The synthesis of the compounds of the general Formula (I) and pharmaceutically acceptable derivatives and salts thereof may be accomplished as outlined below in Schemes **1-12.** In the following description, the groups are as defined above for compounds of Formula (I) unless otherwise indicated. Abbreviations are as defined in the Examples section. Starting materials are commercially available or are made from commercially available starting materials using methods known to those skilled in the art. 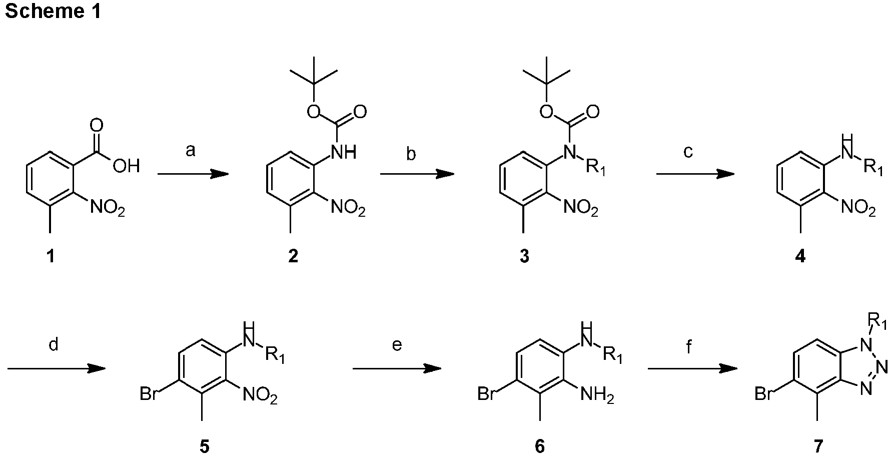 *Conditions:* a) DPPA, TEA, t-BuOH; b) NaH, R₁I, DMF; c) TFA, DCM; d) NBS, DMF; e) SnCl₂-2H₂O, EtOH; f) NaNO₂, H₂SO₄

Scheme **1** shows a general scheme for the preparation of triazole **7.** In scheme **1,** R₁ is C₁₋₃alkyl or -(CH₂)₂-O-(CH₂)₂-OR₄. Starting with commercially available **3**-methyl-2-nitrobenzoic acid, a Curtius rearangement with DPPA provides intermediate **2.** A skilled artisan will appreciate that compound **2** could be prepared from the appropriate aniline compound. Alkylation of the carbamate with an alkyl iodide provides intermediate **3.** Deprotection of the amine with TFA and bromination with NBS provides intermediate **5.** Reduction of the nitro to the aniline and diazotization and cyclization provides the required triazole **7.** 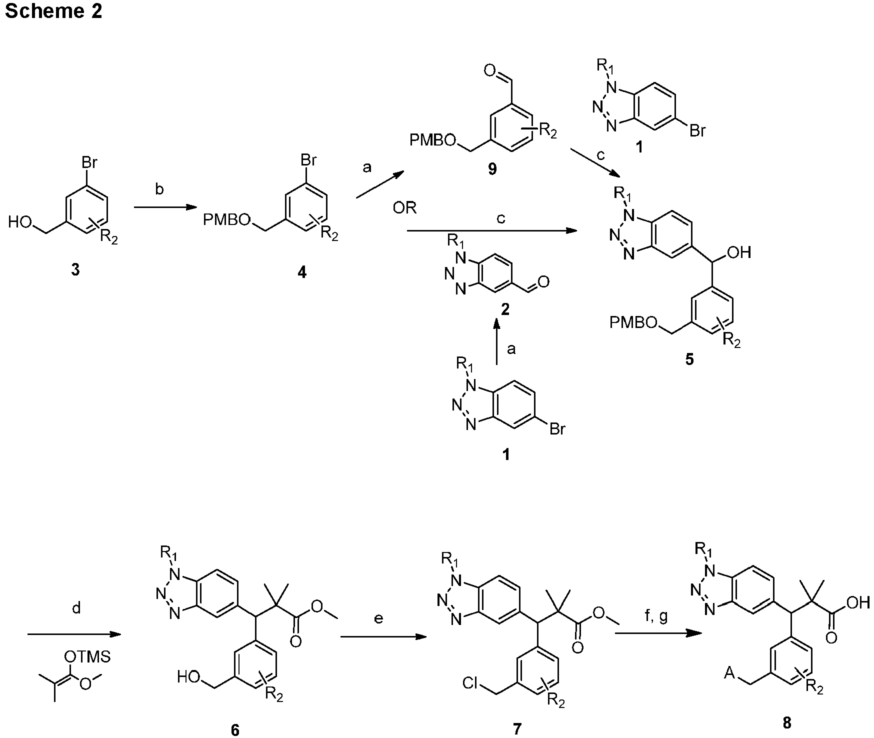 *Conditions:* a) n-BuLi, DMF, THF b) NaH, PMBCI, DMF c) t-BuLi or n-BuLi, THF d) TiCl₄, DCM (or) (i) DBU, Cl₃CCN, CH₃CN; ii) Tf₂NH, iii) DDQ, DCM/H₂O e) SO₂Cl, DCM f) R₁₃H, DIPEA, CH₃CN; (or) R₁₃H, NaH, DMF g) LiOH, MeOH, THF.

Scheme **2** represents a general scheme for the preparation of compounds amLording to Formula (I). In Scheme **2,** R₂ and A are as defined previously. Triazole 1 is either commercially available or may be synthesized from readily available materials. Reaction conditions are as described above in the scheme; however, the skilled artisan will appreciate that certain modifications in the reaction conditions and/or reagents used are possible.

Treatment of triazole **1** with n-butyl lithium and DMF in presence of a suitable solvent produces the desired aldehyde product **2.** The coupling partner for aldehyde **2** is obtained by first protecting the benzylic alcohol **3** as its para-methoxybenzylether. It will be appreciated that alternative protecting groups are possible. Coupling of the aldehyde **2** and bromide **4** can be amLomplished via treatment of the bromide first with t-butyl lithium or n-butyl lithium followed by addition of the aldehyde. However, the skilled artisan will appreciate that other aldehydes, such as substituted phenyl aldehyde may also be applied. Intermediate alcohol **6,** arises from treatment of alcohol **5** with the appropriate silylketene acetal in the presence of a Lewis acid or via one-pot brφnsted base / brφnsted acid system, followed by deprotection with DDQ. Benzylic alcohol **6** can be transformed to the requisite chloride **7** using thionyl chloride. Completion of the synthesis can be amLomplished by deplacement of chloride, following by hydrolysis of the ester to produce **8**

It will be also be appreciated by the skilled artisan that intermediate **5** may be prepared by coupling bromide **1** with aldehyde **9.** 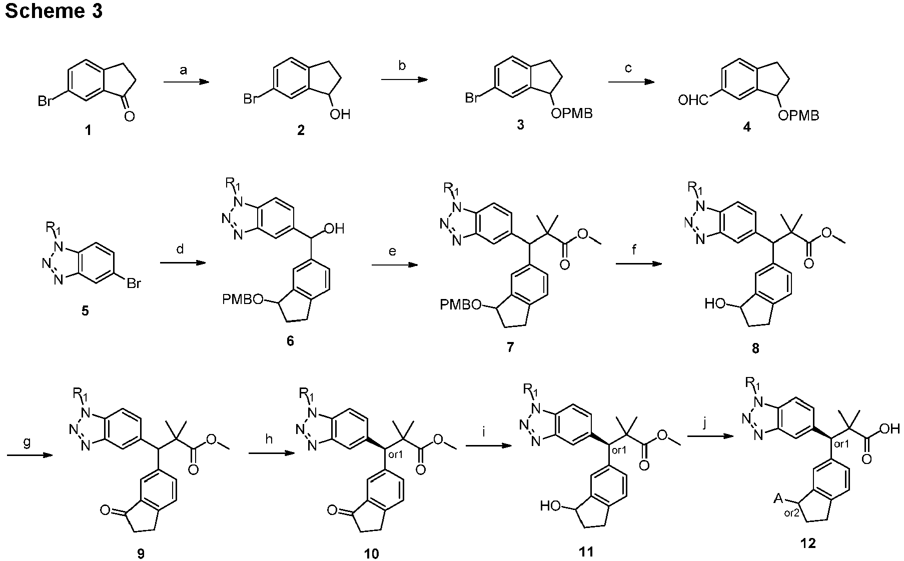 *Conditions:* a) NaBH₄, MeOH, THF; b) PMBCI, NaH; c) n-BuLi, DMF; d) t-BuLi, **4,** THF; e) (i) Cl₃CCN, DBU, acetonitrile; (ii) Tf₂NH, Me₂C=C(OR²)OTMS, acetonitrile; f) DDQ; g) Dess-Martin periodinane, DCM; h) Chiral SFC; (i) NaBH₄, MeOH; j) (i) SOCl₂, DCM; (ii) **A,** K₂CO₃, Nal, acetonitrile; k (iii) NaOH, MeOH/H₂O

Scheme **3** represents a general scheme for the preparation of compounds amLording to Formula (I). In Scheme **2,** R₂ and A are as defined previously. 6-Bromo-2,3-dihydro-1H-inden-1-one **1** is commercially available. Triazole **5** is either commercially available or may be synthesized from readily available materials. Reaction conditions are as described above in the scheme; however, the skilled artisan will appreciate that certain modifications in the reaction conditions and/or reagents used are possible.

Starting with commercially available indanone **1,** reduction with NaBH₄ produces desired alcohol intermediate **2.** Intermediate **2** hydoxyl group may be protected as the PMB ether by treating with NaH and PMBCI to give intermediate **3.** It will be appreciated by the skilled artisan that the protecting group may vary and is not limited to PMB. Further transformation of intermediate **3** to the requisite aldehyde by treatment with butyl lithium and DMF yields desired intermediate **4.** Coupling of **4** and **5** is amLomplished by treatment of **5** with t-butyl liuthium and intermediate **4** to form alcohol **6.** Alcohol **6** was coverted to **7** by first treating with Cl₃CCN and DBU followed by the requisite, commercially available silyl ketene acetal in the presence of Tf₂NH. The deprotection of intermediate **7** with DDQ formed intermediate **8.** Oxidation of **8** with Dess-Martin periodinane to give alcohol **9,** and then resolved by Chiral SFC to give single enantiomerically pure isomer **10.** Intermediate **10** was reduced with NaBH4 to alcohol **11.** The intermediate **11** was first treated with SOCl₂ followed by lactam **A** and K₂CO₃, Nal before hydrolysis with NaOH to form racemic acid, which then followed by chiral SFC to give the desired acid as single isomer **12.** 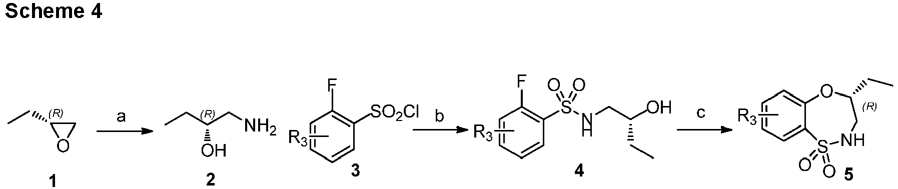 *Conditions:* a) NH₄OH; b) K₂CO₃, THF/H₂O; c) t-BuOK, DMSO

Scheme **4** represents a general scheme for the preparation of sulfonamide **5.** In this, (R)-2-ethyloxirane and substituted 2-fluorobenzene-1-sulfonyl chloride depicted as starting material are commercially available. Reaction conditions are as described above in the scheme; however, the skilled artisan will appreciate that certain modifications in the reaction conditions and/or reagents used are possible. Epoxide ring opening of (R)-2-ethyloxirane yields a single enantiomerically pure **2.** Sulfonamide formation under basic condition with appropriate amino alcohol to give intermediate **4,** followed by displacement of fluoride **3** with potassium tert-butoxide provides the required intermediate **5.** 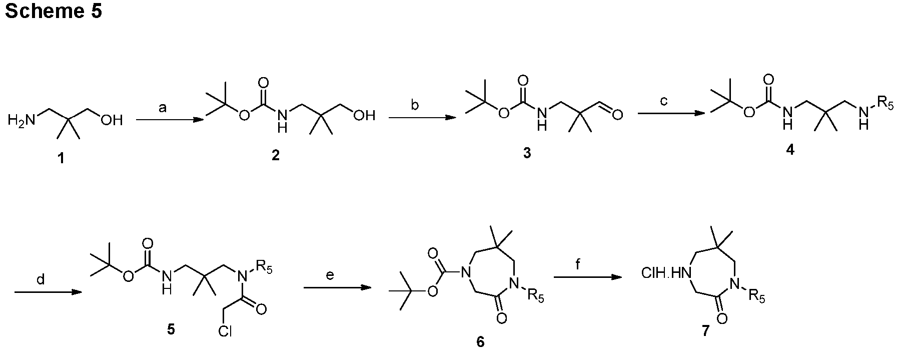 *Conditions:* a) (Boc)₂O, DCM b) TEMPO, KBr, NaHCO₃, NaOCI, DCM C) amine, NaCNBH₃, ACOH, MeOH d) Et₃N, 2-chloroacetyl chloride, DCM e) NaH, DMF f) HCI, dioxane, DCM

Scheme **5** represents a general scheme for the preparation of compounds amLording to Formula (I). 3-Amino-2,2-dimethylpropan-1-ol **1** is commercially available. Reaction conditions are as described above in the scheme; however, the skilled artisan will appreciate that certain modifications in the reaction conditions and/or reagents used are possible.

Protection of amine **1** as its Boc carbamate followed by oxidation of the alcohol to the aldehyde with TEMPO gives intermediate **3.** Reductive amination with an appropriate amine yields compound **4.** Compound **4** was reacted with 2-chloroacetyl chloride in the presence of triethylamine to give compound **5.** Cyclization was sumLessful with NaH to give lactam **6.** Removing the the Boc group with HCI gives lactam **7** as the hydrochloride salt. 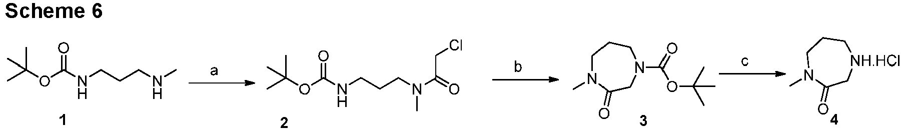 *Conditions:* a) Et₃N, 2-chloroacetyl chloride, THF b) NaH, DMF c) HCI, dioxane, DCM

Scheme **6** represents a general scheme for the preparation of compounds 1-methyl-1,4-diazepan-2-one hydrochloride. tert-Butyl (3-(methylamino)propyl)carbamate **1** is commercially available. Reaction conditions are as described above in the scheme; however, the skilled artisan will appreciate that certain modifications in the reaction conditions and/or reagents used are possible. Alkylation of commercially available starting material **1** with 2-chloroacetyl chloride in the presence of triethylamine produces compound **2.** Cyclization using NaH as base gives lactam **3.** Deprotection the Boc group with HCI furnishes lactam **4** as the hydrochloride salt. 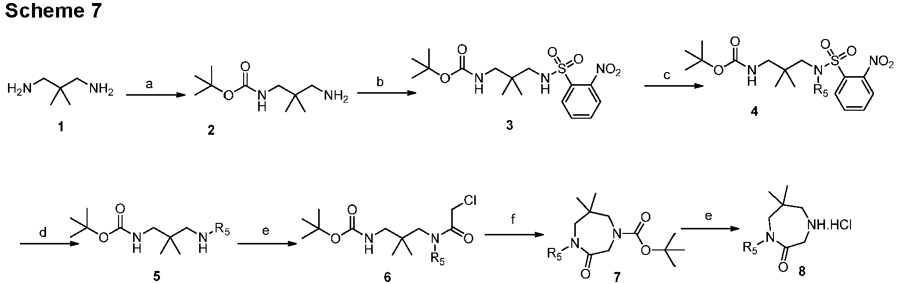 *Conditions:* a) (Boc)₂O, DCM; b) K₂CO₃, 2-nitrobenzene-1-sulfonyl chloride, DCM; c) R₅I, K₂CO₃, DMF, 90 °C; d) PhSH, K₂CO₃, DMF; e) Et₃N, 2-chloroacetyl chloride, DCM; f) NaH, DMF; g) HCI, dioxane, DCM

Scheme **7** represents a general scheme for the preparation of compounds amLording to Formula (I). 2,2-Dimethylpropane-1,3-diamine **1** is commercially available. Reaction conditions are as described above in the scheme; however, the skilled artisan will appreciate that certain modifications in the reaction conditions and/or reagents used are possible. Protection of mono-amine **1** as its Boc carbamate followed by sulfonamide formation with 2-nitrobenzene-1-sulfonyl chloride under basic conditions provides compound **3.** Treatment of compound **3** with an appropriate available alkyl iodide at high temperature provides compound **4,** followed by deprotection to give amine **5.** Compound **5** was reacted with 2-chloroacetyl chloride in the presence of triethylamine to furnish compound **6.** Cyclization was sumLessful with NaH to give lactam **7** which can be subsequently deprotected HCI to give lactam **8** as the hydrochloride salt. 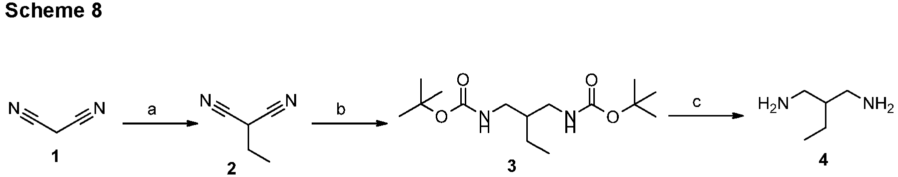 *Conditions:* a) iodoethane, tetrabutylammonium bromide, K₂CO₃; b) Raney-Ni, Boc anhydride, MeOH; c) HCI, dioxane, DCM

Scheme **8** represents a general scheme for the preparation of 6-Ethyl-1-methyl-1,4-diazepan-2-one hydrochloride. Malononitrile **1** is commercially available. Reaction conditions are as described above in the scheme; however, the skilled artisan will appreciate that certain modifications in the reaction conditions and/or reagents used are possible. Treatment of malononitrile with iodoethane under basic conditions gives compound **2.** Hydrogenation followed by Boc protection to aid in purification produces compound **3.** Removal of the Boc groups with HCI provides diamine **4** as hydrochloride salt. Following the same sequence as Scheme **8** above the lactam 6-ethyl-1-methyl-1,4-diazepan-2-one hydrochloride can be produced.

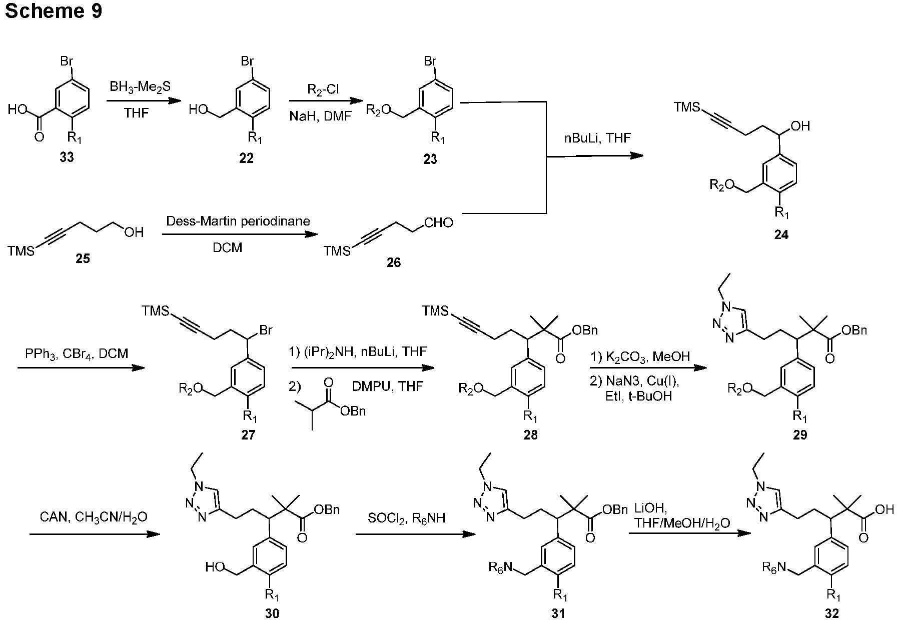

Scheme **9** represents a general scheme for the preparation of compounds according to Formula (I). R₁ R₂, and R₆ are as previously defined. The commercially available carboxylic acid **33** is reduced with a suitable reducing agent like BH₃•Me₂S to provide the intermediate **22.** Intermediate **23** is prepared by alkylation of **22** using a strong base and a suitable protecting group as defined for R₂. Intermediate **26** is prepared by the oxidation of the aceylenic alcohol with Dess-Martin periodinane in DCM. One skilled in the art could also envisage using other suitable alcohol oxidation methods to perform this conversion. A metal halogen exchange reaction is then performed on **23,** after which, aldehyde **26** is added to provide the secondary benzylic alcohol **24.** Conversion of the benzylic alcohol **26** to bromide **27** is achieved using triphenylphosphine and a bromide source such as carbontetrabromide. The reaction of **27** with enolates such as that derived from iso-butyates affords the sterically hindered esters **28.** The use of an additive such as 1,3-dimethyl-3,4,5,6-tetrahydro-2(1H)-pyrimidinone (DMPU) may improve the effectiveness of this reaction. The dialkylated ester **28** containing the TMS protected acetylene can be de-silylated with aqueous potassium carbonate and subjected to a click reaction using sodium azide and ethyl iodide with a copper catalyst to afford the triazoles such as **29.** The protected benzyl alcohol can be deprotected with ceric ammonium nitrate to provide **30.** One skilled in the art could also envisage using other suitable oxidative or acidic methods to perform this reaction. The benzylic chloride, **31,** is prepared by the activation of the benzylic alcohol using a chlorinating reagent like thionyl chloride. The subsequent displacement of the chloride with a suitable electrophile such as a secondary amine or a sulfonamide affords **32.** 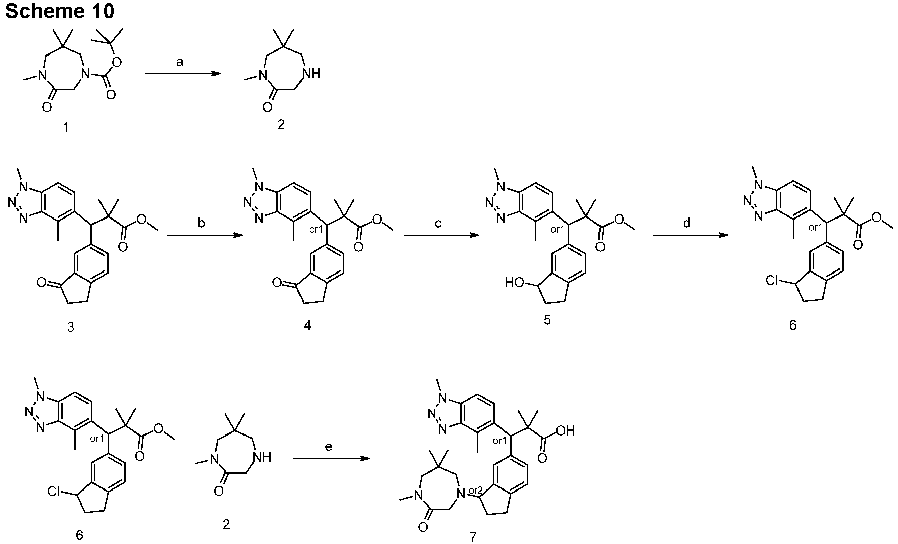 *Conditions:* a) 4 M HCI in p-dioxane; b) Chiral SFC; c) NaBH₄, MeOH d) SOCl₂, DCM; e) (i) K₂CO₃, Nal, MeCN/THF; (ii) NaOH, MeOH/H₂O; (iii) Chiral SFC

Scheme **10** represents a general scheme for the preparation of compounds according to Forumula I. Starting material **1** and **3** are synthesized according to scheme 6 and scheme 3 respectively. Reaction conditions are as described above in the scheme; however, the skilled artisan will appreciate that certain modifications in the reaction conditions and/or reagents used are possible. Treatment of Boc protected lactam amine **1** with 4 M HCI in p-dioxane gave the corresponding amine HCI salt **2.** The racemic bisaryl indanone **3** was chirally separated with chiral SFC gave both enantiomers **4.** Each enantiomer **4** was treated with NaBH₄ to give hydroxyindane **5** which was further treated with SOCl₂ to give corresponding chloride **6.** This bisaryl indane chloride **6** reacted with the above lactam amine **2** and K₂CO₃, Nal in MeCN/THF gave corresponding lactam amine substituted bisaryl indane ester. Further hydrolysis of the ester with NaOH in MeOH/H₂O followed by chiral SFC separation gave desired product **7.** 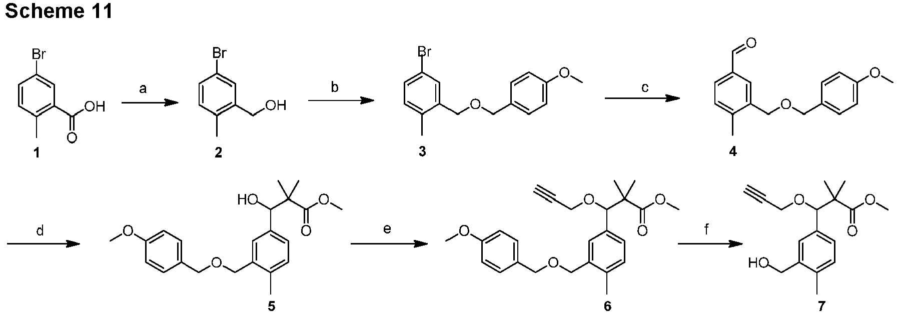 *Conditions:* a) BH₃-Me₂S, THF; b) NaH, DMF, PMB-Cl; c) nBuLi, THF, DMF; d) (i) 1-methylimidazole, ((1-methoxy-2-methylprop-1-en-1-yl)oxy)trimethylsilane, LiCI, DMF, (ii) NaOH, KHF₂, H₂O, HCl; e) 3-bromoprop-1-yne, NaH, DMF; f) DDQ, DCM/H₂O

Scheme **11** represents a general scheme for the preparation of compounds according to Formula I. Reaction conditions are as described above in the scheme; however, the skilled artisan will appreciate that certain modifications in the reaction conditions and/or reagents used are possible. Carboxylic acid **1** can be reduced to the benzyl alcohol in THF using a solution of borane-methyl sulfide complex to provide **2.** Alkylation of the alcohol using a strong base and a suitable protecting group provides intermediate **3.** Formylation of the bromide using n-butyl lithium and DMF affords **4.** Intermediate **5,** arises from treatment of aldehyde **4** with the appropriate silylketene acetal in the presence of lithium chloride. Intermediate **6** can be prepared by treatement of the alcohol with an strong base, like sodium hydride, followed by addition of 3-bromoprop-1-yne. Deprotection of the benzyl alcohol can be achieved using an oxidative reagent such as DDQ to provide **7.** 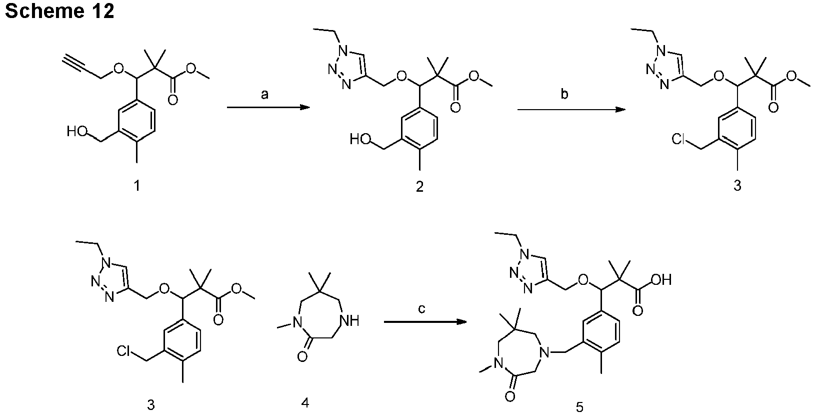 *Conditions:* a) Etl, NaN₃, Cul, i-PrOH/THF/H₂O; b) SOCl₂, DCM; c) (i) DIEA, DMF; (ii) NaOH, MeOH/H₂O

Scheme **12** represents a general scheme for the preparation of compounds according to Formula I. In Scheme 13, R₂, R₆ and A are as defined previously. Starting material **1** and **4** are synthesized according to scheme and scheme 6 respectively. Reaction conditions are as described above in the scheme; however, the skilled artisan will appreciate that certain modifications in the reaction conditions and/or reagents used are possible. Application of click chemistry on starting material ether linked alkyne **1** with Etl, NaN₃, Cul, DIEA in i-PrOH/THF/H₂O gave the corresponding ether linked triazole **2.** This ether linked triazole benzyl alcohol **2** was treated with SOCl₂ to give corresponding benzyl chloride **3.** The benzyl chloride **3** reacted with the lactam amine **4** and DIEA in DMF gave corresponding lactam amine substituted ether linked bisaryl ester. Further hydrolysis of this ester with NaOH in MeOH/H₂Ogave desired product **5.**

### Biological Activity

As stated above, the compounds according to Formula I are Nrf2 activators, and are useful in the treatment or prevention of human diseases that exhibit oxidative stress components such as respiratory and non-respiratory disorders, including COPD, asthma, ALI, ARDS, fibrosis, chronic and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Nonalcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness.

The biological activity of the compounds according to Formula I can be determined using any suitable assay for determining the activity of a candidate compound as a Nrf2 antagonist, as well as tissue and in vivo models.

The biological activity of the compounds of Formula (I) are demonstrated by the following tests.

### BEAS-2B NQO1 MTT Assay

NAD(P)H:quinone oxidoreductase 1 (NQO1), also called DT diaphorase, is a homodimeric FAD-containing enzyme that catalyzes obligatory NAD(P)H-dependent two-electron reductions of quinones and protects cells against the toxic and neoplastic effects of free radicals and reactive oxygen species arising from one-electron reductions. The transcription of NQO1 is finely regulated by Nrf2, and thus NQO1 activity is a good marker for Nrf2 activation. On day one, frozen BEAS-2B cells (ATCC) are thawed in a water bath, counted, and re-suspended at a concentration of 250,000 cells/mL. Fifty microliters of cells are plated in 384 well black clear-bottomed plates. Plates are incubated at 37°C, 5% CO₂ overnight. On day two, plates are centrifuged and 50nL of compound or controls are added to the cells. Plates are then incubated at 37°C, 5% CO₂ for 48 hours. On day four, medium is aspirated from the plate and crude cell lysates are made by adding 13uL of 1X Cell Signaling Technologies lysis buffer with 1 Complete, Mini, EDTA-free Protease Inhibitor Tablet (Roche) for each 10mL of lysis buffer. After lysis plates are incubated for 20 minutes at room temperature. Two microliters of lysate are removed for use in Cell Titer Glo assay (Promega) and MTT cocktail is prepared (Prochaska et. al. 1998) for measurement of NQO1 activity. Fifty microliters of MTT cocktail is added to each well, plate is centrifuged, and analyzed on an Envision plate reader (Perkin Elmer) using Absorbance 570nm label for 30 minutes. Product formation is measured kinetically and the pEC₅₀ of NQO1 specific activity induction is calculated by plotting the change in absorbance (Delta OD/min) versus the log of compound concentration followed by 3-parameter fitting.

pEC₅₀ is the negative log of the EC₅₀.

All examples described herein possessed NQO1 specific enzyme activity in BEAS-2B cells with EC₅₀S between >10µM -<1nM unless otherwise noted (see table below). EC₅₀S <1nM (+++++), EC₅₀s 10nM-1nM (++++), EC₅₀S 10-100nM (+++), EC₅₀S 100nM-1µM (++), EC₅₀S 1-10µM (+).

| **Ex#** | **EC₅₀** | **Ex#** | **EC₅₀** | **Ex#** | **EC₅₀** |
|---|---|---|---|---|---|
| 1 | ++++ | 7 | ++++ | 13 | ++ |
| 2 | ++++ | 8 | ++++ | 14 | ++ |
| 3 | ++ | 9 | +++ | 15 | +++ |
| 4 | ++++ | 10 | +++++ | 16 | +++++ |
| 5 | ++++ | 11 | ++++ | | |
| 6 | ++++ | 12 | +++ | | |

### Methods of Use

The compounds of Formula (I) are useful in treating respiratory and non-respiratory disorders, including COPD, asthma, ALI, ARDS, fibrosis, chronic asthma, acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodeling, cardiac arrhythmias, heart failure with preserved ejection fraction, heart failure with reduced ejection fraction, diabetic cardiomyopathy, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye conditions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness, said disorders are treated by administering to a human in need thereof, a compound of Formula (I). Accordingly, in another aspect the invention is directed to methods of treating such conditions.

In one embodiment, the compounds of Formula (I) are useful in treating respiratory disorders including COPD, asthma, including chronic asthma and acute asthma.

In one embodiment, the compounds of Formula (I) are useful in treating hypertension, heart failure, acute coronary syndrome, myocardial infarction, myocardial repair, cardiac remodeling, cardiac arrhythmias, heart failure with preserved ejection fraction, heart failure with reduced ejection fraction and diabetic cardiomyopathy.

The methods of treatment of the invention comprise administering a safe and effective amount of a compound according to Formula I or a pharmaceutically-acceptable salt thereof to a patient in need thereof.

As used herein, "treat" in reference to a condition means: (1) to ameliorate or prevent the condition or one or more of the biological manifestations of the condition, (2) to interfere with (a) one or more points in the biological cascade that leads to or is responsible for the condition or (b) one or more of the biological manifestations of the condition, (3) to alleviate one or more of the symptoms or effects associated with the condition, or (4) to slow the progression of the condition or one or more of the biological manifestations of the condition.

The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof.

As used herein, "safe and effective amount" in reference to a compound of the invention or other pharmaceutically-active agent means an amount of the compound sufficient to treat the patient's condition but low enough to avoid serious side effects (at a reasonable benefit/risk ratio) within the scope of sound medical judgment. A safe and effective amount of a compound will vary with the particular compound chosen (e.g. consider the potency, efficacy, and half-life of the compound); the route of administration chosen; the condition being treated; the severity of the condition being treated; the age, size, weight, and physical condition of the patient being treated; the medical history of the patient to be treated; the duration of the treatment; the nature of concurrent therapy; the desired therapeutic effect; and like factors, but can nevertheless be routinely determined by the skilled artisan.

As used herein, "patient" refers to a human or other animal.

The compounds of the invention may be administered by any suitable route of administration, including both systemic administration and topical administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Topical administration includes application to the skin as well as intraocular, otic, intravaginal, and intranasal administration.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

Typical daily dosages may vary depending upon the particular route of administration chosen. Typical dosages for oral administration range from 1 mg to 1000 mg per person per day. Preferred dosages are 1 - 500 mg once daily, more preferred is 1 - 100 mg per person per day. IV dosages range form 0.1-000mg/day, preferred is 0.1-500mg/day, and more preferred is 0.1-100mg/day. Inhaled daily dosages range from 10ug-10mg/day, with preferred 10ug-2mg/day, and more preferred 50uug-500ug/day.

Additionally, the compounds of the invention may be administered as prodrugs. As used herein, a "prodrug" of a compound of the invention is a functional derivative of the compound which, upon administration to a patient, eventually liberates the compound of the invention in vivo. Administration of a compound of the invention as a prodrug may enable the skilled artisan to do one or more of the following: (a) modify the onset of the compound in vivo; (b) modify the duration of action of the compound in vivo; (c) modify the transportation or distribution of the compound in vivo; (d) modify the solubility of the compound in vivo; and (e) overcome a side effect or other difficulty encountered with the compound. Typical functional derivatives used to prepare prodrugs include modifications of the compound that are chemically or enzymatically cleaved in vivo. Such modifications, which include the preparation of phosphates, amides, ethers, esters, thioesters, carbonates, and carbamates, are well known to those skilled in the art.

### Compositions

The compounds of the invention will normally, but not necessarily, be formulated into pharmaceutical compositions prior to administration to a patient. Accordingly, in another aspect the invention is directed to pharmaceutical compositions comprising a compound of the invention and one or more pharmaceutically-acceptable excipient.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from 1 mg to 1000 mg.

The pharmaceutical compositions of the invention typically contain one compound of the invention. However, in certain embodiments, the pharmaceutical compositions of the invention contain more than one compound of the invention. For example, in certain embodiments the pharmaceutical compositions of the invention contain two compounds of the invention. In addition, the pharmaceutical compositions of the invention may optionally further comprise one or more additional pharmaceutically active compounds.

As used herein, "pharmaceutically-acceptable excipient" means a pharmaceutically acceptable material, composition or vehicle involved in giving form or consistency to the pharmaceutical composition. Each excipient must be compatible with the other ingredients of the pharmaceutical composition when commingled such that interactions which would substantially reduce the efficacy of the compound of the invention when administered to a patient and interactions which would result in pharmaceutical compositions that are not pharmaceutically acceptable are avoided. In addition, each excipient must of course be of sufficiently high purity to render it pharmaceutically-acceptable.

The compound of the invention and the pharmaceutically-acceptable excipient or excipients will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as dry powders, aerosols, suspensions, and solutions; and (6) topical administration such as creams, ointments, lotions, solutions, pastes, sprays, foams, and gels.

Suitable pharmaceutically-acceptable excipients will vary depending upon the particular dosage form chosen. In addition, suitable pharmaceutically-acceptable excipients may be chosen for a particular function that they may serve in the composition. For example, certain pharmaceutically-acceptable excipients may be chosen for their ability to facilitate the production of uniform dosage forms. Certain pharmaceutically-acceptable excipients may be chosen for their ability to facilitate the production of stable dosage forms. Certain pharmaceutically-acceptable excipients may be chosen for their ability to facilitate the carrying or transporting of the compound or compounds of the invention once administered to the patient from one organ, or portion of the body, to another organ, or portion of the body. Certain pharmaceutically-acceptable excipients may be chosen for their ability to enhance patient compliance.

Suitable pharmaceutically-acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, hemectants, chelating agents, plasticizers, viscosity increasing agents, antioxidants, preservatives, stabilizers, surfactants, and buffering agents. The skilled artisan will appreciate that certain pharmaceutically-acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically-acceptable excipients in appropriate amounts for use in the invention. In addition, there are a number of resources that are available to the skilled artisan which describe pharmaceutically-acceptable excipients and may be useful in selecting suitable pharmaceutically-acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid oral dosage form such as a tablet or capsule comprising a safe and effective amount of a compound of the invention and a diluent or filler. Suitable diluents and fillers include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesium stearate, calcium stearate, and talc.

In another aspect, the invention is directed to a dosage form adapted for administration to a patient parenterally including subcutaneous, intramuscular, intravenous or intradermal. Pharmaceutical formulations adapted for parenteral administration include aqueous and non-aqueous sterile injection solutions which may contain anti-oxidants, buffers, bacteriostats, and solutes that render the formulation isotonic with the blood of the intended recipient; and aqueous and non-aqueous sterile suspensions which may include suspending agents and thickening agents. The formulations may be presented in unit-dose or multi-dose containers, for example sealed ampules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

In another aspect, the invention is directed to a dosage form adapted for administration to a patient by inhalation. For example, the compound of the invention may be inhaled into the lungs as a dry powder, an aerosol, a suspension, or a solution.

Dry powder compositions for delivery to the lung by inhalation typically comprise a compound of the invention as a finely divided powder together with one or more pharmaceutically acceptable excipients as finely divided powders. Pharmaceutically acceptable excipients particularly suited for use in dry powders are known to those skilled in the art and include lactose, starch, mannitol, and mono-, di-, and polysaccharides.

The dry powder compositions for use in accordance with the present invention are administered via inhalation devices. As an example, such devices can encompass capsules and cartridges of for example gelatin, or blisters of, for example, laminated aluminum foil. In various embodiments, each capsule, cartridge or blister may contain doses of composition according to the teachings presented herein. Examples of inhalation devices can include those intended for unit dose or multi-dose delivery of composition, including all of the devices set forth herein. As an example, in the case of multi-dose delivery, the formulation can be pre-metered (e.g., as in Diskus®, see GB2242134, U.S. Patent Nos. 6,032,666, 5,860,419, 5,873,360, 5,590,645, 6,378,519 and 6,536,427 or Diskhaler, see GB 2178965, 2129691 and 2169265, US Pat. Nos. 4,778,054, 4,811,731, 5,035,237) or metered in use (e.g. as in Turbuhaler, see EP 69715, or in the devices described in U.S. Patent No 6,321,747). An example of a unit-dose device is Rotahaler (see GB 2064336). In one embodiment, the Diskus® inhalation device comprises an elongate strip formed from a base sheet having a plurality of recesses spaced along its length and a lid sheet peelably sealed thereto to define a plurality of containers, each container having therein an inhalable formulation containing the compound optionally with other excipients and additive taught herein. The peelable seal is an engineered seal, and in one embodiment the engineered seal is a hermetic seal. Preferably, the strip is sufficiently flexible to be wound into a roll. The lid sheet and base sheet will preferably have leading end portions which are not sealed to one another and at least one of the leading end portions is constructed to be attached to a winding means. Also, preferably the engineered seal between the base and lid sheets extends over their whole width. The lid sheet may preferably be peeled from the base sheet in a longitudinal direction from a first end of the base sheet.

A dry powder composition may also be presented in an inhalation device which permits separate containment of two different components of the composition. Thus, for example, these components are administrable simultaneously but are stored separately, e.g., in separate pharmaceutical compositions, for example as described in WO 03/061743 A1 WO 2007/012871 A1 and/or WO2007/068896, as well as U.S. Patent Nos. 8,113,199, 8,161,968, 8,511,304, 8,534,281, 8,746,242 and 9,333,310.

In one embodiment an inhalation device permitting separate containment of components is an inhaler device having two peelable blister strips, each strip containing pre-metered doses in blister pockets arranged along its length, e.g., multiple containers within each blister strip, e.g., as found in ELLIPTA®. Said device has an internal indexing mechanism which, each time the device is actuated, peels opens a pocket of each strip and positions the blisters so that each newly exposed dose of each strip is adjacent to the manifold which communicates with the mouthpiece of the device. When the patient inhales at the mouthpiece, each dose is simultaneously drawn out of its associated pocket into the manifold and entrained via the mouthpiece into the patient's respiratory tract. A further device that permits separate containment of different components is DUOHALER™ of Innovata. In addition, various structures of inhalation devices provide for the sequential or separate delivery of the pharmaceutical composition(s) from the device, in addition to simultaneous delivery.

Aerosols may be formed by suspending or dissolving a compound of the invention in a liquefied propellant. Suitable propellants include halocarbons, hydrocarbons, and other liquefied gases. Representative propellants include: trichlorofluoromethane (propellant 11), dichlorofluoromethane (propellant 12), dichlorotetrafluoroethane (propellant 114), tetrafluoroethane (HFA-134a), 1,1-difluoroethane (HFA-152a), difluoromethane (HFA-32), pentafluoroethane (HFA-12), heptafluoropropane (HFA- 227a), perfluoropropane, perfluorobutane, perfluoropentane, butane, isobutane, and pentane. Aerosols comprising a compound of the invention will typically be administered to a patient *via* a metered dose inhaler (MDI). Such devices are known to those skilled in the art.

The aerosol may contain additional pharmaceutically acceptable excipients typically used with multiple dose inhalers such as surfactants, lubricants, cosolvents and other excipients to improve the physical stability of the formulation, to improve valve performance, to improve solubility, or to improve taste.

Suspensions and solutions comprising a compound of the invention may also be administered to a patient *via* a nebulizer. The solvent or suspension agent utilized for nebulization may be any pharmaceutically acceptable liquid such as water, aqueous saline, alcohols or glycols, e.g., ethanol, isopropyl alcohol, glycerol, propylene glycol, polyethylene glycol, etc. or mixtures thereof. Saline solutions utilize salts which display little or no pharmacological activity after administration. Both organic salts, such as alkali metal or ammonium halogen salts, e.g., sodium chloride, potassium chloride or organic salts, such as potassium, sodium and ammonium salts or organic acids, e.g., ascorbic acid, citric acid, acetic acid, tartaric acid, etc. may be used for this purpose.

Other pharmaceutically acceptable excipients may be added to the suspension or solution. The compound of the invention may be stabilized by the addition of an inorganic acid, e.g., hydrochloric acid, nitric acid, sulfuric acid and/or phosphoric acid; an organic acid, e.g., ascorbic acid, citric acid, acetic acid, and tartaric acid, etc., a complexing agent such as EDTA or citric acid and salts thereof; or an antioxidant such as antioxidant such as vitamin E or ascorbic acid. These may be used alone or together to stabilize the compound of the invention. Preservatives may be added such as benzalkonium chloride or benzoic acid and salts thereof. Surfactant may be added particularly to improve the physical stability of suspensions. These include lecithin, disodium dioctylsulphosuccinate, oleic acid and sorbitan esters.

The compounds of Formula (I) and pharmaceutically acceptable salts thereof may be used in combination with one or more other agents which may be useful in the prevention or treatment of allergic disease, inflammatory disease, autoimmune disease, for example; antigen immunotherapy, anti-histamines, corticosteroids, (e.g., fluticasone propionate, fluticasone furoate, beclomethasone dipropionate, budesonide, ciclesonide, mometasone furoate, triamcinolone, flunisolide), NSAIDs, leukotriene modulators (e.g., montelukast, zafirlukast, pranlukast), iNOS inhibitors, tryptase inhibitors, IKK2 inhibitors, p38 inhibitors, Syk inhibitors, protease inhibitors such as elastase inhibitors, integrin antagonists (e.g., beta-2 integrin antagonists), adenosine A2a agonists, mediator release inhibitors such as sodium chromoglycate, 5-lipoxygenase inhibitors (zyflo), DP1 antagonists, DP2 antagonists, PI3K delta inhibitors, ITK inhibitors, LP (lysophosphatidic) inhibitors or FLAP (5-lipoxygenase activating protein) inhibitors (e.g., sodium 3-(3-(tert-butylthio)-1-(4-(6-ethoxypyridin-3-yl)benzyl)-5-((5-methylpyridin-2-yl)methoxy)-1H-indol-2-yl)-2,2-dimethylpropanoate), bronchodilators (e.g., muscarinic antagonists, beta-2 agonists), methotrexate, and similar agents; monoclonal antibody therapy such as anti-IgE, anti-TNF, anti-IL-5, anti-IL-6, anti-IL-12, anti-IL-1 and similar agents; cytokine receptor therapies e.g. etanercept and similar agents; antigen non-specific immunotherapies (e.g. interferon or other cytokines/chemokines, chemokine receptor modulators such as CCR3, CCR4 or CXCR2 antagonists, other cytokine/chemokine agonists or antagonists, TLR agonists and similar agents).

Suitably, for the treatment of asthma, compounds or pharmaceutical formulations of the invention may be administered together with an anti-inflammatory agent such as, for example, a corticosteroid, or a pharmaceutical formulation thereof. For example, a compound of the invention may be formulated together with an anti-inflammatory agent, such as a corticosteroid, in a single formulation, such as a dry powder formulation for inhalation. Alternatively, a pharmaceutical formulation comprising a compound of the invention may be administered in conjunction with a pharmaceutical formulation comprising an anti-inflammatory agent, such as a corticosteroid, either simultaneously or sequentially. In one embodiment, a pharmaceutical formulation comprising a compound of the invention and a pharmaceutical formulation comprising an anti-inflammatory agent, such as a corticosteroid, may each be held in device suitable for the simultaneous administration of both formulations via inhalation.

Suitable corticosteroids for administration together with a compound of the invention include, but are not limited to, fluticasone furoate, fluticasone propionate, beclomethasone diproprionate, budesonide, ciclesonide, mometasone furoate, triamcinolone, flunisolide and prednisilone. In one embodiment of the invention a corticosteroids for administration together with a compound of the invention via inhalation includes fluticasone furoate, fluticasone propionate, beclomethasone diproprionate, budesonide, ciclesonide, mometasone furoate, and, flunisolide.

Suitably, for the treatment of COPD, compounds or pharmaceutical formulations of the invention may be administered together with one or more bronchodilators, or pharmaceutical formulations thereof. For example, a compound of the invention may be formulated together with one or more bronchodilators in a single formulation, such as a dry powder formulation for inhalation. Alternatively, a pharmaceutical formulation comprising a compound of the invention may be administered in conjunction with a pharmaceutical formulation comprising one or more bronchodilators, either simultaneously or sequentially. In a further alternative, a formulation comprising a compound of the invention and a bronchodilator may be administered in conjunction with a pharmaceutical formulation comprising a further bronchodilator. In one embodiment, a pharmaceutical formulation comprising a compound of the invention and a pharmaceutical formulation comprising one or more bronchodilators may each be held in device suitable for the simultaneous administration of both formulations via inhalation. In a further embodiment, a pharmaceutical formulation comprising a compound of the invention together with a bronchodilator and a pharmaceutical formulation comprising a further bronchodilator may each be held in one or more devices suitable for the simultaneous administration of both formulations via inhalation.

Suitable bronchodilators for administration together with a compound of the invention include, but are not limited to, β₂-adrenoreceptor agonists and anticholinergic agents. Examples of β₂-adrenoreceptor agonists, include, for example, vilanterol, salmeterol, salbutamol, formoterol, salmefamol, fenoterol carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulphate salt of salbutamol or the fumarate salt of formoterol. Suitable anticholinergic agents include umeclidinium (for example, as the bromide), ipratropium (for example, as the bromide), oxitropium (for example, as the bromide) and tiotropium (for example, as the bromide). In one embodiment of the invention, a compound of the invention may be administered together with a β₂-adrenoreceptor agonist, such as vilanterol, and an anticholinergic agent, such as, umeclidinium.

The compounds may also be used in combination with agents for aiding transplantation including Cyclosporines, Tacrolimus, Mycophenolate mofetil, Prednisone, Azathioprine , Sirolimus, Daclizumab, Basiliximab and OKT3.

They may also be used in combination with agents for Diabetes: metformin (biguanides), meglitinides, sulfonylureas, DPP-4 inhibitors, Thiazolidinediones, Alpha-glucosidase inhibitors, Amylin mimetics, Incretin mimetics and insulin.

The compounds may be used in combination with antihypertensives such as diuretics, ACE inhibitors, ARBS, calcium channel blockers, and beta blockers.

One embodiment disclosed herein encompasses combinations comprising one or two other therapeutic agents. It will be clear to a person skilled in the art that, where appropriate, the other therapeutic ingredient(s) may be used in the form of salts, for example as alkali metal or amine salts or as acid addition salts, or prodrugs, or as esters, for example lower alkyl esters, or as solvates, for example hydrates to optimize the activity and/or stability and/or physical characteristics, such as solubility, of the therapeutic ingredient. It will be clear also that, where appropriate, the therapeutic ingredients may be used in optically pure form.

The combinations referred to above may conveniently be presented for use in the form of a pharmaceutical formulation and thus pharmaceutical formulations comprising a combination as defined above together with a pharmaceutically acceptable diluent or carrier represent a further aspect of the invention.

The individual compounds of such combinations may be administered either sequentially or simultaneously in separate or combined pharmaceutical formulations. In one embodiment, the individual compounds will be administered simultaneously in a combined pharmaceutical formulation. Appropriate doses of known therapeutic agents will readily be appreciated by those skilled in the art.

Thus, also disclosed herein is a pharmaceutical composition comprising a combination of a compound of the invention together with another therapeutically active agent.

### EXAMPLES

The following examples illustrate the invention. These examples are not intended to limit the scope of the present invention, but rather to provide guidance to the skilled artisan to prepare and use the compounds, compositions, and methods of the present invention.

All temperatures are given in degrees Celsius, all solvents are highest available purity and all reactions run under anhydrous conditions in an argon (Ar) or nitrogen (N₂) atmosphere where necessary.

Analtech Silica Gel GF and E. Merck Silica Gel 60 F-254 thin layer plates were used for thin layer chromatography. Both flash and gravity chromatography were carried out on E. Merck Kieselgel 60 (230-400 mesh) silica gel. The CombiFlash® system used for purification in this application was purchased from Isco, Inc. CombiFlash® purification was carried out using prepacked silica gel columns, a detector with UV wavelength at 254 nm and a variety of solvents or solvent combinations.

Preparative HPLC was performed using a Gilson Preparative System with variable wavelength UV detection or an Agilent Mass Directed AutoPrep (MDAP) system with both mass and variable wavelength UV detection or Waters Preparative System with UV / PDA detection or an Shimadzu PREP LC 20AP. A variety of reverse phase columns, e.g., Luna 5m C18(2) 100A, SunFire C18, XBridge C18, Atlantics T3 were used in the purification with the choice of column support dependent upon the conditions used in the purification. The compounds are eluted using a gradient of CH₃CN and water. Neutral conditions used an CH₃CN and water gradient with no additional modifier, acidic conditions used an acid modifier, 0.1% TFA (added to both the CH₃CN and water) or 0.1 % formic acid and basic conditions used a basic modifier, 0.1% NH₄OH (added to the water) or 10 mM ammonium bicarbonate.

Analytical HPLC was run using an Agilent system, Shimadzu/Sciex LCMS with variable wavelength UV detection using reverse phase chromatography with a CH₃CN and water gradient with a 0.02 or 0.1% TFA modifier (added to each solvent). LC-MS was determined using either a PE Sciex Single Quadrupole 150EX LC-MS, or Waters ZQ Single Quadrupole LC-MS or Agilent 1200 series SL (dectectors: Agilent 6140 single quadrupole and Agilent 1200 MWD SL) instruments. The compound is analyzed using a reverse phase column, e.g., Thermo Hypersil Gold C18, eluted using a gradient of CH₃CN and water with a low percentage of an acid modifier such as 0.02% TFA or 0.1 % formic acid or a base modifier such as 5mM ammonium bicarbonate (adjusted to pH 10 with aqueous ammonia). When specified "acid method" refers to 0.1 % formic acid in water and CH₃CN gradient (1.8 min. 0.9 mL/min flow) with a Waters Acquity UPLC HSS C18; 1.8µ; 2.1x50mm at 50 °C; "basic method" refers to 95:5 H₂O+0.1% NH₄OH:CH₃CN (pH = 9.4) and water gradient (1.8 min. 0.9 mL/min flow) with a Waters Acquity UPLC BEH C18; 1.7µ; 2.1×50mm at 50 °C and "overnight basic method" refers to 95:5 H₂O+0.1% NH₄OH:CH₃CN (pH = 9.4) and water gradient (16 min. 0.8 mL/min flow) with a Waters Acquity UPLC BEH C18; 1.7µ; 2.1x50mm at 50 °C.

Preparative Chiral SFC was performed using a Thar/Waters Preparative SFC System with single wavelength UV detection system or PDA detector. A variety of chiral SFC columns, e.g. Chiralpak IA, IC, AY, AD. OD, OJ, C2 were used in the purification. The compounds are eluted using supercritical fluid CO₂ and co-solvents, such as MeOH, EtOH, IPA, and combination of these solvent in different ratio based on the compound selectivity. Modifiers (0.1% of TFA, NH₄OH, DEA) would be used as needed.

Analytical Chiral SFC was run using a Thar/Waters SFC system with variable wavelength UV detection or PDA detector. A variety of chiral SFC columns, e.g. Chiralpak IA, IB, IC, ID, AY, AD, AS, CCL4 were used in the purification. The compounds are eluted using supercritical fluid CO₂ and co-solvents, such as MeOH, EtOH, IPA, and combination of these solvent in different ratio based on the compound selectivity. Modifiers (0.1% of TFA, NH₄OH, DEA) would be used as needed.

Celite® is a filter aid composed of acid-washed diatomaceous silica, and is a registered trademark of Manville Corp., Denver, Colorado. Isolute® is a functionalized silica gel based sorbent, and is a registered trademark of Biotage AB Corp., Sweden.

Nuclear magnetic resonance spectra were recorded at 400 MHz using a Bruker AVANCE 400 or Brucker DPX400 or Varian MR400 400 MHz spectrometer. CDCl₃ is deuteriochloroform, DMSO-D₆ is hexadeuteriodimethylsulfoxide, and MeOD is tetradeuteriomethanol, CD₂Cl₂ is deuteriodichloromethane. Chemical shifts are reported in parts per million (δ) downfield from the internal standard tetramethylsilane (TMS) or calibrated to the residual proton signal in the NMR solvent (e.g., CHCl₃ in CDCl₃). Abbreviations for NMR data are as follows: s = singlet, d = doublet, t = triplet, q = quartet, m = multiplet, dd = doublet of doublets, dt = doublet of triplets, app = apparent, br = broad. J indicates the NMR coupling constant measured in Hertz.

Heating of reaction mixtures with microwave irradiations was carried out on a Biotage Initiator® or CEM microwave reactor, typically employing the high absorbance setting.

Cartridges or columns containing polymer based functional groups (acid, base, metal chelators, etc) can be used as part of compound workup. The "amine" columns or cartridges are used to neutralize or basify acidic reaction mixtures or products. These include NH₂ Aminopropyl SPE-ed SPE Cartridges available from Applied Separations and diethylamino SPE cartridges available from United Chemical Technologies, Inc.

**Table of Abbreviations**

| |
|---|
| [Rh(cod)Cl]₂ or [RhCl(cod)]₂: di-µ-chlorido-bis[η²,η²-(cycloocta-1,5-diene)rhodium |
| ®T3P: 2,4,6-tripropyl-1,3,5,2,4,6-trioxatriphosphorinane 2,4,6-trioxide |
| °C: degree Celsius |
| AcOH: acetic acid |
| ADDP: (E)-diazene-1,2-d iylbis(piperid in-1-ylmethanone) |
| aq = aqueous |
| BINAP: 2,2'-bis(diphenylphosphino)-1,1'-binaphthalene |
| CDI: Carbonyl dimidazole |
| CH₂Cl₂: dichloromethane |
| CH₃CN: acetonitrile |
| CHCl₃: chloroform |
| CS₂CO₃: cesium carbonate |
| DBU: 1,8-diazabicyclo[5.4.0]undec-7-ene |
| DCE: dichloroethane |
| DCM: dichloromethane |
| DIPEA or DIEA: diisopropylethyl amine |
| DME: dimethyl ether |
| DMF: *N,N*-dimethylformamide |
| DMF-DMA or DMF-dimethyl acetal: N,N-dimethylformaide-dimethyl acetal |
| DMSO: dimethyl sulfoxide |
| EDC: 1-ethyl-3-(3-dimethylaminopropyl)carbodiimide |
| Et₂O: diethyl ether |
| Et₃N: triethylamine |
| EtOAc: ethyl acetate |
| EtOH: ethanol |
| g: gram(s) |
| h: hour(s) |
| HATU: O-(7-azabenzotriazol-1-yl)-N,N,N',N'-tetramethyluronium hexafluorophosphate |
| HBTU: N,N,N',N'-tetramethyl-O-(1H-benzotriazol-1-yl)uronium hexafluorophosphate |
| HCl: hydrochloric acid |
| HOAt: 1-hydroxy-7-azabenzotriazole |
| HPLC: high performance liquid chromatography |
| IPA: isopropyl alcohol |
| K₂CO₃: potassium carbonate |
| KOAc: potassium acetate |
| LAH: lithium aluminum hydride |
| LC: liquid chromatography |
| LC-MS: liquid chromatography-mass spectroscopy |
| LiBH₄: lithium borohydride |
| LiHMDS: lithium hexamethyldisilazane |
| LiOH: lithium hydroxide |
| M: molar |
| MeCN: acetonitrile |
| Mel: methyl iodide |
| MeOH: methanol |
| mg: milligram(s) |
| MgCl₂: magnesium chloride |
| MgSO₄: magnesium sulfate |
| MHz: megahertz |
| min: minute(s) |
| mL: milliliter(s) |
| mmol: millimole(s) |
| MS: mass spectroscopy |
| N₂: nitrogen gas |
| Na₂CO₃: sodium carbonate |
| Na₂SO₄: sodium sulfate |
| NaBH₃CN or NaCNBH₃: sodium cyanoborohydride |
| NaCl: sodium chloride |
| NaH: sodium hydride |
| NaHCO₃: sodium bicarbonate |
| NaHMDS: sodium hexamethyldisilazane |
| NaHSO₄: sodium bisulfate |
| NaOAc: sodium acetate |
| NaOH: sodium hydroxide |
| NBS: N-bromosuccinimide |
| nBuLi: n-butyl lithium |
| NH₄Cl: ammonium chloride |
| NMR: nuclear magnetic resonance |
| P(tBu)₃: tri-t-butyl phosphine |
| Pd(PhP₃)₄: tetrakistriphenylphosphine palladium |
| Pd/C: pallidium on carbon |
| Pd₂(dba)₃: *tris*(dibenzylideneacetone)-dipalladium(0) |
| PdCl₂(dppf) or Pd(dppf)Cl₂ : [1,1'-*bis*(diphenylphosphino)-ferrocene] dichloropalladium(II) |
| Petrol: petroleum ether |
| PS-PPh₃: polymer supported triphenylphosphine |
| PtO₂: platinum(IV) oxide |
| RT: room temperature |
| T3P: 2,4,6-tripropyl-1,3,5,2,4,6-trioxatriphosphorinane-2,4,6-trioxide solution |
| TEA: triethylamine |
| TFA: trifluoroacetic acid |
| TFFH: tetrafluoroformamidinium hexafluorophosphate |
| THF: tetrahydrofuran |
| triflic anhydride: trifluoromethanesulfonic anhydride |
| TsOH: p-toluenesulfonic acid |
| wt%: weight percent |

### Example 1

### (S)-3-(1,4-dimethyl-1H-benzo[d][1,2,3]triazol-5-yl)-2,2-dimethyl-3-(4-methyl-3-((4-methyl-3-oxo-1,4-(S)-3-(3-((4-cyclopropyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

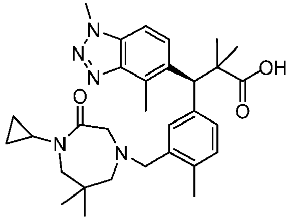

### 3-Methyl-2-nitrobenzamide

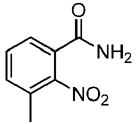

To a solution of 3-methyl-2-nitrobenzoic acid (100 g, 552 mmol) in dichloromethane (DCM) (1000 mL), oxalyl chloride (72.5 mL, 828 mmol) was added at 25 °C. The reaction mixture was stirred at ambient temperature for 1 h. The solvent was removed under reduced pressure. The residue was dissolved in CH₂Cl₂ (100mL). The solvent was added to ammonium hydroxide (1000 mL, 7704 mmol) at ambient temperature and was stirred for 30 minutes. Then the reaction mixture was extracted with ethyl acetate (3×500 mL). The combined organic layer was dried over MgSO₄ and concentrated to give 67 g (60.6%) of the title compound. LC-MS *m*/*z* 181.1 (M+H)⁺, 1.40 (ret. time).

### 3-Methyl-2-nitroaniline

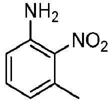

To a mixture of NaOH (2.220 g, 55.5 mmol) in water (12 mL), Br₂ (0.322 mL, 6.26 mmol) was added at 0°C. Then 3-methyl-2-nitrobenzamide (1g, 5.55 mmol) was added in one portion, and the mixture was warmed slowly in a water bath. The material soon turned dark in color, and at 50-55° C (internal temperature) oil droplets began to separate. The temperature was raised gradually to 70° and maintained at this point for one hour. A solution of 0.7 g. of sodium hydroxide in 4 mL. of water was added slowly and the temperature was increased to 80°C for an additional hour. The reaction was cooled to ambient temperature and extracted with ethyl acetate (3 x 50 mL). The combined organic layer was dried and concentrated to give 0.7 g (90%) of the title compound. LC-MS *m*/*z* 153.1 (M+H)⁺, 1.65 (ret. time).

### 4-bromo-3-methyl-2-nitroaniline

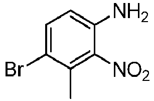

A mixture of NBS (51.5 g, 289 mmol), 3-methyl-2-nitroaniline (44 g, 289 mmol) and acetic acid (450 mL) was stirred at 110 °C for 1 h. The mixture was cooled to ambient temperature and poured into water (100 mL). The solid was collected to give 55 g (78%) of the title compound. LC-MS *m*/*z* 230.9 (M+H)⁺, 1.78 (ret. time).

### 4-Bromo-N,3-dimethyl-2-nitroaniline

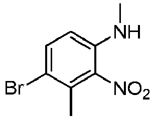

To a solution of 4-bromo-3-methyl-2-nitroaniline (20g, 87 mmol) in N,N-dimethylformamide (DMF) (200 mL), NaH (3.81 g, 95 mmol) was added at 25 °C. The reaction mixture was stirred at 25 °C for 30 minutes. Then iodomethane (12.90 g, 91 mmol) was added. The reaction mixture was stirred for 12 h. The reaction mixture was poured into water and the solid was collected to give 18 g (59.4%) of the title compound. LC-MS *m*/*z* 247.0 (M+H)⁺, 1.90 (ret. time).

### 4-Bromo-N¹,3-dimethylbenzene-1,2-diamine

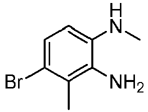

To a solution of 4-Bromo-N,3-dimethyl-2-nitroaniline (30 g, 122 mmol) in ethanol (600 mL), tin(II) chloride (93 g, 490 mmol), was added. The reaction mixture was stirred at 75 °C for 2 h. Then the solvent was adjusted to pH=14 by using 40% NaOH. It was extracted with ethyl acetate (3 x 500 mL). The combined organic layer was dried over MgSO₄ and concentrated to give 26 g (39.5%) of the title compound.

### 5-bromo-1,4-dimethyl-1H-benzo[d][1,2,3]triazole

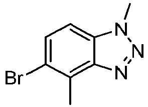

To 4-bromo-N¹,3-dimethylbenzene-1,2-diamine (30 g, 139 mmol) in 17 ml of 10 % H₂SO₄ at 0 °C, sodium nitrite (13.47 g, 195 mmol) was added in small portions over a 20 minute period. After the reaction mixture was stirred for 30 minutes further, 200 mL of water was added. The resulting precipitate was collected by filtration, washed with water and dried. The mother liquor was left to stand 16 h and a second batch of precipitate formed, which was collected as before. The combined solids were purified by silica gel chromatographyy eluting with ethyl acetate to give the title compound 10 g (21.57%). LC-MS *m*/*z* 226.0 (M+H)⁺, 1.71 (ret. time).

### (1,4-Dimethyl-1H-benzo[d][1,2,3]triazol-5-yl)(3-(((4-methoxybenzyl)oxy)methyl)-4-methylphenyl)methanol

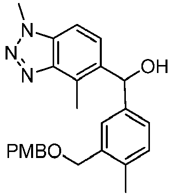

Tert-butyllithium (19.52 mL, 33.2 mmol) was added dropwise to a solution of 5-bromo-1,4-dimethyl-1H-benzo[d][1,2,3]triazole (3.91 g, 17.31 mmol) in tetrahydrofuran (THF) (108 ml) at -78 °C under a nitrogen atmosphere and stirred for 30 minutes. A solution of 3-(((4-methoxybenzyl)oxy)methyl)-4-methylbenzaldehyde (3.9 g, 14.43 mmol) in tetrahydrofuran (THF) (36.1 ml) was then added dropwise and stirred at -78 °C for 1.5 hours followed by warming to ambient temperature and stirring for an additional hour. Saturated aqueous ammonium chloride (100 mL) was added to the solution. Same scale reaction was run side by side. The 2 batches were combined and the mixture was extracted with ethyl acetate (3 x 100 mL). The combined organic layer was washed (brine), dried (sodium sulfate), filtered and the solvent was concentrated. The crude product was purified by silica gel chromatographyy to give title compound (8.8 g, 21.08 mmol, 73.0 % yield). LC/MS: m/z 418.0 (M+H)⁺, 1.11 min (ret. time). 1H NMR (400MHz, CHLOROFORM-d) δ = 7.76 - 7.69 (m, 1H), 7.34 (s, 2H), 7.24 (d, J=8.3 Hz, 2H), 7.21 - 7.12 (m, 2H), 6.87 (d, J=8.3 Hz, 2H), 6.27 (s, 1H), 4.49 (d, J=5.0 Hz, 4H), 4.29 (s, 3H), 3.83 (s, 3H), 2.81 (s, 3H), 2.31 (s, 3H), 2.07 (s, 1H)

### Methyl 3-(1,4-dimethyl-1H-benzo[d][1,2,3]triazol-5-yl)-3-(3-(hydroxymethyl)-4-methylphenyl)-2,2-dimethylpropanoate

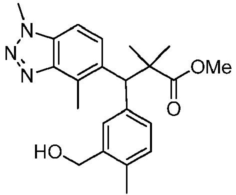

2,2,2-trichloroacetonitrile (4.23 ml, 42.2 mmol) and DBU (0.146 ml, 1.054 mmol) were added sequentially to a solution of (1,4-dimethyl-1H-benzo[d][1,2,3]triazol-5-yl)(3-(((4-methoxybenzyl)oxy)methyl)-4-methylphenyl)methanol (8.8 g, 21.08 mmol) in acetonitrile (263 ml) at ambient temperature and stirred for 45 minutes. ((1-Methoxy-2-methylprop-1-en-1-yl)oxy)trimethylsilane (9.19 g, 52.7 mmol) followed by 1,1,1-trifluoro-N-((trifluoromethyl)sulfonyl)methanesulfonamide (0.593 g, 2.108 mmol) were then added and the solution stirred at ambient temperature for 2 h. The reaction was quenched with saturated sodium bicarbonate (10 mL) and extracted with DCM (3 x 15 mL), dried over sodium sulfate, filtered and concentrated. The residue was re-dissolved in dichloromethane (DCM) (263 mL). Water (15.19 mL, 843 mmol) was added and the solution was cooled to 0 °C, 4,5-dichloro-3,6-dioxocyclohexa-1,4-diene-1,2-dicarbonitrile (9.57 g, 42.2 mmol) was added. The solution was stirred at 0 °C for 1 h. The reaction was quenched with saturated sodium bicarbonate (10 mL) and extracted with DCM (3 x 15 mL) and dried over sodium sulfate. The crude product was purified by flash chromatography on a silica gel chromatographyy to give the title compound (6.9 g, 18.09 mmol, 86 % yield). LC/MS: m/z 382.0 (M+H)⁺, 1.00 min (ret. time).

### (S)-Methyl 3-(1,4-dimethyl-1H-benzo[d][1,2,3]triazol-5-yl)-3-(3-(hydroxymethyl)-4-methylphenyl)-2,2-dimethylpropanoate and (R)-methyl 3-(1,4-dimethyl-1H-benzo[d][1,2,3]triazol-5-yl)-3-(3-(hydroxymethyl)-4-methylphenyl)-2,2-dimethylpropanoate

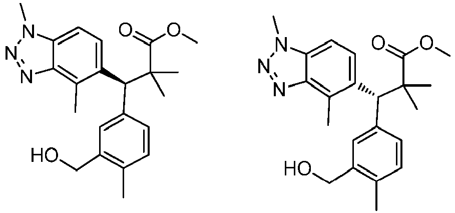

Methyl 3-(1,4-dimethyl-1H-benzo[d][1 ,2,3]triazol-5-yl)-3-(3-(hydroxymethyl)-4-methylphenyl)-2,2-dimethylpropanoate (4.5 g, 11.80 mmol) was separated by chiral SFC chromatography to obtain isomer 1-(S)-methyl 3-(1,4-dimethyl-1H-benzo[d][1,2,3]triazol-5-yl)-3-(3-(hydroxymethyl)-4-methylphenyl)-2,2-dimethylpropanoate (1.4 g, 3.49 mmol, 29.6 %) and isomer 2-(R)-methyl 3-(1,4-dimethyl-1H-benzo[d][1,2,3]triazol-5-yl)-3-(3-(hydroxymethyl)-4-methylphenyl)-2,2-dimethylpropanoate (1.1 g, 2.74 mmol, 23.22 %).

### (S)-Methyl 3-(3-(chloromethyl)-4-methylphenyl)-3-(1,4-dimethyl-1H-benzo[d][1,2,3]triazol-5-yl)-2,2-dimethylpropanoate

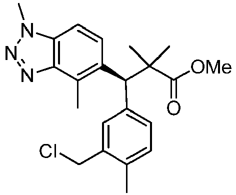

To a solution of (S)-methyl 3-(1,4-dimethyl-1H-benzo[d][1,2,3]triazol-5-yl)-3-(3-(hydroxymethyl)-4-methylphenyl)-2,2-dimethylpropanoate (710 mg, 1.861 mmol) in dichloromethane (10 mL) at ambient temperature was added thionyl chloride (0.272 mL, 3.72 mmol). The reaction was stirred for 40 min. The resulting mixture was concentrated to give the title compound (750 mg, 1.875 mmol, 101 % yield). LC-MS m/z 400.2 (M+H)⁺, 1.21 min (ret. time)

### tert-Butyl (3-hydroxy-2,2-dimethylpropyl)carbamate

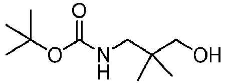

To a solution of 3-amino-2,2-dimethylpropan-1-ol (25 g, 242 mmol) in dichloromethane (DCM) (200 mL) was added di-tert-butyl dicarbonate (56.3 mL, 242 mmol) dropwise. It was stirred at ambient temperature for 4 h. The reaction mixture was filtered and the filtrate was concentrated. It was purified via flash column chromatography using 15 % ethyl acetate in n-hexane as solvent to give the title compound (35 g, 172 mmol, 71.0 % yield) as an off white solid. LCMS: m/z 204.00 (M+)⁺, 1.82 min (ret. time)

### tert-Butyl (2,2-dimethyl-3-oxopropyl)carbamate

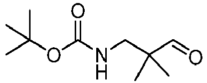

To a solution of tert-butyl (3-hydroxy-2,2-dimethylpropyl)carbamate (30 g, 148 mmol) in dichloromethane (DCM) (200 mL) was added TEMPO (3.46 g, 22.14 mmol) and KBr (18.45 mL, 36.9 mmol). The reaction mixture was cooled to -10 °C. In a separate flask, sodium bicarbonate (74.4 g, 885 mmol) was dissolved in sodium hypochlorite solution (250 mL, 365 mmol) to yield 0.78M buffered NaOCI solution. From this solution, 25 mL (1.5 eq.) was added to the reaction mixture and stirred for 16 h. The organic phase was separated and aqueous layer extracted with DCM (2 x 100 mL). The combined organic layer washed with brine solution (80 mL), dried over anhydrous Na₂SO₄, filtered and concentrated under reduced pressure to give the crude compound. It was purified with flash column chromatography eluting with 15 % ethyl acetate in n-hexane to give the title compound (17 g, 63.4 mmol, 42.9 % yield) as an off white solid. LCMS: m/z 202.17 (M+H)⁺,1.94 min (ret. time)

### tert-Butyl (3-(cyclopropylamino)-2,2-dimethylpropyl)carbamate

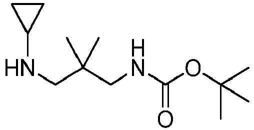

To a solution of tert-butyl (2,2-dimethyl-3-oxopropyl)carbamate (7 g, 34.8 mmol) in methanol (1.000 mL) and acetic acid (9 mL) at 0 °C was added cyclopropanamine (2.184 g, 38.3 mmol). The reaction mixture was stirred at 0 °C for 1 h. NaCNBH₄ (4.37 g, 69.6 mmol) was then added at 0 °C and stirred at ambient temperature for 16 h. The reaction was quenched with 10% NaHCO₃ solution (15 mL) and extracted with DCM (2 x 100 mL). The combined organic layer was washed with brine solution (60 mL), dried over anhydrous Na₂SO₄, filtered and concentrated. The crude residue was purified via flash column chromatography eluting with EtOAc:Hexane (4:6) to give the title compound (7 g, 21.78 mmol, 62.6 % yield). LCMS: m/z 243.1 (M+H)⁺, 5.64 min (ret. time)

### tert-Butyl (3-(2-chloro-N-cyclopropylacetamido)-2,2-dimethylpropyl)carbamate

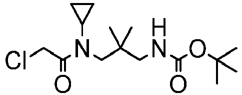

To a solution of tert-butyl (3-(cyclopropylamino)-2,2-dimethylpropyl)carbamate (15 g, 61.9 mmol) in dichloromethane (DCM) (100 mL) at 0 °C was added TEA (17.25 mL, 124 mmol) and chloroacetyl chloride (7.44 mL, 93 mmol). The reaction mixture was stirred at ambient temperature for 4 h. The reaction mixture was extracted with DCM (2 x 100 mL). The combined organic layer washed with brine solution (80 mL), dried over anhydrous Na₂SO₄, filtered and concentrated under reduced pressure to give the title compound (14 g, 34.8 mmol, 56.2 % yield) as a pale yellow liquid. LCMS: m/z 319.18 (M+H)⁺, 2.40 min (ret. time)

### tert-Butyl 4-cyclopropyl-6,6-dimethyl-3-oxo-1,4-diazepane-1 -carboxylate

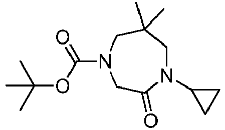

To a solution of tert-butyl (3-(2-chloro-N-cyclopropylacetamido)-2,2-dimethylpropyl)carbamate (14 g, 34.8 mmol) in N,N-dimethylformamide (DMF) (100 mL) at 0 °C was added sodium hydride (1.518 g, 34.8 mmol) portion wise. The reaction mixture was stirred at ambient temperature for 16 h. The reaction mixture was poured in ice-cold water and extracted with EtOAC (2 x 80 mL). The combined organic layer was washed with brine solution (50 mL), dried over anhydrous Na₂SO₄, filtered and concentrated to give the crude product which was purified via flash column chromatography eluting with EtOAc: Hexane (4:6) to give the title compound (7 g, 22.69 mmol, 65.2 % yield) as a pale yellow liquid. LCMS: m/z 283.24 (M+H)⁺, 2.12 min (ret. time)

### 1-Cyclopropyl-6,6-dimethyl-1,4-diazepan-2-one hydrochloride

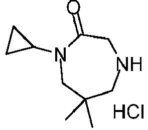

To a solution of tert-butyl 4-cyclopropyl-6,6-dimethyl-3-oxo-1,4-diazepane-1-carboxylate (14 g, 45.4 mmol) in 1,4-dioxane (5 mL) at 0 °C was added 4M HCI in 1,4 dioxane (30 ml, 120 mmol). The reaction mixture was stirred at ambient temperature for 2 h. The reaction mixture was filtered and washed with diethyl ether (10 ml) to give the title compound (3.285 g, 14.44 mmol, 31.8 % yield). LCMS: m/z 183.2 (M+H)⁺, 3.144 (ret. time)

### (S)-3-(1,4-Dimethyl-1H-benzo[d][1,2,3]triazol-5-yl)-2,2-dimethyl-3-(4-methyl-3-((4-methyl-3-oxo-1,4-(S)-3-(3-((4-cyclopropyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

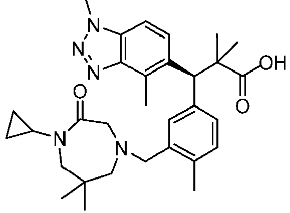

A mixture of (S)-methyl 3-(3-(chloromethyl)-4-methylphenyl)-3-(1,4-dimethyl-1H-benzo[d][1,2,3]triazol-5-yl)-2,2-dimethylpropanoate (70 mg, 0.175 mmol), 1-cyclopropyl-6,6-dimethyl-1,4-diazepan-2-one hydrochloride (38.3 mg, 0.175 mmol) and DIEA (0.122 mL, 0.700 mmol) in acetonitrile (3 mL) was heated in a Biotage microwave at high absorption for 1 h at 120 °C. The solvent was concentrated and the resulting residue was re-dissolved in methanol (3.00 mL), 2M LiOH (0.525 mL, 1.050 mmol) was added and the reaction heated in a Biotage microwave at high absorption for 1.5 h at 120 °C. The solvent was concentrated and the crude product was acidified with 1 N HCl to around pH 1, 1 mL DMSO was added and concentrated. The sample was filtered and purified with neutral preparative HPLC to give the title compound (59 mg, 0.111 mmol, 63.4 % yield) as white solid. LC-MS *m*/*z* 532.2 (M+H)⁺, 0.93 min (ret. time)

### Example 2 (S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((6,6-dimethyl-3-oxo-4-propyl-1,4-diazepan-1-yl)methyl)-4-methylphenyl)-2,2-dimethylpropanoic acid

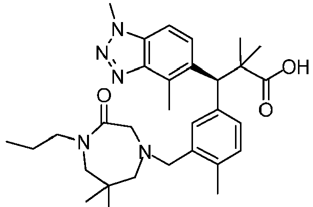

### tert-Butyl (2,2-dimethyl-3-(2-nitro-N-propylphenylsulfonamido)propyl)carbamate

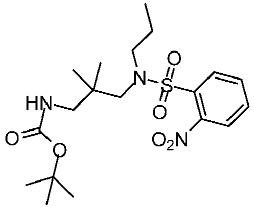

To a solution of tert-butyl (2,2-dimethyl-3-(2-nitrophenylsulfonamido)propyl)carbamate (15g, 38.7 mmol) in N,N-dimethylformamide (DMF) (150 mL) at 0°C was added potassium carbonate (8.03 g, 58.1 mmol) and 1-iodopropane (13.16 g, 77 mmol). The reaction mixture was stirred at ambient temperature for 16 h. The reaction was quenched with water (20 mL) and extracted with EtOAc (3 x 20 mL). The combined organic layer was washed with brine (20 mL), dried over anhydrous Na₂SO₄, filtered and concentrated to give the title compound (14g, 32.4 mmol, 84 % yield). LC-MS *m*/*z* 430.23 (M+H)⁺, 2.68 min (ret. time)

### tert-Butyl (2,2-dimethyl-3-(propylamino)propyl)carbamate

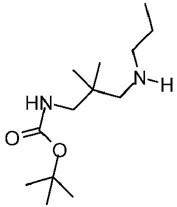

To a solution of tert-butyl (2,2-dimethyl-3-(2-nitro-N-propylphenylsulfonamido)propyl)carbamate (14g, 32.6 mmol) in N,N-dimethylformamide (DMF) (120 mL) at 0 °C was added potassium carbonate (6.76 g, 48.9 mmol) and thiophenol (10.07 mL, 98 mmol). The reaction mixture was stirred at ambient temperature for 2 h. The reaction was quenched with 1N HCl to pH =1 and then extracted with EtOAc (2 x 30 mL). The aqueous layer was basified with 1N NaOH to pH = 14 and then extracted with ethyl acetate twice. The combined organic layer was washed with cold water and brine (20 mL), dried over anhydrous Na₂SO₄, filtered and concentrated to give the title compound (7g, 28.6 mmol, 88 % yield). ¹H NMR (400 MHz, CDCl₃) δ ppm 0.87 - 0.95 (m, 9 H), 1.42 - 1.47 (m, 11 H), 2.42 (s, 2 H), 2.54 (t, *J* = 7.02 Hz, 2 H), 2.88-3.0 (m, 2 H), 5.93 (br s, 1 H)

### tert-Butyl (3-(2-chloro-N-propylacetamido)-2,2-dimethylpropyl)carbamate

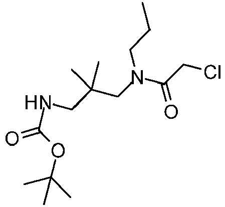

To a solution of tert-butyl (2,2-dimethyl-3-(propylamino)propyl)carbamate (7g, 28.6 mmol) in dichloromethane (DCM) (70 mL) at 0°C was added 2-chloroacetyl chloride (6.47 g, 57.3 mmol) and triethylamine (7.98 mL, 57.3 mmol). The reaction mixture was stirred at ambient temperature for 2 h. The reaction was quenched with water (15 mL) and extracted with DCM (2 x 10 mL). The combined organic layer was washed with brine (10 mL), dried over anhydrous Na₂SO₄, filtered and evaporated. It was purified with column chromatography eluting with 18 % EtOAc in pet ether to give the title compound (8g, 21.69 mmol, 76 % yield) as a brown liquid. LC-MS *m*/*z* 321.07 (M+H)⁺, 3.76 min (ret. time)

### tert-Butyl 6,6-dimethyl-3-oxo-4-propyl-1,4-diazepane-1-carboxylate

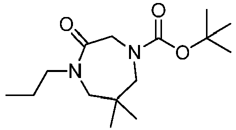

To a solution of tert-butyl (3-(2-chloro-N-propylacetamido)-2,2-dimethylpropyl)carbamate (7 g, 21.82 mmol) in N,N-dimethylformamide (DMF) (50 mL) at 0 °C was added NaH (2.86 g, 65.5 mmol). The reaction mixture was stirred at ambient temperature for 6 h. The reaction was quenched with cold water (30 mL) and extracted with EtOAc (2 x 40 mL). The combined organic layer was washed with brine (20 mL), dried over anhydrous Na₂SO₄, filtered and concentrated. It was purified with column chromatography eluting with 10% EtOAc in pet ether to give the title compound (3.8 g, 12.94 mmol, 59.3 % yield) as a brown liquid. LC-MS *m*/*z* 285.11 (M+H)⁺, 2.55 min (ret. time)

### 6,6-Dimethyl-1-propyl-1,4-diazepan-2-one hydrochloride

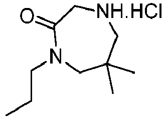

To a solution of tert-butyl 6,6-dimethyl-3-oxo-4-propyl-1,4-diazepane-1-carboxylate (4.3 g, 15.12 mmol) in 1,4-dioxane (30 mL) at 0°C was added 4M HCI in 1,4-dioxane (9.4 ml, 37.6 mmol). Then reaction mixture was stirred at ambient temperature for 2 h. The reaction mixture was concentrated. The crude compound was washed with diethyl ether to afford the title compound (2.4 g, 10.61 mmol, 70.2 % yield) as an off white solid. LC-MS *m*/*z* 185.03 (M+H)⁺, 3.19 min (ret. time)

### (S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((6,6-dimethyl-3-oxo-4-propyl-1,4-diazepan-1-yl)methyl)-4-methylphenyl)-2,2-dimethylpropanoic acid

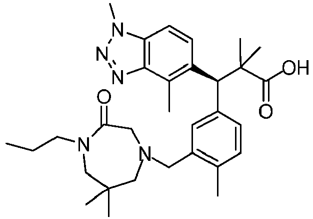

A mixture of (S)-methyl 3-(3-(chloromethyl)-4-methylphenyl)-3-(1,4-dimethyl-1H-benzo[d][1,2,3]triazol-5-yl)-2,2-dimethylpropanoate (70 mg, 0.175 mmol), 6,6-dimethyl-1-propyl-1,4-diazepan-2-one hydrochloride (38.6 mg, 0.175 mmol) and DIEA (0.122 mL, 0.700 mmol) in acetonitrile (3 mL) was heated in a Biotage microwave at high absorption for 1 h at 120 °C. The solvent was concentrated and the crude product was re-dissolved in methanol (3.00 mL), 2 M LiOH (0.525 mL, 1.050 mmol) was added and the reaction heated in a Biotage microwave at high absorption for 1.5 h at 120 °C. The solvent was concentrated and the crude product was acidified with 1 N HCl to around pH 1, 1 mL DMSO was added and concentrated. The sample was filtered and purified with neutral preparative HPLC to give the title compound (69 mg, 0.129 mmol, 73.9 % yield) as white solid. LC-MS *m*/*z* 534.2 (M+H)⁺, 0.97 min (ret. time)

### Example 3 (S)-3-(1,4-Dimethyl-1H-benzo[d][1,2,3]triazol-5-yl)-2,2-dimethyl-3-(4-methyl-3-((4-methyl-3-oxo-1,4-diazepan-1-yl)methyl)phenyl)propanoic acid

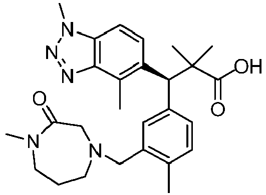

### tert-Butyl (3-(2-chloro-N-methylacetamido)propyl)carbamate

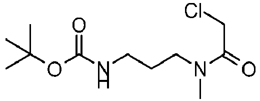

To a solution of tert-butyl (3-(methylamino)propyl)carbamate (8 g, 42.5 mmol) in dichloromethane (DCM) (100 mL) was added TEA (11.85 mL, 85 mmol). The reaction mixture was cooled to 0°C and then chloroacetyl chloride (5.11 mL, 63.7 mmol) was added dropwise. It was stirred at ambient temperature for 1 h. The reaction was quenched with saturated sodium bicarbonate solution (80 mL) and extracted with DCM (2 x 80 mL). The combined organic layer was washed with brine solution (80 mL), dried over anhydrous Na₂SO₄, filtered and concentrated. The crude residue was purified by flash column chromatography eluting with EtOAc: Hexane (3:7) to give the title compound (7 g, 24.37 mmol, 57.4 % yield) as light yellow liquid. LCMS *m*/*z* 265.35 (M+H)⁺,1.79 min (ret. time)

### tert-Butyl 4,6,6-trimethyl-3-oxo-1,4-diazepane-1-carboxylate

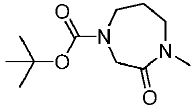

To a solution of tert-butyl (3-(2-chloro-N-methylacetamido)propyl)carbamate (7 g, 26.4 mmol) in N,N-dimethylformamide (DMF) (70 mL) at 0°C under nitrogen atmosphere was added NaH (2.115 g, 52.9 mmol). It was stirred at ambient temperature for 6 h. The reaction was quenched with ice cold water (50 mL). It was extracted with DCM (3 x 50 mL). The combined organic layer was washed with ice cold water (3 x 50 mL), dried over anhydrous Na₂SO₄, filtered and concentrated. The crude residue was purified on flash column chromatography eluting with EtOAc : hexane (1:1) to give the title compound (4 g, 15.60 mmol, 59.0 % yield) as light yellow liquid. ¹H NMR (400 MHz, CDCl₃) δ ppm 1.46 (s, 9 H), 1.85-1.95 (m, 2 H), 2.98 (s, 3 H), 3.32 - 3.42 (m, 2 H), 3.45-3.55 (m, 2 H), 4.02 - 4.30 (m, 2 H).

### 1-Methyl-1,4-diazepan-2-one hydrochloride

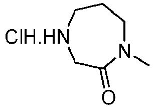

To a solution of tert-butyl 4-methyl-3-oxo-1,4-diazepane-1-carboxylate (4 g, 17.52 mmol) in 1,4-dioxane (20 mL) at 0°C was added 4M HCI in 1,4-dioxane (8.76 mL, 35.0 mmol) under nitrogen atmosphere. It was stirred for 2 h. The reaction mixture was concentrated. Diethyl ether (40 mL) was added to the crude material and stirred for 30 min. The solid was filtered and dried to give the title compound (2.1 g, 12.45 mmol, 71.1 % yield) as an off white solid. LCMS *m*/*z* 129.2(M+H)⁺, 2.06 min (ret. time)

### (S)-3-(1,4-Dimethyl-1H-benzo[d][1,2,3]triazol-5-yl)-2,2-dimethyl-3-(4-methyl-3-((4-methyl-3-oxo-1,4-diazepan-1-yl)methyl)phenyl)propanoic acid

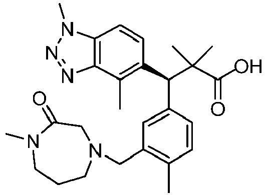

A mixture of (S)-methyl 3-(3-(chloromethyl)-4-methylphenyl)-3-(1,4-dimethyl-1H-benzo[d][1,2,3]triazol-5-yl)-2,2-dimethylpropanoate (70 mg, 0.175 mmol), 1-methyl-1,4-diazepan-2-one hydrochloride (28.8 mg, 0.175 mmol) and DIEA (0.122 mL, 0.700 mmol) in acetonitrile (3 mL) was heated in a Biotage microwave at high absorption for 1 h at 120 °C. The solvent was concentrated and the crude product was re-dissolved in methanol (3.00 mL), 2M LiOH (0.525 mL, 1.050 mmol) was added and the reaction was heated in a Biotage microwave at high absorption for 1.5 h at 120 °C. The solvent was concentrated and the crude product was acidified with 1 N HCl to around pH 1, 1 mL DMSO was added and concentrated. The sample was filtered and purified with preparative HPLC (with 0.1 % formic acid as modifier) to give the title compound (12.7 mg, 0.027 mmol, 15.19 % yield). LC-MS *m*/*z* 478.2 (M+H)⁺, 0.67 min (ret. time)

### Example 4 (S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((4-ethyl-6,6-dimethyl-3-oxo-1,4-diazepan-1-yl)methyl)-4-methylphenyl)-2,2-dimethylpropanoic acid

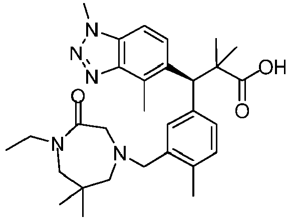

### tert-Butyl (3-amino-2,2-dimethylpropyl)carbamate

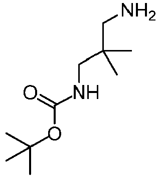

To a solution of 2,2-dimethylpropane-1,3-diamine (80 g, 783 mmol) in dichloromethane (DCM) (500 mL) at 0 °C was added Boc-anhydride (91 mL, 391 mmol). It was stirred at ambient temperature for 12 h. The reaction mixture was concentrated. The crude product was purified with column chromatography (Neutral Allumina) by using MeOH : DCM (1:9) as solvent to give the title compound (50 g, 247 mmol, 31.6 % yield) as a white solid. ¹H NMR (400 MHz, CDCl₃) δ ppm 0.85 (s, 6 H), 1.44 (s, 9 H), 2.35 - 2.52 (m, 2 H), 3.00 (br d, *J*=6.14 Hz, 2 H), 5.16 (br s, 1 H).

### tert-Butyl (2,2-dimethyl-3-(2-nitrophenylsulfonamido)propyl)carbamate

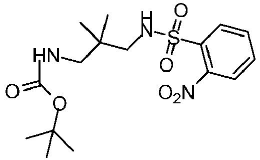

To a solution of tert-butyl (3-amino-2,2-dimethylpropyl)carbamate (50 g, 247 mmol) in dichloromethane (DCM) (400 mL) at 0 °C was added K₂CO₃ (51.2 g, 371 mmol) and 2-nitrobenzene-1-sulfonyl chloride (54.8 g, 247 mmol) portion wise. It was stirred at ambient temperature for 4 h. Water (500 mL) was added and extracted with DCM (2 x 500 mL). The combined organic layer was washed with brine solution (100 mL), dried over anhydrous Na₂SO₄, filtered and concentrated. It was purified with column chromatography eluting with EtOAc: Hexane (3:7 to give the title compound (51 g, 129 mmol, 52.2 % yield) as gummy liquid. LCMS *m*/*z* 386.14 (M-H)⁺, 3.637 min (ret. time)

### tert-Butyl (3-(N-ethyl-2-nitrophenylsulfonamido)-2,2-dimethylpropyl)carbamate

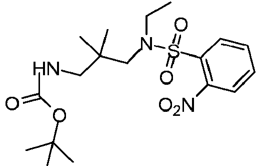

To a solution of tert-butyl (2,2-dimethyl-3-(2-nitrophenylsulfonamido)propyl)carbamate (3g, 7.74 mmol) in N,N-dimethylformamide (DMF) (30 mL) at 0°C was added potassium carbonate (2.140 g, 15.49 mmol) and ethyl iodide (2.503 mL, 31.0 mmol). The reaction mixture was stirred at 80 °C for 16 h in a sealed tube. The reaction was quenched with water (10 mL). It was extracted with EtOAc (3 x 10 mL). The combined organic layer was washed with brine (20 mL), dried over anhydrous Na₂SO₄, filtered and concentrated to give the title compound (2.5 g, 6.00 mmol, 77 % yield). LC-MS *m*/*z* 416.19 (M+H)⁺, 3.76 min (ret. time)

### tert-Butyl (3-(ethylamino)-2,2-dimethylpropyl)carbamate

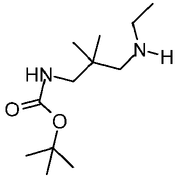

To a solution of tert-butyl (3-(N-ethyl-2-nitrophenylsulfonamido)-2,2-dimethylpropyl)carbamate (20.5 g, 49.3 mmol) in N,N-dimethylformamide (DMF) (150 mL) at 0°C was added benzenethiol (15.24 mL, 148 mmol) and potassium carbonate (10.23 g, 74.0 mmol). The reaction mixture was stirred at ambient temperature for 2 h. The reaction was quenched with 1 N HCl to pH = 1. It was extracted with EtOAc (2 x 20 mL). The aqueous layer was basified with 1N NaOH to pH = 14. It was extracted with ethyl acetate twice. The combined organic layer was washed with cold water and brine (20 mL), dried over anhydrous Na₂SO₄, filtered and concentrated to give the title compound which was used in the next step without further purification (12 g, 52.1 mmol, 106 % yield). ¹H NMR (400 MHz, CDCl₃) δ ppm 0.87 - 0.95 (s, 6 H), 1.09 (t, *J* = 7.13 Hz, 3H), 1.44 (s, 9 H), 2.41 (s, 2 H), 2.61 (q, *J* = 7.02 Hz, 2 H), 3.01 (br d, *J*=5.92 Hz, 2 H), 5.72 (br s, 1 H).

### tert-Butyl (3-(2-chloro-N-ethylacetamido)-2,2-dimethylpropyl)carbamate

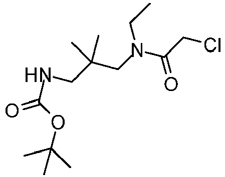

To a solution of tert-butyl (3-(ethylamino)-2,2-dimethylpropyl)carbamate (12 g, 52.1 mmol) in dichloromethane (DCM) (70 mL) at 0°C was added 2-chloroacetyl chloride (5.88 g, 52.1 mmol) and triethylamine (14.52 mL, 104 mmol). The reaction mixture was stirred at ambient temperature for 2 h. The reaction was quenched with water (15 mL). It was extracted with DCM (2 x 10 mL). The combined organic layer was washed with brine (10 mL), dried over anhydrous Na₂SO₄, filtered and concentrated. The crude product was purified with silica gel chromatographyy eluting with 18 % EtOAc in pet ether to give the title compound (9 g, 21.07 mmol, 40.4 % yield). LC-MS *m*/*z* 307.40 (M+H)⁺, 2.31 min (ret. time)

### tert-Butyl 4-ethyl-6,6-dimethyl-3-oxo-1,4-diazepane-1-carboxylate

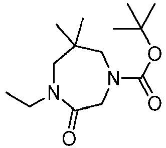

To a solution of tert-butyl (3-(2-chloro-N-ethylacetamido)-2,2-dimethylpropyl)carbamate (9 g, 29.3 mmol) in N,N-dimethylformamide (DMF) (90 mL) at 0°C was added NaH (3.84 g, 88 mmol). The reaction mixture was stirred at ambient temperature for 6 h. The reaction was quenched with cold water (10 mL). The aqueous layer was extracted with EtOAc (2 x 20 mL). The combined organic layer was washed with brine (10 mL), dried over anhydrous Na₂SO₄, filtered and concentrated. The crude product was purified with silica gel chromatography eluting with 28 % EtOAc / pet ether to give the title compound (4g, 14.09 mmol, 48.0 % yield). LC-MS *m*/*z* 271.0 (M+H)⁺, 2.06 min (ret. time)

### 1-Ethyl-6,6-dimethyl-1,4-diazepan-2-one hydrochloride

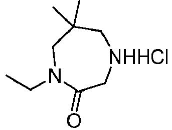

To a solution of tert-butyl 4-ethyl-6,6-dimethyl-3-oxo-1,4-diazepane-1-carboxylate (4 g, 14.79 mmol) in 1,4-dioxane (30 mL) at 0°C was added 4M HCI in dioxane (15 mL, 60.0 mmol). The reaction mixture was stirred at ambient temperature for 2 h. The reaction mixture was concentrated and washed with diethyl ether to give the title compound (2.6 g, 12.53 mmol, 85 % yield) as an off white solid. LC-MS *m*/*z* 171.2 (M+H)⁺, 2.99 min (ret. time)

### (S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((4-ethyl-6,6-dimethyl-3-oxo-1,4-diazepan-1-yl)methyl)-4-methylphenyl)-2,2-dimethylpropanoic acid

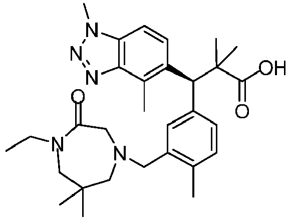

A mixture of (S)-methyl 3-(3-(chloromethyl)-4-methylphenyl)-3-(1,4-dimethyl-1H-benzo[d][1,2,3]triazol-5-yl)-2,2-dimethylpropanoate (70 mg, 0.175 mmol), 1-ethyl-6,6-dimethyl-1,4-diazepan-2-one hydrochloride (36.2 mg, 0.175 mmol) and DIEA (0.122 mL, 0.700 mmol) in acetonitrile (3 mL) was heated in a Biotage microwave at high absorption for 1 h at 120 °C. The solvent was concentrated and the crude product was re-dissolved in methanol (3.00 mL), 2M LiOH (0.525 mL, 1.050 mmol) was added and the reaction heated in a Biotage microwave at high absorption for 1.5 h at 120 °C. The solvent was concentrated and the crude product was acidified with 1 N HCl to around pH 1, 1 mL DMSO was added and concentrated. The sample was filtered and purified with neutral preparative HPLC to give the title compound (60.5 mg, 0.116 mmol, 66.5 % yield) as white solid. LC-MS *m*/*z* 520.2 (M+H)⁺, 0.91 min (ret. time)

### Example 5 3-(1,4-Dimethyl-1H-benzo[d][1,2,3]triazol-5-yl)-3-(3-((6-ethyl-4-methyl-3-oxo-1,4-diazepan-1-yl)methyl)-4-methylphenyl)-2,2-dimethylpropanoic acid

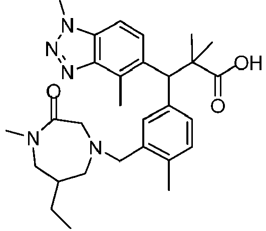

### 2-Ethylmalononitrile

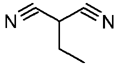

To a suspension of malononitrile (15 g, 227 mmol) and iodoethane (17.71 g, 114 mmol) at 0 °C was added tetrabutylammonium bromide (1.830 g, 5.68 mmol). It was stirred for 30 min. K₂CO₃ (15.69 g, 114 mmol) was then added to reaction mixture. It was stirred at ambient temperature for 1 h. The reaction mixture was concentrated. It was quenched with ice water, extracted with DCM twice. The organic layer was dried under anhydrous Na₂SO₄, filtered and concentrated. The crude product was purified with flash column chromatography using EtOAc: hexane (10:90) as solvent. The eluted fractions were concentrated under vacuum to afford the title compound (6 g, 63.8 mmol, 28.1 % yield) as pale yellow liquid. ¹H NMR (400MHz, CDCl₃) δ 3.6 (t, 1H), 2.1 (m, 2H), 1.21 (t, 3H).

### di-tert-Butyl (2-ethylpropane-1,3-diyl)dicarbamate

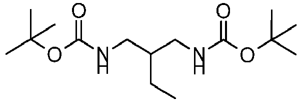

To a solution of 2-ethylmalononitrile (12 g, 128 mmol) in methanol (100 mL) was added boc-anhydride (29.6 mL, 128 mmol) and Raney-Ni (13.91 g, 63.8 mmol). The reaction was stirred under hydrogenation at 90 Psi for 16 h. The reaction mixture was filtrated through celite. The filtrate was evaporated under reduced pressure. The crude product was purified with flash column chromatography by using EtOAc: Hexane (10:88) as solvent. The eluted fractions were concentrated under vacuum to afford to give the title compound (8 g, 26.5 mmol, 20.75 % yield) as color less liquid. LCMS *m*/*z* 303.3 (M+H)⁺, 4.95 min (ret. time)

### 2-Ethylpropane-1,3-diamine hydrochloride

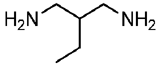

To a solution of di-tert-butyl (2-ethylpropane-1,3-diyl)dicarbamate (12 g, 39.7 mmol) in 1,4-dioxane (10 mL) at 0 °C was added 4 M HCI in dioxane (5 mL, 20.00 mmol). It was stirred at ambient temperature for 6 h. The reaction mixture was concentrated. The crude compound was washed with diethyl ether to afford the title compound (2.2 g, 20.10 mmol, 50.6 % yield) as gummy liquid. ¹H NMR (400MHz, CDCl₃) δ 3.92-3.82 (m, 4H), 1.9 (m, 1H), 1.4 (m, 2H), 0.85 (t, 3H).

### tert-Butyl (2-(aminomethyl)butyl)carbamate

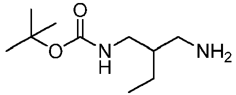

To a suspension of 2-ethylpropane-1,3-diamine (2.2 g, 21.53 mmol) in tetrahydrofuran (THF) (20 mL) at 0 °C was added boc-anhydride (5.00 mL, 21.53 mmol) and stirred for 30 min. It was then stirred at ambient temperature for 1 h. The reaction mixture was concentrated and quenched with ice water. It was extracted with DCM twice. The organic layer was dried under anhydrous Na₂SO₄, filtered and concentrated. The crude product was purified with flash column chromatography using MeOH: DCM (9:91) as solvent. The eluted fractions were concentrated under vacuum to afford the title compound (1.2 g, 5.93 mmol, 27.6 % yield) as pale yellow liquid. ¹H NMR (400MHz, CDCl₃), δ 5.1 (br, S, 1H), 3.2-3.0 (m, 4H), 1.6 (m, 1H), 1.2 (m, 2H), 0.8 (t, 3H).

### tert-Butyl (2-((2-nitrophenylsulfonamido)methyl)butyl)carbamate

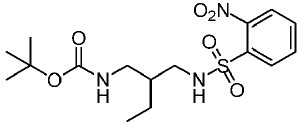

To a suspension of tert-butyl (2-(aminomethyl)butyl)carbamate (600 mg, 2.97 mmol) in dichloromethane (DCM) (5 mL) at 0 °C was added 2-nitrobenzene-1-sulfonyl chloride (657 mg, 2.97 mmol) and K₂CO₃ (820 mg, 5.93 mmol) and stirred for 30 min. The reaction mixture was then stirred at ambient temperature for 16 h. The reaction was quenched with ice water, extracted with DCM twice. The organic layer was dried under anhydrous Na₂SO₄, filtered and concentrated. It was purified with flash column chromatography eluting with EtOAc: hexane (25:75) to give the title compound (800 mg, 2.065 mmol, 69.6 % yield) as colorless liquid. LCMS *m*/*z* 386.14 (M-H)⁻, 3.63 min (ret. time)

### tert-Butyl (2-((N-methyl-2-nitrophenylsulfonamido)methyl)butyl)carbamate

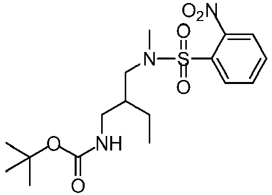

To a solution of tert-butyl (2-((2-nitrophenylsulfonamido)methyl)butyl)carbamate (400 mg, 1.032 mmol) in N,N-dimethylformamide (DMF) (10 mL) at 0 °C was added K₂CO₃ (214 mg, 1.549 mmol) and methyl iodide (0.084 mL, 1.342 mmol). The reaction was stirred at ambient temperature for 4 h. The reaction was quenched with cold water (10 mL) and extracted with EtOAc (2 x 50 mL). The combined organic layer was washed with brine solution (10 mL), dried over anhydrous Na₂SO₄, filtered and concentrated. The crude residue was purified with flash column chromatography by using EtOAc: Hexane (3:.7) as solvent to give the title compound (300 mg, 0.747 mmol, 72.4 % yield) as gummy liquid. LCMS m/z 402.26 (M+H)⁺, 2.48 min (ret. time)

### tert-Butyl (2-((methylamino)methyl)butyl)carbamate

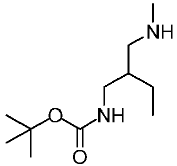

To a solution of tert-butyl (2-((N-methyl-2-nitrophenylsulfonamido)methyl)butyl)carbamate ( 600 mg, 1.494 mmol) in N,N-dimethylformamide (DMF) (15 mL) at 0 °C was added K₂CO₃ (413 mg, 2.99 mmol) and thiophenol (0.308 mL, 2.99 mmol). The reaction was stirred at ambient temperature for 16 h. It was quenched with water (10 mL), acidified with 1N HCI. It was extracted with ethyl acetate (2 x 10 mL). The aqueous layer was basified with 1N NaOH and extracted with ethyl acetate (2 x 20 mL). The organic layer was washed with brine (10 mL) and concentrated under reduced pressure to give the title compound (250 mg, 1.156 mmol, 77 % yield) as a gummy liquid. ¹H NMR (400MHz, CDCl₃) δ 5.5 (br s, 1H), 3.3 (m, 1H), 3.0 (m, 1H), 2.5 (m, 1H), 2.5 (m, 1H), 2.0 (s, 3H),1.4 (s, 9H),1.2(m, 3H), 0.9 (t, 3H).

### tert-Butyl 4-2-chloro-N-methlacetamido)methyl)hexanoate

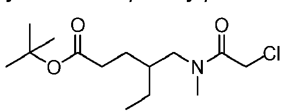

To a suspension of tert-butyl (2-((methylamino)methyl)butyl)carbamate (250 mg, 1.156 mmol) in dichloromethane (DCM) (10 mL) at 0 °C was added TEA (0.322 mL, 2.311 mmol) and chloroacetyl chloride (0.185 mL, 2.311 mmol). It was stirred for 30 min and then stirred at ambient temperature for 3 h. The reaction was quenched with ice water and extracted with DCM (2x). The combined organic layer was dried with anhydrous Na₂SO₄, and filtered and concentrated. The crude product was purified on flash column chromatography by using EtOAc: hexane (35:65) as solvent. The eluted fractions were concentrated under vacuum to afford the title compound (90 mg, 0.308 mmol, 26.7 % yield) as colorless liquid. LCMS *m*/*z* 237.1 (M-Boc)⁺, 4.25 min (ret. time)

### tert-Butyl 6-ethyl-4-methyl-3-oxo-1,4-diazepane-1-carboxylate

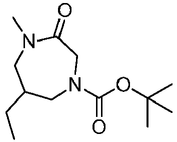

To a suspension oftert-butyl 4-((2-chloro-N-methylacetamido)methyl)hexanoate (90 mg, 0.308 mmol) in N,N-dimethylformamide (DMF) (5 mL) at 0 °C was added NaH (14.80 mg, 0.617 mmol). The reaction mixture was stirred at ambient temperature for 6 h. The reaction was quenched with ice water and extracted with EtOAc twice. The organic layer was washed with ice water twice, brine solution, dried under anhydrous Na₂SO₄, filtered and concentrated. The crude product was purified on flash column chromatography by using EtOAc: Hexane (40:60) as solvent. The eluted fractions were concentrated under vacuum to afford the title compound (40 mg, 0.139 mmol, 45.1 % yield) as pale yellow liquid. LCMS *m*/*z* 201.6 (M-Boc)⁺, 4.06 min (ret. time)

### 6-Ethyl-1-methyl-1,4-diazepan-2-one hydrochloride

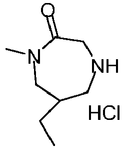

To a solution of tert-butyl 6-ethyl-4-methyl-3-oxo-1,4-diazepane-1-carboxylate (40 mg, 0.156 mmol) in 1,4-dioxane (3 mL) at 0 °C was added 4 M HCI in dioxane (3 mL, 12.00 mmol). The reaction mixture was stirred at ambient temperature for 6 h. The reaction mixture was concentrated and washed with n-pentane and diethyl ether to give the title compound (20 mg, 0.099 mmol, 63.2 % yield) as gummy solid. LCMS m/z 157.2 (M+H)⁺, 3.17 min (ret. time)

### 3-(1,4-Dimethyl-1H-benzo[d][1,2,3]triazol-5-yl)-3-(3-((6-ethyl-4-methyl-3-oxo-1,4-diazepan-1-yl)methyl)-4-methylphenyl)-2,2-dimethylpropanoic acid

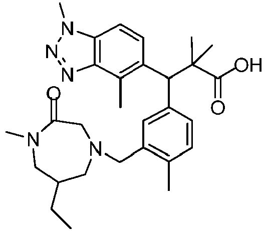

A mixture of 6-ethyl-1-methyl-1,4-diazepan-2-one hydrochloride (31.3 mg, 0.163 mmol), methyl 3-(3-(chloromethyl)-4-methylphenyl)-3-(1,4-dimethyl-1H-benzo[d][1 ,2,3]triazol-5-yl)-2,2-dimethylpropanoate (65 mg, 0.163 mmol) and DIEA (0.114 mL, 0.650 mmol) in acetonitrile (3 mL). The reaction mixture was heated in a Biotage microwave at high absorption for 1 h at 120 °C. The solvent was concentrated and the crude product was re-dissolved in methanol (3.00 mL), 2M LiOH (0.488 mL, 0.975 mmol) was added and the reaction heated in a Biotage microwave at high absorption for 1 h at 120 °C. The solvent was concentrated and the crude product was acidified with 1 N HCl to around pH 1, 1 mL DMSO was added and concentrated. The sample was filtered and purified with preparative HPLC (using formic acid condition as modifier) to give the title compound (12.6 mg, 0.025 mmol, 15.33 % yield). LC-MS *m*/*z* 506.2 (M+H)⁺, 0.78 min (ret. time)

### Example 6 (S)-3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid, 0.3Formic acid salt

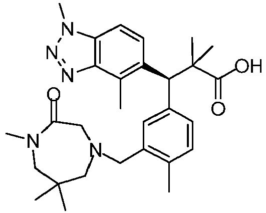

### tert-Butyl (2,2-dimethyl-3-(N-methyl-2-nitrophenylsulfonamido)propyl)carbamate N38084-15-A1

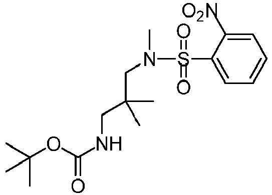

To a solution of tert-butyl (2,2-dimethyl-3-(2-nitrophenylsulfonamido)propyl)carbamate (10 g, 25.8 mmol) in N,N-dimethylformamide (DMF) (100 mL) at 0 °C was added potassium carbonate (5.35 g, 38.7 mmol) portion wise. It was stirred at ambient temperature for 12 h. It was quenched with water (10 mL) and extracted with DCM (2 x 50 mL). The combined organic layer was washed with brine solution (10 mL) and dried over anhydrous Na₂SO₄, filtered and concentrated. The crude residue was purified with flash column chromatography using EtOAc: hexane (3:.7) as solvent. The eluted fractions were concentrated under vacuum to give the title compound (8 g, 19.68 mmol, 76 % yield) as a gummy liquid. LC-MS *m*/*z* 402.50 (M+H)⁺, 2.502 min (ret. time)

### tert-Butyl (2,2-dimethyl-3-(methylamino)propyl)carbamate

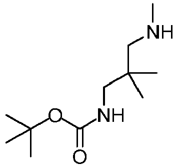

To a solution of tert-butyl (2,2-dimethyl-3-(N-methyl-2-nitrophenylsulfonamido)propyl)carbamate (8 g, 19.93 mmol) in N,N-Dimethylformamide (DMF) at 0 °C was added K₂CO₃ (2.75 g, 19.93 mmol) and thiophenol (2.052 mL, 19.93 mmol). It was stirred at ambient temperature for 6 h. It was quenched with water (10 mL) and then acidified with 1N HCl. It was extracted with ethyl acetate (2 x 10 mL). The aqueous layer was basified with 1N NaOH and extracted with ethyl acetate (2 x 20 mL). The second organic layer was washed with brine (10 mL) and concentrated to give the title compound (4 g, 18.49 mmol, 93 % yield) as a gummy liquid. ¹H NMR (400 MHz, CDCl₃) δ ppm 0.93 (s, 6 H), 1.43 (s, 9 H), 2.86 (d, *J* = 6.80 Hz, 2 H), 3.16 (s, 3 H), 3.18 - 3.23 (m, 2 H), 5.91 (br s, 1 H)

### tert-Butyl (3-(2-chloro-N-methylacetamido)-2,2-dimethylpropyl)carbamate

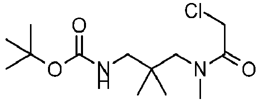

To a solution of tert-butyl (2,2-dimethyl-3-(methylamino)propyl)carbamate (4 g, 18.49 mmol) in dichloromethane (DCM) (40 mL) at 0 °C was added chloroacetyl chloride (1.777 mL, 22.19 mmol). It was stirred at ambient temperature for 12 h under nitrogen. The reaction was quenched with saturated sodium bicarbonate solution (20 mL) and extracted with DCM (2 x 20 mL). The organic layer was concentrated. The crude residue was purified with flash column chromatography using EtOAc: hexane (4:6) as solvent to give the title compound (4.5 g, 13.60 mmol, 73.6 % yield) as gummy liquid. LC-MS *m*/*z* 193.0 (M-Boc)⁺, 3.426 min (ret. time)

### tert-Butyl 4,6,6-trimethyl-3-oxo-1,4-diazepane-1-carboxylate

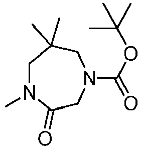

To a solution of tert-butyl (3-(2-chloro-N-methylacetamido)-2,2-dimethylpropyl)carbamate (4.2 g, 14.34 mmol) in N,N-dimethylformamide (DMF) (40 mL) at 0 °C was added NaH (0.516 g, 21.52 mmol) portionwise. It was stirred at ambient temperature for 6 h. It was quenched with cold water (100 mL) and extracted with DCM (2 x 50 mL). The combined organic layer was washed with brine solution (10 mL), dried over anhydrous Na₂SO₄, filtered and concentrated. It was purified with flash column chromatography using EtOAc : hexane (4:6) as solvent to give the title compound (1.9 g, 7.31 mmol, 50.9 % yield) as an off- white solid. LC-MS *m*/*z* 257.16 (M+H)⁺, 1.907 min (ret. time)

### 1,6,6-Trimethyl-1,4-diazepan-2-one, hydrochloride

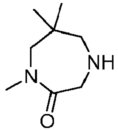

To a solution of tert-butyl 4,6,6-trimethyl-3-oxo-1,4-diazepane-1-carboxylate (1.345 g, 5.25 mmol) in 1,4-dioxane (5 mL) was added 4 M HCI in 1,4 dioxane (3.00 mL, 12.00 mmol) and stirred at ambient temperature for 19 h 45 min. 4 M HCI in 1,4 dioxane (1.312 mL, 5.25 mmol) was added and continued stirring for 47 h. It was concentrated and dried to give the title compound (0.924 g, 4.80 mmol, 91 %). LCMS *m*/*z* 156 (M+H)⁺, 1.17 min (ret time) ¹H NMR (400 MHz, DMSO-d₆) δ 9.80 (br. s., 1H), 5.24 (br. s., 3H), 3.81 (br. s., 2H), 2.96 (br. s., 2H), 2.91 (s, 2H), 0.99 (s, 6H)

### (S)-Methyl 3-(3-(chloromethyl)-4-methylphenyl)-3-(1,4-dimethyl-1H-benzo[d][1,2,3]triazol-5-yl)-2,2-dimethylpropanoate

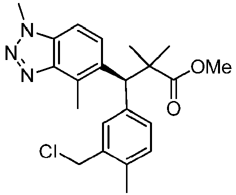

To a solution of (S)-methyl 3-(1,4-dimethyl-1H-benzo[d][1,2,3]triazol-5-yl)-3-(3-(hydroxymethyl)-4-methylphenyl)-2,2-dimethylpropanoate (710 mg, 1.861 mmol) in dichloromethane (DCM) (10 mL) at ambient temperature was added thionyl chloride (0.272 mL, 3.72 mmol). It was stirred for 40 min. It was concentrated to give the title compound (750 mg, 1.875 mmol, 101 % yield). LC-MS *m*/*z* 400.2 (M+H)⁺, 1.21 min (ret. time)

### (S)-3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid, 0.3Formic acid salt

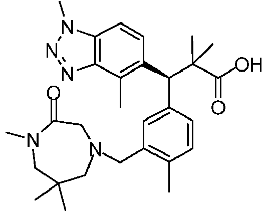

A mixture of tert-butyl 4,6,6-trimethyl-3-oxo-1,4-diazepane-1-carboxylate (103 mg, 0.400 mmol) and 4M HCI in dioxane (0.200 mL, 0.800 mmol) in DCM (2 mL) was stirred at ambient temperature for 18 h. The solvent was concentrated and the crude product was re-dissolved in 1 mL CH₃CN and then added to a mixture of (S)-methyl 3-(3-(chloromethyl)-4-methylphenyl)-3-(1,4-dimethyl-1H-benzo[d][1 ,2,3]triazol-5-yl)-2,2-dimethylpropanoate (80 mg, 0.200 mmol) and DIEA (0.140 mL, 0.800 mmol) in acetonitrile (3 mL). The reaction mixture was heated in a Biotage microwave at high absorption for 1 h at 120 °C. The solvent was concentrated and the crude product was re-dissolved in methanol (3.00 mL), 2M LiOH (0.600 mL, 1.200 mmol) was added and the reaction heated in a Biotage microwave at high absorption for 1 h at 120 °C. The solvent was concentrated and the crude product was acidified with 1 N HCI to around pH 1, 1 mL DMSO was added and concentrated. The sample was filtered and purified with neutral with preparative HPLC to give the title compound (56 mg, 0.108 mmol, 53.9 % yield). LC-MS m/z 506.3 (M+H)⁺, 0.86 min (ret. time)

### Example 7 3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid

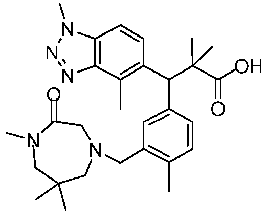

### Methyl 3-(3-(chloromethyl)-4-methylphenyl)-3-(1,4-dimethyl-1H-benzo[d][1,2,3]triazol-5-yl)-2,2-dimethylpropanoate

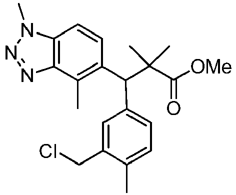

To a solution of methyl 3-(1,4-dimethyl-1H-benzo[d][1,2,3]triazol-5-yl)-3-(3-(hydroxymethyl)-4-methylphenyl)-2,2-dimethylpropanoate (3.9 g, 10.22 mmol) in dichloromethane (DCM) (30 mL) was added thionyl chloride (1.492 mL, 20.45 mmol). The mixture was stirred for 40 min and then concentrated to give the title compound (4 g, 10.00 mmol, 98 % yield). LC-MS *m*/*z* 400.1 (M+H)⁺, 1.21 min (ret. time)

### 3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid

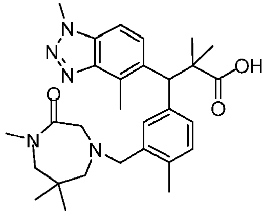

A mixture of tert-butyl 4,6,6-trimethyl-3-oxo-1,4-diazepane-1-carboxylate (103 mg, 0.400 mmol) and 4M HCI in dioxane (0.200 mL, 0.800 mmol) in DCM (2 mL) was stirred at ambient temperature for 18 h. The solvent was concentrated and the crude product was re-dissolved in 1 mL CH₃CN and then added to a mixture of methyl 3-(3-(chloromethyl)-4-methylphenyl)-3-(1,4-dimethyl-1H-benzo[d][1,2,3]triazol-5-yl)-2,2-dimethylpropanoate (80 mg, 0.200 mmol) and DIEA (0.140 mL, 0.800 mmol) in acetonitrile (3 mL). The reaction mixture was heated in a Biotage microwave at high absorption for 1 h at 120 °C. The solvent was concentrated and the crude product was re-dissolved in methanol (3.00 mL), 2M LiOH (0.600 mL, 1.200 mmol) was added and the reaction heated in a Biotage microwave at high absorption for 1 h at 120 °C. The solvent was concentrated and the crude product was acidified with 1 N HCI to around pH 1, 1 mL DMSO was added and concentrated. The sample was filtered and purified with neutral preparative HPLC to give the title compound (43 mg, 0.085 mmol, 42.5 % yield). LC-MS *m*/*z* 506.4 (M+H)⁺, 0.87 min (ret. time)

### Example 8 3-(3-((4-Cyclobutyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

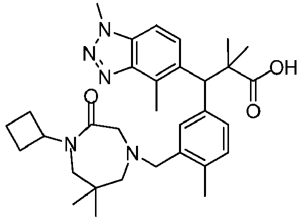

### tert-Butyl (3-(cyclobutylamino)-2,2-dimethylpropyl)carbamate

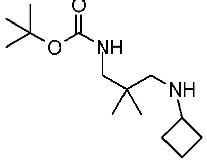

To a solution of tert-butyl (3-amino-2,2-dimethylpropyl)carbamate (45 g, 222 mmol) in methanol (400 mL) at 0°C was added cyclobutanone (17.15 g, 245 mmol) and acetic acid (12.73 mL, 222 mmol). The reaction mixture was stirred under nitrogen for 2 h, then NaCNBH₄ (28.0 g, 445 mmol) was added. It was stirred at ambient temperature for 48 h. The reaction mixture was concentrated. The residue was quenched with 1N NaOH solution (150 mL) and extracted with ethyl acetate (3 x 100 mL). The combined organic layer was washed with brine solution (200 mL), dried over anhydrous Na₂SO₄, filtered and concentrated. The crude residue was purified with flash column chromatography eluting with 5 % methanol in DCM to give the title compound (16 g, 62.4 mmol, 28.1 % yield) as a yellow liquid. ¹H NMR (400 MHz, CDCl₃) δ ppm 0.92 (s, 6 H), 1.45 (s, 9 H), 1.65 - 1.84 (m, 4 H), 2.16-2.24 (m, 3 H), 3.00 (br d, *J*=6.14 Hz, 2 H), 5.42 (br s, 1 H)

### tert-Butyl (3-(2-chloro-N-cyclobutylacetamido)-2,2-dimethylpropyl)carbamate

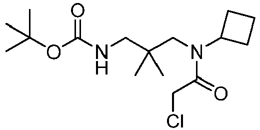

To a solution of tert-butyl (3-(cyclobutylamino)-2,2-dimethylpropyl)carbamate (32 g, 125 mmol) in dichloromethane (DCM) (320 mL) was added TEA (34.8 mL, 250 mmol). The reaction mixture was cooled to 0 °C, chloroacetyl chloride (15.00 mL, 187 mmol) was added slowly. The reaction mixture was stirred at ambient temperature for 1 h under nitrogen atmosphere. The reaction was quenched with saturated sodium bicarbonate solution (150 mL) and extracted with DCM (2 x 150 mL). The combined organic layer was washed with brine solution (200 mL), dried over anhydrous Na₂SO₄, filtered and concentrated. The crude residue was purified with flash column chromatography eluting with EtOAc: hexane (2:8) to give the title compound (28 g, 82 mmol, 65.5 % yield) as an off white solid. LCMS *m*/*z* 333.43 (M+H)⁺, 2.51 min (ret. time)

### tert-Butyl 4-cyclobutyl-6,6-dimethyl-3-oxo-1,4-diazepane-1-carboxylate

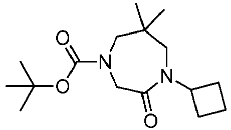

To a solution of tert-butyl (3-(2-chloro-N-cyclobutylacetamido)-2,2-dimethylpropyl)carbamate (14 g, 42.1 mmol) in N,N-dimethylformamide (DMF) (140 mL) at 0 °C under nitrogen atmosphere was added NaH (3.36 g, 84 mmol) portion wise. It was stirred for 6 h. The reaction was quenched with ice cold water (50 mL) and extracted with DCM (3 x 50 mL). The combined organic layer was washed with ice cold water (3 x 50 mL), dried over anhydrous Na₂SO₄, filtered and concentrated. The crude residue was purified with flash column chromatography eluting with EtOAc: hexane (2.5:7.5) to give the title compound (8 g, 26.9 mmol, 63.9 % yield) as off white solid. LCMS *m*/*z* 297.32 (M+H)⁺, 2.34 min (ret. time)

### 1-Cyclobutyl-6,6-dimethyl-1,4-diazepan-2-one hydrochloride

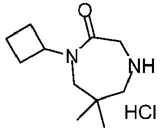

To a solution of tert-butyl 4-cyclobutyl-6,6-dimethyl-3-oxo-1,4-diazepane-1-carboxylate (4 g, 13.50 mmol) in 1,4-dioxane (35 mL) at 0 °C under nitrogen atmosphere was added 4M HCI in 1,4-dioxane (6.75 mL, 27.0 mmol). It was stirred for 2 h. The reaction mixture was concentrated. Diethyl ether (40 mL) was added to the crude residue and stirred for 30 min. The solid was filtered and dried to give the title compound (2.733 g, 11.36 mmol, 84 % yield) as an off white solid. LCMS *m*/*z* 197.2 (M+H)⁺, 3.28 min (ret. time)

### 3-(3-((4-Cyclobutyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

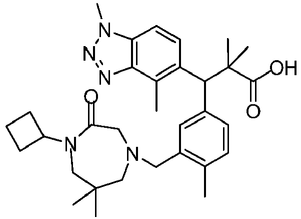

A mixture of 1-cyclobutyl-6,6-dimethyl-1,4-diazepan-2-one hydrochloride (37.8 mg, 0.163 mmol), methyl 3-(3-(chloromethyl)-4-methylphenyl)-3-(1,4-dimethyl-1H-benzo[d][1,2,3]triazol-5-yl)-2,2-dimethylpropanoate (65 mg, 0.163 mmol) and DIEA (0.114 mL, 0.650 mmol) in acetonitrile (3 mL was heated in a Biotage microwave at high absorption for 1 h at 120 °C. The solvent was concentrated and the crude product was re-dissolved in methanol (3.00 mL), 2M LiOH (0.488 mL, 0.975 mmol) was added and the reaction heated in a Biotage microwave at high absorption for 1 h at 120 °C. The solvent was concentrated and the crude product was acidified with 1 N HCl to around pH 1, 1 mL DMSO was added and concentrated. The sample was filtered and purified with neutral preparative HPLC to give the title compound (34.3 mg, 0.063 mmol, 38.7 % yield) LC-MS *m*/*z* 546.5 (M+H)⁺, 0.99 min (ret. time)

### Example 9 3-(3-((4-(Cyclopropylmethyl)-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

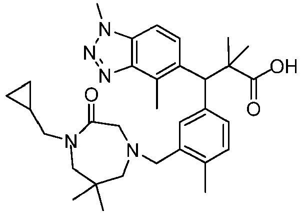

### tert-Butyl (3-(N-(cyclopropylmethyl)-2-nitrophenylsulfonamido)-2,2-dimethylpropyl)carbamate

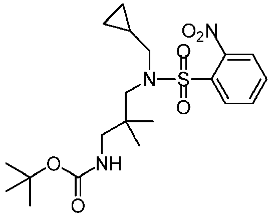

To a solution of tert-butyl (2,2-dimethyl-3-(2-nitrophenylsulfonamido)propyl)carbamate (15 g, 38.7 mmol) in N,N-dimethylformamide (DMF) (30 mL) at 0 °C was added K₂CO₃ (5.35 g, 38.7 mmol), potassium iodide (6.43 g, 38.7 mmol) and bromocyclobutane (5.23 g, 38.7 mmol). It was heated at 110 °C for 48h. The reaction was quenched with water (100 mL), extracted with ethyl acetate (2 x 50 mL). The combined organic layer was washed with brine (20 mL) and concentrated. The crude residue was purified with flash column chromatography eluting with EtOAc: hexane (3:.7) to give the title compound (4.5 g, 10.19 mmol, 26.3 % yield) as gummy liquid. LCMS *m*/*z* 442.27 (M+H)⁺, 3.918 min (ret. time)

### tert-Butyl (3-((cyclopropylmethyl)amino)-2,2-dimethylpropyl)carbamate

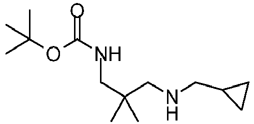

To a solution of tert-butyl (3-(N-(cyclopropylmethyl)-2-nitrophenylsulfonamido)-2,2-dimethylpropyl)carbamate (4.5 g, 10.19 mmol) in N,N-dimethylformamide (DMF) (30 mL) at 0 °C was dded K₂CO₃ (1.409 g, 10.19 mmol) and thiophenol (1.049 mL, 10.19 mmol). It was stirred at ambient temperature for 6 h. It was quenched with water (20 mL) and acidified with 1N HCl. It was extracted with ethyl acetate (2 x 40 mL). The aqueous layer was basified with 1N NaOH and extracted with ethyl acetate (2 x 40 mL). This combined organic layer was washed with brine (20 mL) and concentrated to give the title compound (2.3 g, 8.97 mmol, 88 % yield) as a gummy liquid. ¹H NMR (400 MHz, CDCl₃) δ ppm 0.05 - 0.14 (m, 2 H), 0.42 - 0.50 (m, 2 H), 0.87 - 0.96 (m, 7 H), 1.41 - 1.48 (m, 9 H), 2.18-2.32 (m, 2H), 2.40 - 2.46 (m, 2 H), 2.59 - 2.68 (m, 2 H), 5.86 (br s, 1 H)

### tert-Butyl (3-(2-chloro-N-(cyclopropylmethyl)acetamido)-2,2-dimethylpropyl)carbamate

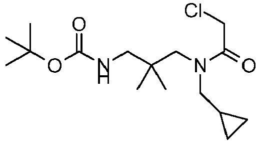

To a solution of tert-butyl (3-((cyclopropylmethyl)amino)-2,2-dimethylpropyl)carbamate (2.3 g, 8.97 mmol) in dichloromethane (DCM) (20 mL) at 0 °C was added TEA (1.876 mL, 13.46 mmol) and chloroacetyl chloride (0.862 mL, 10.77 mmol). It was stirred at ambient temperature for 3 h under nitrogen. The reaction was quenched with saturated sodium bicarbonate solution (20 mL) and extracted with DCM (2 x 20 mL). The combined organic layer was concentrated. The crude residue was purified with flash column chromatography eluting with EtOAc: hexane (2:8) to give the title compound (1.9 g, 5.71 mmol, 63.6 % yield) as yellow liquid. LC-MS *m*/*z* 333.23 (M+H)⁺, 3.748 min (ret. time)

### tert-Butyl 4-(cyclopropylmethyl)-6,6-dimethyl-3-oxo-1,4-diazepane-1-carboxylate

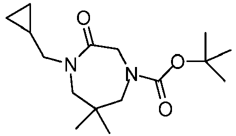

To a solution of tert-butyl (3-(2-chloro-N-(cyclopropylmethyl)acetamido)-2,2-dimethylpropyl)carbamate (1.9 g, 5.71 mmol) in N,N-dimethylformamide (DMF) (15 mL) at 0 °C was added NaH (0.137 g, 5.71 mmol). The reaction mixture was stirred at ambient temperature for 16 h. The reaction was quenched with ice water (30 mL) and extracted with EtOAc (3 x 20 ml). The combined organic layer was dried over anhydrous Na₂SO₄, filtered and concentrated. The crude residue was purified with flash column chromatography eluting with EtOAc: hexane (4:6) to give the title compound (800 mg, 2.390 mmol, 41.9 % yield) as yellow liquid. LC-MS *m*/*z* 297.1 (M+H)⁺, 5.461 min (ret. time)

### 3-(3-((4-(Cyclopropylmethyl)-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid

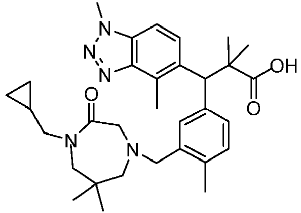

A mixture of tert-butyl 4-(cyclopropylmethyl)-6,6-dimethyl-3-oxo-1 ,4-diazepane-1-carboxylate (96 mg, 0.325 mmol) and 4 M HCI in dioxane (0.163 mL, 0.650 mmol) in DCM (2 mL) was stirred at ambient temperature for 18 h. The solvent was concentrated and the crude product was re-dissolved in 1 mL CH₃CN and then added to a mixture of methyl 3-(3-(chloromethyl)-4-methylphenyl)-3-(1,4-dimethyl-1H-benzo[d][1,2,3]triazol-5-yl)-2,2-dimethylpropanoate (65 mg, 0.163 mmol) and DIEA (0.114 mL, 0.650 mmol) in acetonitrile (3 mL). The reaction mixture was heated in a Biotage microwave at high absorption for 1 h at 120 °C. The solvent was concentrated and the crude product was re-dissolved in methanol (3.00 mL), 2 M LiOH (0.488 mL, 0.975 mmol) was added and the reaction heated in a Biotage microwave at high absorption for 1 h at 100 °C, another 1 h at 120 °C. 1N HCI (1mL) was added and then extracted with ethyl acetate (2 x 5 mL). The aqueous layer was acidified with 1N HCl to pH around 2 and then extracted with 1:4 ratio IPA:DCM (2 x 8 mL). This organic layer was concentrated to give the crude product. It was purified with neutral with preparative HPLC to give the title compound (22 mg, 0.040 mmol, 24.80 % yield). LC-MS *m*/*z* 546.2 (M+H)⁺, 0.97 min (ret. time)

### Example 10 rel-3S-(1,4-Dimethyl-1H-benzo[d][1,2,3]triazol-5-yl)-2,2-dimethyl-3-(rel-(R)-3-(4,6,6-trimethyl-3-oxo-1,4-diazepan-1-yl)-2,3-dihydro-1H-inden-5-yl)propanoic acid

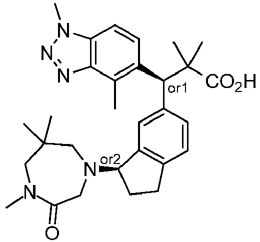

### 6-Bromo-2,3-dihydro-1H-inden-1-ol

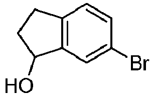

To a solution of 6-bromo-2,3-dihydro-1H-inden-1-one (5 g, 23.69 mmol) in methanol (100 mL) was added sodium borohydride (0.986 g, 26.1 mmol) under N₂. The reaction mixture was stirred at ambient temperature for 2 hrs. The solvent was removed by reduced pressure. The residue was re-dissolved in 100 mL of ethyl acetate and 20 mL of 1N HCl. The organic layer was separated and the aqueous layer was extracted with ethyl acetate (3x). The combined organic layer was washed with brine and dried over anhydrous Na₂SO₄. After filtration and concentration, the title compound 6-bromo-2,3-dihydro-1H-inden-1-ol (4.9 g, 23.0 mmol, 97%) was obtained and was carried over to next step without further purification. LC-MS *m*/*z* 197.0 (M+H-18)⁺, 1.80 min (ret. time)

### ((6-Bromo-2,3-dihydro-1H-inden-1-yl)oxy)(tert-butyl)dimethylsilane

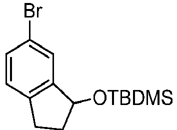

To a solution of 6-bromo-2,3-dihydro-1H-inden-1-ol (4.9 g, 23.00 mmol) in dichloromethane (DCM) (50 mL) at 0 °C, imidazole (3.13 g, 46.0 mmol), DMAP (0.140 g, 1.150 mmol) and tert-butylchlorodimethylsilane (5.20 g, 34.5 mmol) were added. The reaction mixture was stirred at 0 °C to 25 °C for 2 hrs. The reaction mixture was quenched with water and extracted with DCM (3x). The combined organic layer was washed with water (2x) and brine (2x), dried over Na₂SO₄ and concentrated. The residue was purified with silica gel chromatography (25 g, PE:EA= 5%) to give ((6-bromo-2,3-dihydro-1H-inden-1-yl)oxy)(tert-butyl)dimethylsilane (5.53 g, 15.67 mmol, 68.1 % yield) as yellow oil. LC-MS *m*/*z* doesn't show MS, 2.81 min (ret. time)

### 3-((tert-Butyldimethylsilyl)oxy)-2,3-dihydro-1H-indene-5-carbaldehyde

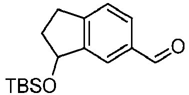

To a solution of ((6-bromo-2,3-dihydro-1H-inden-1-yl)oxy)(tert-butyl)dimethylsilane (5.53 g, 16.89 mmol) in tetrahydrofuran (THF) (60 mL) was added butyllithium (2.5M, 8.11 mL, 20.27 mmol) at -78 °C. The reaction mixture was stirred at -78 °C for half an hour under N₂, then DMF (6.54 mL, 84 mmol) was slowly added into the reaction. The reaction mixture was continuously stirred for 2 hrs at -78 °C. Then the reaction was quenched with saturated NH₄Cl aqueous solution. The mixture was extracted with ethyl acetate (3x). The combined organic layer was washed with brine and dried over anhydrous Na₂SO₄. After filtration and concentration, the residue was purified with silica gel chromatography (PE:EA= 4:1) to provide the title compound (3-((tert-butyldimethylsilyl)oxy)-2,3-dihydro-1H-inden-5-yl)(1,4-dimethyl-1H-benzo[d][1,2,3]triazol-5-yl)methanol (4.4 g, 15.92 mmol, 94%). LC-MS *m*/*z* doesn't show MS, 2.49 min (ret. time)

### 3-((tert-Butyldimethylsilyl)oxy)-2,3-dihydro-1H-inden-5-yl)(1,4-dimethyl-1H-benzo[d][1,2,3]triazol-5-yl)methanol

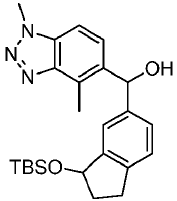

To a solution of 5-bromo-1,4-dimethyl-1H-benzo[d][1,2,3]triazole (3.60 g, 15.92 mmol) in tetrahydrofuran (THF) (100 mL) was added tert-butyllithium (13.47 mL, 17.51 mmol) at -78 °C under nitrogen. The reaction mixture was stirred at -78 °C for 1 h, then 3-((tertbutyldimethylsilyl)oxy)-2,3-dihydro-1H-indene-5-carbaldehyde (4.40 g, 15.92 mmol) in THF (20 mL) was slowly added. The reaction mixture was stirred at -78 °C for 2 h and ambient temperature for 8 h. The reaction was quenched with saturated NH₄Cl solution and extracted with ethyl acetate (3x). The combined organic layer was washed with brine, dried over anhydrous Na₂SO₄ and concentrated. The residue was purified silica gel chromatographyy (petroleum ether:ethyl acetate=1:1) to provide the title compound (3-((tertButyldimethylsilyl)oxy)-2,3-dihydro-1H-inden-5-yl)(1,4-dimethyl-1H-benzo[d][1,2,3]triazol-5-yl)methanol (2.24 g, 11.76 mmol, 11.08% yield). LC/MS *m*/*z* 424.1 (M+H)⁺, 1.97 (ret. time).

### Methyl 3-(3-((tert-butyldimethylsilyl)oxy)-2,3-dihydro-1H-inden-5-yl)-3-(1,4-dimethyl-1H-benzo[d][1,2,3]triazol-5-yl)-2,2-dimethylpropanoate

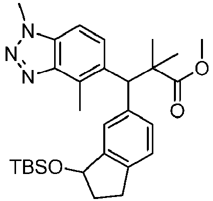

To a solution of (3-((tert-butyldimethylsilyl)oxy)-2,3-dihydro-1H-inden-5-yl)(1,4-dimethyl-1H-benzo[d][1,2,3]triazol-5-yl)methanol (2 g, 4.72 mmol) in dry acetonitrile (40 mL), DBU (0.014 mL, 0.094 mmol) and 2,2,2-trichloroacetonitrile (0.818 g, 5.67 mmol) were added slowly under nitrogen at 25 °C. The reaction mixture was stirred at 25 °C for half an hour after which ((1-methoxy-2-methylprop-1-en-1-yl)oxy)trimethylsilane (2.057 g, 11.80 mmol) was added, followed by 1,1,1-trifluoro-N-((trifluoromethyl)sulfonyl)methanesulfonamide (0.066 g, 0.236 mmol). The reaction mixture was stirred at 25 °C for 2 h after which 40 mL of H₂O was added to quench the reaction and extracted with ethyl acetate (3x). The combined organic layer was washed with brine and concentrated. The residue was purified by silica gel chromatography (petroleum ether:ethyl acetate=4:1) to give the title compound methyl 3-(3-((tert-butyldimethylsilyl)oxy)-2,3-dihydro-1H-inden-5-yl)-3-(1,4-dimethyl-1H-benzo[d][1,2,3]triazol-5-yl)-2,2-dimethylpropanoate as a yellow solid. LC/MS *m*/*z* 508.2 (M+H)⁺, 2.52 (ret. time).

### Methyl 3-(1,4-dimethyl-1H-benzo[d][1,2,3]triazol-5-yl)-3-(3-hydroxy-2,3-dihydro-1H-inden-5-yl)-2,2-dimethylpropanoate

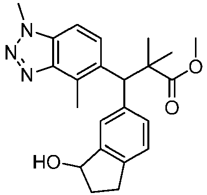

To a solution of methyl 3-(3-((tert-butyldimethylsilyl)oxy)-2,3-dihydro-1H-inden-5-yl)-3-(1,4-dimethyl-1H-benzo[d][1,2,3]triazol-5-yl)-2,2-dimethylpropanoate (1.1 g, 2.166 mmol) in tetrahydrofuran (THF) (15 mL) at 0 °C, TBAF (0.623 g, 2.383 mmol) was added. The reaction mixture was stirred at 0 °C for 1 h after which the mixture was quenched with saturated NH₄Cl solution (20 mL) and extracted with ethyl acetate (3x). The combined organic layer was washed with water, brine, dried over Na₂SO₄ and concentrated. The residue was purified by silica gel chromatographyy (petroleum ether:ethyl acetate=1:1) to provide the title compound methyl 3-(1,4-dimethyl-1H-benzo[d][1,2,3]triazol-5-yl)-3-(3-hydroxy-2,3-dihydro-1H-inden-5-yl)-2,2-dimethylpropanoate (650 mg, 1.487 mmol, 68.6 % yield) as a yellow oil. LC/MS *m*/*z* 394.1 (M+H)⁺, 1.88 (ret. time).

### Methyl 3-(1,4-dimethyl-1H-benzo[d][1,2,3]triazol-5-yl)-2,2-dimethyl-3-(3-oxo-2,3-dihydro-1H-inden-5-yl)propanoate

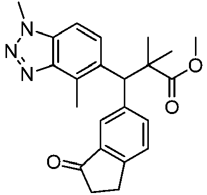

To a solution of methyl 3-(1,4-dimethyl-1H-benzo[d][1,2,3]triazol-5-yl)-3-(3-hydroxy-2,3-dihydro-1H-inden-5-yl)-2,2-dimethylpropanoate (650 mg, 1.652 mmol) in dichloromethane (DCM) (50 mL) was added Dess-Martin periodinane (1401 mg, 3.30 mmol) and one drop of water. The reaction mixture was stirred at 25 °C for 8 h after which the mixture was filtered and the filtrate was concentrated. The crude prodcut was purified by silica gel chromatographyy (petroleum ether:ethyl acetate= 1:1) to provide the title compound methyl 3-(1 ,4-dimethyl-1H-benzo[d][1 ,2,3]triazol-5-yl)-2,2-dimethyl-3-(3-oxo-2,3-dihydro-1 H-inden-5-yl)propanoate (570 mg) as a yellow oil. LC/MS *m*/*z* 392.2 (M+H)⁺, 1.65 (ret. time).

### rel-(S)-Methyl 3-(1,4-dimethyl-1H-benzo[d][1,2,3]triazol-5-yl)-2,2-dimethyl-3-(3-oxo-2,3-dihydro-1H-inden-5-yl)propanoate

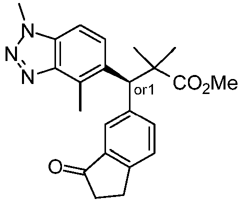

Methyl 3-(1,4-dimethyl-1H-benzo[d][1,2,3]triazol-5-yl)-2,2-dimethyl-3-(3-oxo-2,3-dihydro-1H-inden-5-yl)propanoate (0.57 g, 1.456 mmol) was resolved by chiral SFC (Column: Chiralpak IA 20x250mm, 5u; Co-solvent: 20% EtOH; Flowrate: 50 g/min; Back presure: 100Bar) to give single enantiomerically pure rel-(R)-methyl 3-(1,4-dimethyl-1H-benzo[d][1,2,3]triazol-5-yl)-2,2-dimethyl-3-(3-oxo-2,3-dihydro-1H-inden-5-yl)propanoate (peak 1) (177.9 mg, 0.454 mmol, 31.2 % yield). (chiral SFC ret. time: 2.91 min) LCMS m/z 392.2 (M+H)⁺, 0.99 min (ret. time) and rel-(R)-methyl 3-(1,4-dimethyl-1H-benzo[d][1,2,3]triazol-5-yl)-2,2-dimethyl-3-(3-oxo-2,3-dihydro-1H-inden-5-yl)propanoate (peak 2) (184.4 mg, 0.471 mmol, 32.4 % yield) (chiral SFC ret. time: 3.93 min) LCMS m/z 392.2 (M+H)⁺, 0.99 min (ret. time)

### rel-(3S)-Methyl 3-(1,4-dimethyl-1H-benzo[d][1,2,3]triazol-5-yl)-3-(3-hydroxy-2,3-dihydro-1H-inden-5-yl)-2,2-dimethylpropanoate

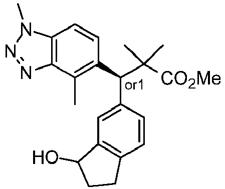

To a solution of rel-(S)-methyl 3-(1,4-dimethyl-1H-benzo[d][1,2,3]triazol-5-yl)-2,2-dimethyl-3-(3-oxo-2,3-dihydro-1H-inden-5-yl)propanoate (peak 1) (177 mg, 0.452 mmol) in methanol (3.5 mL) was added NaBH₄ (17.11 mg, 0.452 mmol). The reaction was stirred at ambient temperature for 30 min then concentrated under reduced pressure, extracted with DCM (2 x 5 mL), dried over Na₂SO₄, filtered, and concentrated under reduced pressure to give the title compound (203.9 mg, 0.518 mmol, 115 % yield). LCMS m/z 394.2 (M+H)⁺, 0.96 min (ret. time).

The compounds in Table 1 were prepared by a method similar to the one described for the preparation of rel-(3S)-Methyl 3-(1,4-dimethyl-1H-benzo[d][1 ,2,3]triazol-5-yl)-3-(3-hydroxy-2,3-dihydro-1H-inden-5-yl)-2,2-dimethylpropanoate. As is appreciated by those skilled in the art, these analogous examples may involve variations in general reaction conditions.

**Table 1**

| Structure | Name | LCMS [M+H]⁺ | Retention Time (min) |
|---|---|---|---|
| 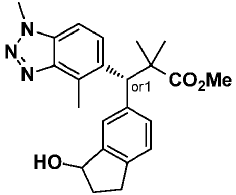 | rel-(3R)-methyl 3-(1,4-dimethyl-1 H-benzo[d][1 ,2,3]triazol-5-yl)-3-(3-hydroxy-2,3-dihydro-1 H-inden-5-yl)-2,2-dimethylpropanoate | 394.2 | 0.96 |

### rel-3S-(1,4-Dimethyl-1H-benzo[d][1,2,3]triazol-5-yl)-2,2-dimethyl-3-(rel-(R)-3-(4,6,6-trimethyl-3-oxo-1,4-diazepan-1-yl)-2,3-dihydro-1H-inden-5-yl)propanoic acid

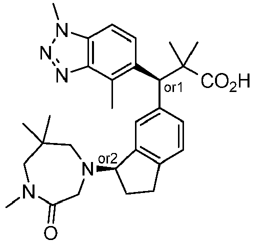

To the mixture of rel-(3S)-methyl 3-(1,4-dimethyl-1H-benzo[d][1,2,3]triazol-5-yl)-3-(3-hydroxy-2,3-dihydro-1H-inden-5-yl)-2,2-dimethylpropanoate (100 mg, 0.254 mmol) in dichloromethane (1.0 mL) was added SOCl₂ (0.037 mL, 0.508 mmol). The resulting reaction mixture was stirred at ambient temperature for 30 min then the solvent was removed under vacuum. The resulting residue was dissolved in acetonitrile (3.0 mL) and tetrahydrofuran (1.0 mL) to give intermediate solution.

To tert-butyl 4,6,6-trimethyl-3-oxo-1,4-diazepane-1-carboxylate (130 mg, 0.508 mmol) was added HCI (4.0 M in p-dioxane) (0.635 mL, 2.54 mmol). The resulting reaction mixture was stirred at ambient temperature for 20 h then the solvent was removed under vacuum. The resulting residue was added the above intermediate solution followed by sodium iodide (19.05 mg, 0.127 mmol) and K₂CO₃ (140 mg, 1.017 mmol). The resulting reaction mixture was stirred at 40 °C for 21 h then filtered. The filter cake was washed with acetonitrile (3 mL)and the filtrate was concentrated under vacuum. The resulting residue was dissolved in methanol (3.0 mL) then was added NaOH (3 N) (0.678 mL, 2.033 mmol). The resulting reaction mixture was heated with microwave at 130 °C for 1 h. The reaction mixture was acidified with HCI (3 N) (0.678 mL, 2.033 mmol), evaporated down under vacuum, purified by reverse phase HPLC (formic acid modifier) then further separated with chiral SFC (Chiralpak IA 20x250mm, 5u; Co-solvent: 25% MeOH; Flowrate: 50 g/min; Back presure: 100Bar) to give the title compound (32.5 mg, 0.063 mmol, 24.70 % yield). LC/MS: *m*/*z* 518.1 (M+H)⁺, 0.79 min (ret. time).

### Example 11 rel-3S-(1,4-Dimethyl-1H-benzo[d][1,2,3]triazol-5-yl)-2,2-dimethyl-3-(rel-(S)-3-(4,6,6-trimethyl-3-oxo-1,4-diazepan-1-yl)-2,3-dihydro-1H-inden-5-yl)propanoic acid

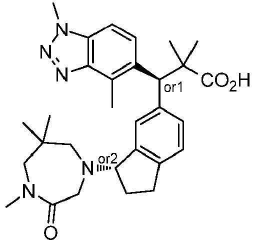

To the mixture of rel-(3S)-methyl 3-(1,4-dimethyl-1H-benzo[d][1,2,3]triazol-5-yl)-3-(3-hydroxy-2,3-dihydro-1H-inden-5-yl)-2,2-dimethylpropanoate (100 mg, 0.254 mmol) in dichloromethane (1.0 mL) was added SOCl₂ (0.037 mL, 0.508 mmol). The resulting reaction mixture was stirred at ambient temperature for 30 min then the solvent was removed under vacuum. The resulting residue was dissolved in acetonitrile (3.0 mL) and tetrahydrofuran (1.0 mL) to give intermediate solution.

To tert-butyl 4,6,6-trimethyl-3-oxo-1,4-diazepane-1-carboxylate (130 mg, 0.508 mmol) was added HCI (4.0 M in p-dioxane) (0.635 mL, 2.54 mmol). The resulting reaction mixture was stirred at ambient temperature for 20 h then the solvent was removed under vacuum. The resulting residue was added the above intermediate solution followed by sodium iodide (19.05 mg, 0.127 mmol) and K₂CO₃ (140 mg, 1.017 mmol). The resulting reaction mixture was stirred at 40 °C for 21 h then was filtered. The filter cake was washed with acetonitrile (3 mL)and the filtrate was concentrated under vacuum. The resulting residue was dissolved in methanol (3.0 mL) then was added NaOH (3 N) (0.678 mL, 2.033 mmol). The resulting reaction mixture was heated with microwave at 130 °C for 1 h. The reaction mixture was acidified with HCI (3 N) (0.678 mL, 2.033 mmol), evaporated down under vacuum, purified by reverse phase HPLC (formic acid modifier) then further separated with chiral SFC (Chiralpak IA 20x250mm, 5u; Co-solvent: 25% MeOH; Flowrate: 50 g/min; Back presure: 100Bar) to give the title compound (13.5 mg, 0.026 mmol, 10.26 % yield). LC/MS: *m*/*z* 518.1 (M+H)⁺, 0.78min (ret. time).

### Example 12 rel-(3R)-3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid

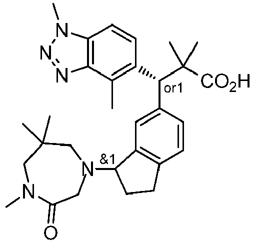

To the mixture of rel-(3R)-methyl 3-(1,4-dimethyl-1H-benzo[d][1,2,3]triazol-5-yl)-3-(3-hydroxy-2,3-dihydro-1H-inden-5-yl)-2,2-dimethylpropanoate (100 mg, 0.254 mmol) in dichloromethane (1.0 mL) was added SOCl₂ (0.037 mL, 0.508 mmol). The resulting reaction mixture was stirred at ambient temperature for 30 min then the solvent was removed under vacuum. The residue was dissolved in acetonitrile (3.0 mL) and tetrahydrofuran (1.0 mL) to give intermediate solution.

To tert-butyl 4,6,6-trimethyl-3-oxo-1,4-diazepane-1-carboxylate (130 mg, 0.508 mmol) was added HCI (4.0 M in p-dioxane) (0.635 mL, 2.54 mmol). The resulting residue was added the above intermediate solution then sodium iodide (19.05 mg, 0.127 mmol) and K₂CO₃ (140 mg, 1.017 mmol). The resulting reaction mixture was stirred at 40 °C for 21 h. The reaction mixture was filtered. The filter cake was washed with acetonitrile (3 mL)and the filtrate was concentrated under vacuum then was dissolved in methanol (3.0 mL) and was added NaOH (3 N) (0.678 mL, 2.033 mmol). The resulting reaction mixture was heated with microwave at 130 °C for 1 h. The reaction mixture was acidified with HCl (3 N) (0.678 mL, 2.033 mmol), evaporated down under vacuum, purified by reverse phase HPLC (formic acid modifier) to give the title compound (1.8 mg, 3.48 µmol, 1.368 % yield). LC/MS: *m*/*z* 518.4 (M+H)⁺, 0.77min (ret. time).

### Example 13 3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid

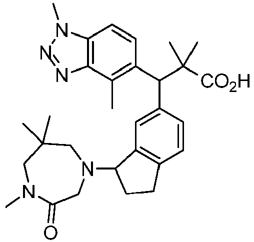

To the mixture of methyl 3-(1,4-dimethyl-1H-benzo[d][1,2,3]triazol-5-yl)-3-(3-hydroxy-2,3-dihydro-1H-inden-5-yl)-2,2-dimethylpropanoate (0.060 g, 0.152 mmol) in dichloromethane (0.50 mL) was added SOCl₂ (0.022 mL, 0.305 mmol). The resulting reaction mixture was stirred at ambient temperature for 10 min then the solvent was removed under vacuum. The residue was dissolved in acetonitrile (1.5 mL) and tetrahydrofuran (0.50 mL) to give intermediate solution.

To tert-butyl 4,6,6-trimethyl-3-oxo-1,4-diazepane-1-carboxylate (0.078 g, 0.305 mmol) was added HCI (4.0 M in p-dioxane) (0.381 mL, 1.525 mmol). The resulting reaction mixture was stirred at ambient temperature for 1 h then was evaporated down under. To this residue was added the above intermediate solution followed by sodium iodide (0.011 g, 0.076 mmol) and K₂CO₃ (0.084 g, 0.610 mmol). The resulting reaction mixture was stirred at 40 °C for 16 h. The reaction mixture was filtered. The filter cake was washed with acetonitrile (2 mL)and the filtrate was concentrated under vacuum. The resulting residue was dissolved in methanol (1.5 mL) then was added NaOH (3 N) (0.407 mL, 1.220 mmol). The resulting reaction mixture was heated with microwave at 130 °C for 1 h then was acidified with HCI (3 N) (0.407 mL, 1.220 mmol), evaporated down under vacuum, purified by reverse phase HPLC (formic acid modifier) to give the title compound (26.4 mg, 0.051 mmol, 33.4 % yield). LC/MS: *m*/*z* 518.5 (M+H)⁺, 0.78min (ret. time).

### Example 14 3-((1-Ethyl-1H-1,2,3-triazol-4-yl)methoxy)-2,2-dimethyl-3-(4-methyl-3-((4,6,6-trimethyl-3-oxo-1,4-diazepan-1-yl)methyl)phenyl)propanoic acid

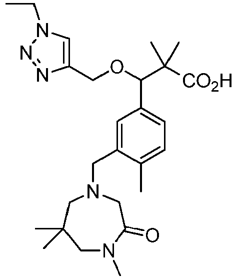

### (5-Bromo-2-methylphenyl)methanol

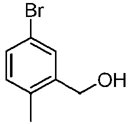

To a solution of 5-bromo-2-methylbenzoic acid (70 g, 326 mmol) in tetrahydrofuran (THF) (700 mL) stirred under nitrogen at 0 °C was added a toluene solution of borane-methyl sulfide complex (244 mL, 488 mmol) drop wise during 15 min. The reaction mixture was stirred for 16 h. The reaction was cooled to 0 °C and quenched with methanol (500 mL) drop wise. The reaction mixture was stirred at ambient temperature for 3 h and then concentrated. The crude residue was diluted with ethyl acetate (1 L) and washed with 1N HCI (500 mL), brine solution (500 mL) and dried over Na₂SO₄, filtered and concentrated to give the title compound (49 g, 244 mmol, 74.9 % yield). ¹H NMR (400 MHz, DMSO) δ= 7.52 (d, *J* = 2.6 Hz, 1H), 7.31 (dd, *J* = 8.0, 2.2 Hz, 1H), 7.12-7.03 (m, 1H), 5.22 (td, *J* = 5.5, 1.8 Hz, 1H), 4.48 (dd, *J* = 5.1, 1.8 Hz, 2H), 2.17 (s, 3H).

### 4-Bromo-2-(((4-methoxybenzyl)oxy)methyl)-1-methylbenzene

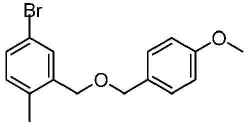

To a stirred solution of (5-bromo-2-methylphenyl)methanol (100 g, 497 mmol) in dry DMF (800 mL) was added NaH (21.88 g, 547 mmol). After the reaction mixture was stirred for 30 minutes, 1-(chloromethyl)-4-methoxybenzene (82 g, 522 mmol) was added at 0 °C and the reaction mixture was stirred for another 2 h at ambient temperature. The reaction was then diluted with Et₂O (200 mL) and water (200 mL). The organic phase was washed with brine (300 mL) and dried with Na₂SO₄ and concentrated under reduced pressure. The residue was purified via silica gel column to yield 4-bromo-2-(((4-methoxybenzyl)oxy)methyl)-1-methylbenzene (140 g, 436 mmol, 88 % yield) as a clear oil. ¹H NMR (400 MHz, CHLOROFORM-d) δ ppm 2.27 (s, 3 H) 3.84 (s, 3 H) 4.49 (s, 2 H), 4.54 (s, 2H), 6.92 (d, *J* = 8.8, 2H), 6.94 (d, *J* = 8.4, 1H), 7.31-7.35 (m, 3H), 7.54 (d, *J* = 2, 1H).

### 3-(4-Methoxybenzyl)oxy)methyl)-4-methylbenzaldehyde

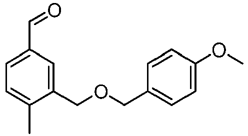

To a stirred solution of 4-bromo-2-(((4-methoxybenzyl)oxy)methyl)-1-methylbenzene (80 g, 249 mmol) in THF (800 mL) at -78 °C under N₂, 2.5 M n-BuLi in hexane (120 mL, 299 mmol) was carefully added. The reaction mixture was stirred at -78 °C for 65 min, and then DMF (38.6 mL, 498 mmol) was added. The reaction mixture was stirred at -78 °C to 25 °C for another 30 min. The mixture was quenched with saturated NH₄Cl (300 mL), and extracted with EtOAc (2 x 500 mL). The organic layer was washed with water (300 mL) and brine (2 x 100 mL), dried (Na₂SO₄) and concentrated. The residue was washed with petroleum ether: EtOAc =10 / 1 (2000 mL) to give the title compound (50 g, 185 mmol, 74.3 % yield) as a solid. LC-MS m/z 288.1 (M+H₂O)⁺, 2.04 min (ret. time).

### Methyl 3-hydroxy-3-(3-(((4-methoxybenzyl)oxy)methyl)-4-methylphenyl)-2,2-dimethylpropanoate

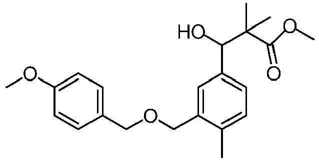

A mixture of 3-(((4-methoxybenzyl)oxy)methyl)-4-methylbenzaldehyde (4 g, 14.80 mmol), 1-methylimidazole (0.118 mL, 1.480 mmol), ((1-methoxy-2-methylprop-1-en-1-yl)oxy)trimethylsilane (5.16 g, 29.6 mmol) and lithium chloride (0.125 g, 2.96 mmol) in N,N-dimethylformamide (DMF) (15 mL) was stirred at ambient temperature for 17 h. NaOH (14.80 mL, 14.80 mmol) was added and the reaction mixture was stirred for 2 h. Water (20 mL) and potassium hydrogen fluoride (1.734 g, 22.20 mmol) were added and stirred for 2 h, followed by adding of 1N HCI (14.80 mL, 14.80 mmol). It was stirred for 16 h. The reaction mixture was diluted with ice water (10 mL) and extracted with EtOAc (2 x 20 mL). The combined organic layer was washed with brine solution (20 mL), dried over Na₂SO₄, filtered and concentrated. The crude residue was purified with flash column chromatography eluting with EtOAc: Hexane (2:8) to give the title compound (2 g, 5.37 mmol, 36.3 % yield) as yellow liquid. LCMS *m*/*z* 390.23 (M+18)⁺, 4.07 min (ret. time)

### Methyl 3-(3-(((4-methoxybenzyl)oxy)methyl)-4-methylphenyl)-2,2-dimethyl-3-(prop-2-yn-1-yloxy)propanoate

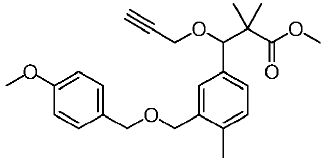

To a solution of methyl 3-hydroxy-3-(3-(((4-methoxybenzyl)oxy)methyl)-4-methylphenyl)-2,2-dimethylpropanoate (24 g, 64.4 mmol) and 3-bromoprop-1-yne in toluene (14.37 g, 97 mmol) in N,N-dimethylformamide (DMF) (250 mL) at 0°C was added NaH (2.320 g, 97 mmol) under nitrogen. It was stirred for 30 min. The reaction was quenched with saturated NaHCO₃ solution and extracted with EtOAc. The organic layer was concentrated under vacuum to afford the title compound (26 g, 55.2 mmol, 86 % yield) as liquid. LCMS m/z 428.17 (M+18)⁺, 2.92 min (ret.ime)

### Methyl 3-(3-(hydroxymethyl)-4-methylphenyl)-2,2-dimethyl-3-(prop-2-yn-1-yloxy)propanoate

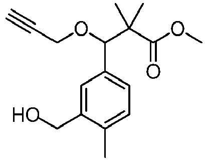

To a solution of methyl 3-(3-(((4-methoxybenzyl)oxy)methyl)-4-methylphenyl)-2,2-dimethyl-3-(prop-2-yn-1-yloxy)propanoate (13 g, 31.7 mmol) in dichloromethane (DCM) (40 mL) and water (10 mL) at 0 °C was added DDQ (10.78 g, 47.5 mmol). It was stirred at ambient temperature for 30 min. It was quenched with ice water, extracted with DCM twice. The organic layer was dried under anhydrous Na₂SO₄, filtered and concentrated. The crude residue was purified on flash column chromatography eluting with EtOAc: hexane (12:88). Desired fractions were concentrated under to give the title compound (8 g, 13.63 mmol, 43.0 % yield) as a colorless liquid. LC MS *m*/*z* 291.20 (M+H)⁺, 2.20 min (ret. time)

### Methyl 3-((1-ethyl-1H-1,2,3-triazol-4-yl)methoxy)-3-(3-(hydroxymethyl)-4-met hylphenyl)-2,2-dimethylpropanoate

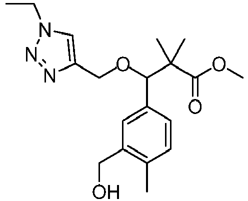

To the mixture of methyl 3-(3-(hydroxymethyl)-4-methylphenyl)-2,2-dimethyl-3-(prop-2-yn-1-yloxy)propanoate (1.452 g, 5.0 mmol) in isopropanol (10 mL), tetrahydrofuran (5 mL) and water (4 mL) was added sodium azide (0.813 g, 12.50 mmol), DIEA (0.175 mL, 1.000 mmol), iodoethane (0.929 mL, 11.50 mmol) and copper(I) iodide (0.143 g, 0.750 mmol). The resulting reaction mixture was heated with microwave at 80 °C for 1 h. The reaction mixture was evaporated down, purified with flash chromatography over silica gel column to give the title compound (1.2142 g, 3.36 mmol, 67.2 % yield). LC/MS: *m*/*z* 362.1 (M+H)⁺, 0.82 min (ret. time).

### 3-((1-Ethyl-1H-1,2,3-triazol-4-yl)methoxy)-2,2-dimethyl-3-(4-methyl-3-((4,6,6-trimethyl-3-oxo-1,4-diazepan-1-yl)methyl)phenyl)propanoic acid

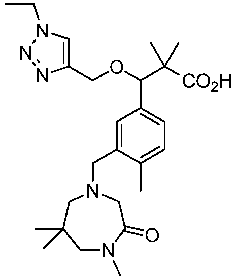

To the mixture of methyl 3-((1-ethyl-1H-1,2,3-triazol-4-yl)methoxy)-3-(3-(hydroxymethyl)-4-methylphenyl)-2,2-dimethylpropanoate (70 mg, 0.194 mmol) in dichloromethane (0.5 mL) was added SOCl₂ (0.028 mL, 0.387 mmol). The resulting reaction mixture was stirred at ambient temperature for 20 min then the solvent was removed under vacuum. The residue was dissolved in N,N-dimethylformamide (3.0 mL) then was added 1,6,6-trimethyl-1,4-diazepan-2-one, hydrochloride (56.0 mg, 0.291 mmol) and DIEA (0.135 mL, 0.775 mmol). The resulting reaction mixture was heated with microwave at 100 °C for 1 h. The solvent was removed under vacuum. The resulting residue was dissolved in methanol (2.0 mL) then was added NaOH (3.0 N) (0.516 mL, 1.549 mmol). The resulting reaction mixture was heated with microwave at 100 °C for 60 min, heated again with microwave at 120 °C for 60 min. To the reaction mixture was added more methanol (2.0 mL) and NaOH (3.0 N) (0.516 mL, 1.549 mmol). The resulting reaction mixture was heated wtih microwave at 120 °C for 60 min. The reaction mixture was acidified with HCl (3.0 N) (0.516 mL, 1.549 mmol), evaporated down under vacuum, purified by reverse phase HPLC (formic acid modifier) to give the title compound (25.1 mg, 0.052 mmol, 26.7 % yield). LC/MS: *m*/*z* 486.3 (M+H)⁺, 0.80 min (ret. time).

### Example 15

### 3-(4-Chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoic acid

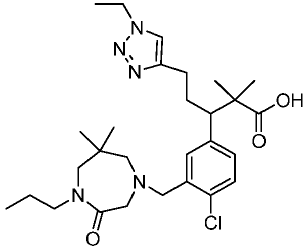

### (5-Bromo-2-chlorophenyl)methanol

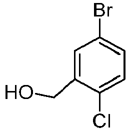

To a solution of 5-bromo-2-chlorobenzoic acid (5 g, 21.23 mmol), in tetrahydrofuran (THF) (10 mL), 10 M borane-methyl sulfide complex (2.123 mL, 21.23 mmol) was added at 0 °C. The reaction was then warmed to 23 °C and stirred for 14h. The reaction mixture was cooled and additional 10 M borane-methyl sulfide complex (1.062 mL, 10.62 mmol) was added to the reaction mixture. Afterwards, the reaction was cooled on an ice water bath and methanol (2.00 mL) was added slowly. When most of the bubbling ceased the volatile solvents were removed in vacuo and the residue was dissolved in EtOAc (10ml) and washed with saturated aq. NaHCO₃. The aqueous layer was back extracted with EtOAc (50ml) and the combined EtOAc layers were washed with water (5ml) and saturated aq. NaCl, dried with Na₂SO₄ and concentrated to afford the title compound. (4.62 g, 98% yield) ¹H NMR (400 MHz, METHANOL-d₄) δ 7.70 (s, 1H), 7.40 (d, *J*=8.28 Hz, 1H), 7.28 (d, *J*=8.53 Hz, 1H), 4.66 (s, 2H)

### 4-Bromo-1-chloro-2-(((4-methoxybenzyl)oxy)methyl)benzene

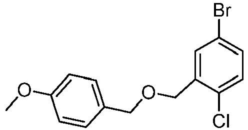

A solution of (5-bromo-2-chlorophenyl)methanol (5.361g, 24.21 mmol) in N,N-dimethylformamide (DMF) (40 mL) under argon, was cooled in an ice-bath and 60% sodium hydride (1.936 g, 48.4 mmol) was added carefully in several portions. The reacton was stirred at 23 °C for 1 h and then cooled to 10 °C in an ice bath. 1-(chloromethyl)-4-methoxybenzene (4.92 mL, 36.3 mmol) was added and the reaction was stirred at 23 °C for 14 h. The reaction was quenched with water (25 mL), stirred for 5 min and diluted with water (100 mL) and EtOAc (300 mL). The aqueous layer was extracted with additonal EtOAc (2 x 200 mL) and the combined organic layers were washed with water (4 x 100 mL) saturated aqueous NaCl (2 x 100 mL), dried with Na₂SO₄, and concentrated. The residue was purified by flash chromatography eluting with 0-10% EtOAc / hexane to provide the title compound. (5.81 g, 64% yield) LC/MS *m*/*z* = 339 (M+H)⁺, 1.40 min (ret.time)

### 5-(Trimethylsilyl)pent-4-ynal

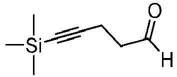

To a solution of dess-martinperiodinane (19.54 g, 46.1 mmol) in dichloromethane (DCM) (30 mL) at 0 °C was added a solution of 5-(trimethylsilyl)pent-4-yn-1-ol (6.81 mL, 37.5 mmol) in dichloromethane (DCM) (30 mL) portionwise and stirred for 1h. The reaction was then quenched with 0.1N solution of sodium thiosulfate which contained Na₂CO₃ and stirred for 10 min. The aqueous layer was washed with DCM (2 X 30 mL). The organic layers were combined, dried over sodium sulfate, and evaporated. The residue was purified by flash chromatography eluting with 0-25% EtOAc / hexane to provide the title compound. (4.78 g, 83% yield) 1H NMR (400 MHz, CHLOROFORM-d) δ 9.79 (s, 1H), 2.63-2.70 (m, 2H), 2.50-2.57 (m, 2H), 0.14 (s, 9H).

### 1-(4-Chloro-3-(((4-methoxybenzyl)oxy)methyl)phenyl)-5-(trimethylsilyl)pent-4-yn-1-ol

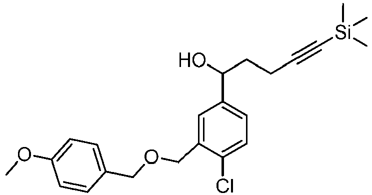

In an oven dried, nitrogen flushed flask, 5-(trimethylsilyl)pent-4-ynal (4.22 g, 27.4 mmol) was carefully dissolved in dry tetrahydrofuran (THF) (81 ml), then stirred with activated molecular sieves for 2 h. In a separate flask, 4-bromo-1-chloro-2-(((4-methoxybenzyl)oxy)methyl)benzene (4.45 g, 13.03 mmol) was dissolved in dry tetrahydrofuran (THF) (81 ml) and stirred with activated molecular sieves for 2 h. This solution was then cooled to -78 °C and stirred for 30 min. The solution of 5-(trimethylsilyl)pent-4-ynal was then added slowly to the lithiate and stirred -78 °C for 2 h. The reaction was then diluted with water (25mL) and EtOAc (75 mL). The aqueous layer was extracted with an additional portion of EtOAc (50 mL) and the combined EtOAc layers were washed with water (50 mL), saturated aqueous NaCl (50 mL), dried with Na₂SO₄ and concentrated. The residue was purified by reverse phase preparative HPLC under neutral conditions to provide the title compound. (1.00 g, 18% yield) ¹H NMR (400 MHz, CHLOROFORM-d) δ 7.49 (s, 1H), 7.28-7.35 (m, 3H), 7.20-7.25 (m, 1H), 6.90 (d, *J*=8.03 Hz, 2H), 4.86 (dd, *J*=5.02, 7.53 Hz, 1H), 4.60 (d, *J*=15.31 Hz, 4H), 3.88 (br. s., 1H), 3.81 (s, 3H), 2.23-2.43 (m, 2H), 1.83-2.02 (m, 2H), 0.13-0.19 (m, 9H).

### (5-Bromo-5-(4-chloro-3-(((4-methoxybenzyl)oxy)methyl)phenyl)pent-1-yn-1-yl)trimethylsilane

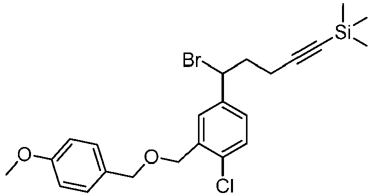

To a solution of 1-(4-chloro-3-(((4-methoxybenzyl)oxy)methyl)phenyl)-5-(trimethylsilyl)pent-4-yn-1-ol (1 g, 2.398 mmol) in dichloromethane (DCM) (48.0 ml) under argon, was added triphenylphosphine (polymer bound) (3.18 g, 7.19 mmol) followed by perbromomethane (0.954 g, 2.88 mmol) and allowed to stir at ambient temperature for **1**h. The reaction was then filtered and concentrated. The residue was purified by flash chromatography eluting with 0-10% EtOAc / hexane to provide the title compound. (0.830 g, 72% yield) ¹H NMR (400 MHz, CHLOROFORM-d) δ 7.57 (s, 1H), 7.35 (d, *J*=8.28 Hz, 3H), 7.29 (br. s., 1H), 6.93 (d, *J*=8.03 Hz, 2H), 5.08-5.14 (m, 1H), 4.62 (d, *J*=10.79 Hz, 4H), 3.85 (s, 3H), 2.22-2.51 (m, 4H), 0.19 (s, 9H)

### Benzyl 3-(4-chloro-3-(((4-methoxybenzyl)oxy)methyl)phenyl)-2,2-dimethyl-7-(trimethylsilyl)hept-6-ynoate

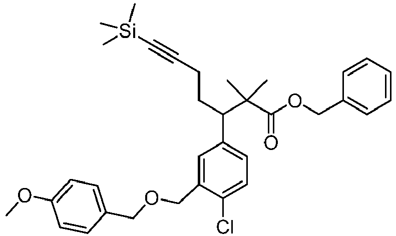

A-70 °C solution of diisopropylamine (2.424 ml, 17.29 mmol) in tetrahydrofuran (THF) (25.8 ml) under argon, was treated with 1.6M n-butyllithium in hexanes (6.49 ml, 10.38 mmol) and stirred at -70 °C for 15 min, warmed to 0 °C for 15 min, and then re-cooled to - 70 °C. Benzyl isobutyrate (1.967 ml, 11.07 mmol) in tetrahydrofuran (THF) (25.8 ml) was added dropwise to the solution and stirred at -70 °C for 45 min, warmed to -50 °C for 15 min, and recooled to -70 °C. (5-Bromo-5-(4-chloro-3-(((4-methoxybenzyl)oxy)methyl)phenyl)pent-1-yn-1-yl)trimethylsilane (0.830 g, 1.729 mmol) in tetrahydrofuran (THF) (25.8 ml) was then added dropwise to the enolate followed by dry 1,3-dimethyltetrahydropyrimidin-2(1H)-one (4.18 ml, 34.6 mmol) and stirred 1h at -70 °C, warmed to -45 °C and stirred for 1h. Afterwards, the reaction was diluted with water and EtOAc (30ml). The layers were separated and the aqueous layer was extracted with EtOAc. The combined EtOAc layers were washed with water, saturated aqueous NaCl, dried with Na₂SO₄ and concentrated. The residue was purified by flash chromatography eluting with 0-10% EtOAc / hexane to provide the title compound. (0.600 g, 60% yield). ¹H NMR (400 MHz, CHLOROFORM-d) δ 7.21-7.44 (m, 9H), 7.00 (d, *J*=8.28 Hz, 1H), 6.90 (d, *J*=8.03 Hz, 2H), 5.02-5.13 (m, 2H), 4.52-4.61 (m, 4H), 3.83 (s, 3H), 3.11 (d, *J*=12.05 Hz, 1H), 1.77-2.08 (m, 4H), 1.21 (s, 3H), 1.11 (s, 3H), 0.15 (s, 9H).

### Benzyl 3-(4-chloro-3-(((4-methoxybenzyl)oxy)methyl)phenyl)-2,2-dimethylhept-6-ynoate

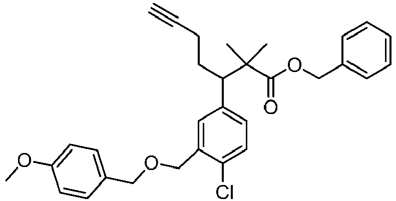

Benzyl 3-(4-chloro-3-(((4-methoxybenzyl)oxy)methyl)phenyl)-2,2-dimethyl-7-(trimethylsilyl)hept-6-ynoate (600 mg, 1.039 mmol) was dissolved in methanol (5.547 ml) and was combined with potassium carbonate (718 mg, 5.20 mmol) and stirred for 2h. Upon completion, the methanol was condensed, and the residual oil was diluted with water, and extracted with DCM (3X). The organic layers were dried with MgSO₄, and removed in vacuo to afford the title compound (400 mg, 0.792 mmol, 76 % yield). LC/MS *m*/*z* 527(M+Na)+, 1.56 min (ret. time)

### Benzyl 3-(4-chloro-3-(((4-methoxybenzyl)oxy)methyl)phenyl)-5-(1-ethyl-1H-1,2,3-triazol-4-yl)-2,2-dimethylpentanoate

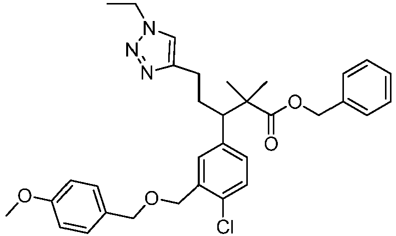

Sodium azide (129 mg, 1.980 mmol), iodoethane (146 µl, 1.822 mmol), copper(I) iodide (22.63 mg, 0.119 mmol), and Hunig's base (27.7 µl, 0.158 mmol) were added to a solution of benzyl 3-(4-chloro-3-(((4-methoxybenzyl)oxy)methyl)phenyl)-2,2-dimethylhept-6-ynoate (400 mg, 0.792 mmol) in tert-butanol (1951 µl)) and water (1951 µl) and heated on the microwave at 70 °C for 1 h.. The reaction was cooled and diluted with EtOAc (150 mL) and water (50 mL). The aqueous layer was extracted again with EtOAc (75 mL) and the combined EtOAc layers were washed with water (50 mL), saturated aqueous NaCl (25 mL), dried over Na₂SO₄ and concentrated. The residue was purified by flash chromatography eluting with 0-70% EtOAc / hexane to provide the title compound. (0.400 g, 75% yield). LC/MS *m*/*z* = 576 (M+H)+, 1.48 min (ret. time).

### Benzyl 3-(4-chloro-3-(hydroxymethyl)phenyl)-5-(1-ethyl-1H-1,2,3-triazol-4-yl)-2,2-dimethylpentanoate

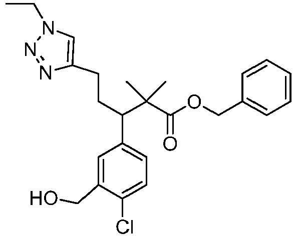

Benzyl 3-(4-chloro-3-(((4-methoxybenzyl)oxy)methyl)phenyl)-5-(1-ethyl-1H-1,2,3-triazol-4-yl)-2,2-dimethylpentanoate (810 mg, 1.406 mmol) in acetonitrile (6522 µl) was combined with ceric ammonium nitrate (2312 mg, 4.22 mmol) and water (725 µl) and stirred for 2 h. The reaction was diluted with EtOAc (100 mL) and water (50 mL), and the phases were separated. The aqueous layer. was extracted again with EtOAc (50 mL) and the combined EtOAc layers were washed with water (50 mL), saturated aqueous NaCl, dried with Na₂SO₄, and concentrated. The residue was purified by flash chromatography eluting with 0-100% EtOAc / hexane to provide the title compound. (0.510 g, 74% yield). LC/MS *m*/*z* = 456 (M+H)+, 1.10 min (ret. time).

### Benzyl 3-(4-chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoate

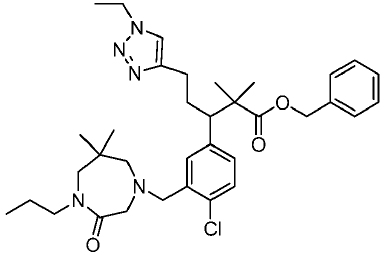

To a solution of benzyl 3-(4-chloro-3-(hydroxymethyl)phenyl)-5-(1-ethyl-1H-1,2,3-triazol-4-yl)-2,2-dimethylpentanoate (60 mg, 0.132 mmol) in dichloromethane (DCM) (6 mL), was added thionyl chloride (0.019 mL, 0.263 mmol) and stirred at ambient temperature for 30 min. The solvent was removed and the residue was dissolved in acetonitrile (10.00 mL) and 6,6-dimethyl-1-propyl-1,4-diazepan-2-one, hydrochloride (58.1 mg, 0.263 mmol) and DIEA (0.138 mL, 0.790 mmol) were added to the solution. The reaction was heated on a microwave to 120 °C for 2.5 h. The reaction mixture was condensed and the residue was purified by reverse phase preparative HPLC under neutral conditions to provide the title compound. (0.080 g, 0.129 mmol, 98% yield). LC/MS *m*/*z* = 622 (M+H)+, 1.27 min (ret. time).

### 3-(4-Chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoic acid

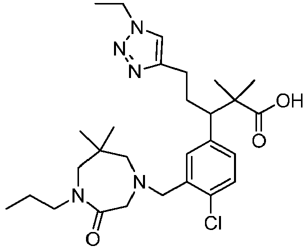

Benzyl 3-(4-chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoate (75 mg, 0.121 mmol) was dissolved in tetrahydrofuran (THF) (2.000 mL), water (1.000 mL) and methanol (2 mL) in a microwave reaction vial. Lithium hydroxide (14.43 mg, 0.603 mmol) was added to the reaction mixture and stirred on the microwave at 100 °C for 1 h. The reaction was acidified with a solution of 10% formic acid and concentrated. The residue was purified by reverse phase preparative HPLC under formic acid conditions to provide the title compound. (0.046 g, 71% yield). LC/MS *m*/*z* = 532 (M+H)+, 1.03 min (ret. time).

### Example 16 3-(4-Chloro-3-((4,6,6-trimethyl-3-oxo-1 ,4-diazepan-1-yl)methyl)phenyl)-5-(1-ethyl-1H-1,2,3-triazol-4-yl)-2,2-dimethylpentanoic acid

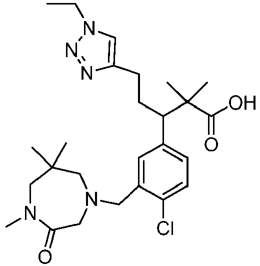

### Benzyl 3-(4-chloro-3-((4,6,6-trimethyl-3-oxo-1,4-diazepan-1-yl)methyl)phenyl)-5-(1-ethyl-1H-1,2,3-triazol-4-yl)-2,2-dimethylpentanoate

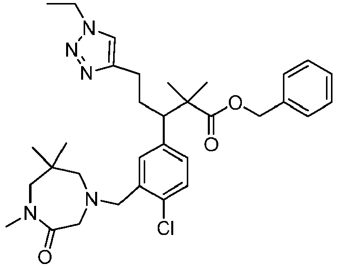

To a solution of benzyl 3-(4-chloro-3-(hydroxymethyl)phenyl)-5-(1-ethyl-1H-1,2,3-triazol-4-yl)-2,2-dimethylpentanoate (60 mg, 0.132 mmol) in dichloromethane (DCM) (6 mL), was added thionyl chloride (0.019 mL, 0.263 mmol) and stirred at ambient temperature for 30 min. The solvent was removed and the residue was dissolved in acetonitrile (6.00 mL). DIEA (0.138 mL, 0.790 mmol) and 1,6,6-trimethyl-1,4-diazepan-2-one, hydrochloride (50.7 mg, 0.263 mmol) were added and the reaction was heated via microwave to 120 °C for 2.5 h. The reaction was concentrated and the residue was purified by reverse phase preparative HPLC under neutral conditions to provide the title compound (0.054 g, 69% yield).. LC/MS *m*/*z* = 594 (M+H)+, 1.13 min (ret. time).

### 3-(4-Chloro-3-((4,6,6-trimethyl-3-oxo-1,4-diazepan-1-yl)methyl)phenyl)-5-(1-ethyl-1H-1,2,3-triazol-4-yl)-2,2-dimethylpentanoic acid

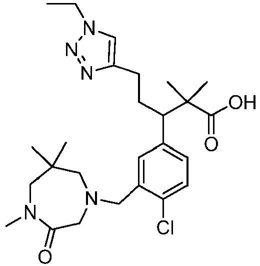

Benzyl 3-(4-chloro-3-((4,6,6-trimethyl-3-oxo-1,4-diazepan-1-yl)methyl)phenyl)-5-(1-ethyl-1H-1,2,3-triazol-4-yl)-2,2-dimethylpentanoate (54 mg, 0.091 mmol) was dissolved in tetrahydrofuran (THF) (2.000 mL), water (1.000 mL) and methanol (2 mL) in a microwave reaction vial. Lithium hydroxide (10.88 mg, 0.454 mmol) was added to the reaction mixture and stirred at 100 °C for 2 h on the microwave. The reaction was quenched with 10% formic acid until acidic and the solvents were concentrated. The residue was purified by reverse phase preparative HPLC under formic acid conditions to provide the title compound (0.025 g, 54% yield). LC/MS *m*/*z* = 504 (M+H)+, 0.90 min (ret. time).

## Claims

1. A compound of Formula (I): wherein: 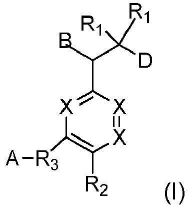
B is benzotriazolyl, phenyl, triazolopyridinyl, -O(CH₂)-triazolyl or -(CH₂)₂-triazolyl, wherein each of the benzotriazolyl, phenyl, triazolopyridinyl, -O(CH₂)-triazolyl or -(CH₂)₂-triazolyl is unsubstituted or substituted by 1, 2, or 3 substituents independently selected from -C₁₋₃alkyl, -O-C₁₋₃alkyl, CN, -(CH₂)₂-O-(CH₂)₂-OR₄ and halo;
D is -C(O)OH, -C(O)NR₆R₇, -C(O)NHSO₂CH₃, -SO₂NHC(O)CH₃, 5-(trifluoromethyl)-4H-1,2,4-triazol-2-yl, -NR₆-C(O)-R₇, -NR₆-C(O)-NR₆R₇; -NR₆-C(O)-O-R₇ or tetrazolyl;
R₁ is independently hydrogen, -OH, -C₁₋₃alkyl, - C₁₋₃alkylOR', F, -C₃₋₆spirocycloalkyl, oxetane, or the two R₁ groups together with the carbon to which they are attached form a cyclopropyl group;
R' is hydrogen or -C₁₋₃alkyl;
R₂ is hydrogen, -C₁₋₄alkyl, -CF₃, or halo;
R₃ is -(CH₂)ₘ;
R₄ is hydrogen or -C₁₋₃alkyl;
or, when R₂ is -C₁₋₄alkyl, R₃ is -(CH₂)ₘ-, and m is 2 or 3, R₂ and R₃ together form a cycloalkyl ring fused to the phenyl ring to which they are attached;
A is 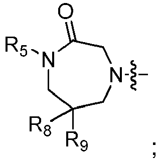
R₅ is hydrogen, -C₁₋₅alkyl or -(CH₂)ₙ-C₃₋₅cycloalkyl;
R₆ is hydrogen or -C₁₋₄alkyl;
R₇ is hydrogen, -C₁₋₅alkyl, -C₃₋₇cycloalkyl, -C₄₋₈heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-SO₂C₁₋₃alkyl, -C₁₋₃alkyl-C₄₋₈heterocycloalkyl, -C₁₋₃alkyl-C(O)NR₄R₆, or heteroaryl, wherein each of -C₁₋₅ alkyl, -C₃₋₇ cycloalkyl, -C₄₋₇ heterocycloalkyl, -C₁₋₅alkoxy, -C₁₋₃alkyl-O-C₁₋₃alkyl, -C₁₋₃alkyl-NH-C₁₋₃alkyl, -C₁₋₃alkyl-C(O)NR₄R₆, or heteroaryl is unsubstituted or substituted by one or two substituents selected from -OH, -CO₂H,
-C(O)NR₄R₆,-C(O)OR₄, -N-C(O)-C₁₋₃alkyl, F, -CN, -CH-F₂, -CF₃, -(CH₂)ₙ-O-(CH₂)ₘ-CH₃, and-C₃₋₇cycloalkyl, a 5-6-membered heteroaryl ring containing 1, 2 or 3 heteroatoms selected from O, N and S;
or R₆ and R₇ together with the nitrogen atom to which they are attached form a 5-8-membered heterocyclic ring, an 8-11-membered bicyclic heterocyclic ring or a 9- 10-membered bridged bicyclic heterocyclic ring, wherein each 5-8-membered ring, 8-11-membered bicyclic ring, or 9-10-membered bridged bicyclic ring optionally includes one - C(O) or one -S(O)₂, and wherein each 5-8-membered ring, 8-11-membered bicyclic ring, or 9-10-membered bridged bicyclic ring optionally contains one, two or three oxygen ring atoms, one, two or three sulfur ring atoms or one, two or three nitrogen ring atoms, and wherein each 5-8-membered ring, 8-11-membered bicyclic ring, or 9-10-membered bridged bicyclic ring is unsubstituted or substituted by one, two or three substituents independently selected from -C₁₋₅alkyl, -C₃₋₇cycloalkyl, -C₄₋₇heterocycloalkyl, -(CH₂)phenyl, halogen,-NR₃R₄, -CHF₂, -CF₃, and -(CH₂)ₙ-O-(CH₂)ₘ-CH₃;
R₈ is hydrogen or -C₁₋₄alkyl;
Rg is hydrogen or -C₁₋₄alkyl;
m is 1, 2 or 3;
n is 0, 1, 2 or 3; and
X is independently CH or N;
or a pharmaceutically acceptable salt thereof.

2. A compound of claim 1 wherein:
B is benzotriazolyl which is unsubstituted or substituted by 1, 2, or 3 substituents independently selected from -C₁₋₃alkyl;
D is -C(O)OH;
R₁ is independently hydrogen or -C₁₋₃alkyl;
R₂ is methyl or chloro;
R₃ is (-CH₂)ₘ;
A is 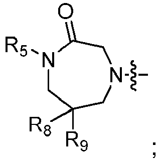
R₅ is -C₁₋₅alkyl or -(CH₂)ₙ-C₃₋₅cycloalkyl;
R₈ is hydrogen or methyl;
Rg is hydrogen or methyl;
m is 1;
n is 0 or 1; and
X is CH;
or a pharmaceutically acceptable salt thereof.

3. The compound of claim 1 selected from :
(S)-3-(1,4-dimethyl-1H-benzo[d][1,2,3]triazol-5-yl)-2,2-dimethyl-3-(4-methyl-3-((4-methyl-3-oxo-1,4-(S)-3-(3-((4-cyclopropyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropanoic acid;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((6,6-dimethyl-3-oxo-4-propyl-1,4-diazepan-1-yl)methyl)-4-methylphenyl)-2,2-dimethylpropanoic acid;
(S)-3-(1,4-Dimethyl-1H-benzo[d][1,2,3]triazol-5-yl)-2,2-dimethyl-3-(4-methyl-3-((4-methyl-3-oxo-1,4-diazepan-1-yl)methyl)phenyl)propanoic acid;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-1H-benzo[d][1,2,3]triazol-5-yl)-3-(3-((4-ethyl-6,6-dimethyl-3-oxo-1,4-diazepan-1-yl)methyl)-4-methylphenyl)-2,2-dimethylpropanoic acid;
3-(1,4-Dimethyl-1H-benzo[d][1,2,3]triazol-5-yl)-3-(3-((6-ethyl-4-methyl-3-oxo-1,4-diazepan-1-yl)methyl)-4-methylphenyl)-2,2-dimethylpropanoic acid;
(S)-3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid, 0.3 Formic acid salt;
3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propanoic acid;
3-(3-((4-Cyclobutyl-6,6-dimethyl-3-oxo-1,4-diazepan-1-yl)methyl)-4-methylphenyl)-3-(1 ,4-dimethyl-1 H-benzo[d][1,2,3]triazol-5-yl)-2,2-dimethylpropanoic acid;
3-(3-((4-(Cyclopropylmethyl)-6,6-dimethyl-3-oxo-1,4-diazepan-1-yl)methyl)-4-methylphenyl)-3-(1,4-dimethyl-1 H-benzo[d][1 ,2,3]triazol-5-yl)-2,2-dimethylpropanoic acid;
rel-3S-(1,4-Dimethyl-1H-benzo[d][1,2,3]triazol-5-yl)-2,2-dimethyl-3-(rel-(R)-3-(4,6,6-trimethyl-3-oxo-1,4-diazepan-1-yl)-2,3-dihydro-1H-inden-5-yl)propanoic acid;
rel-3S-(1,4-Dimethyl-1H-benzo[d][1,2,3]triazol-5-yl)-2,2-dimethyl-3-(rel-(S)-3-(4,6,6-trimethyl-3-oxo-1,4-diazepan-1-yl)-2,3-dihydro-1H-inden-5-yl)propanoic acid;
rel-(3R)-3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid;
3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propanoic acid;
3-((1-Ethyl-1H-1,2,3-triazol-4-yl)methoxy)-2,2-dimethyl-3-(4-methyl-3-((4,6,6-trimethyl-3-oxo-1,4-diazepan-1-yl)methyl)phenyl)propanoic acid;
3-(4-Chloro-3-((6,6-dimethyl-3-oxo-4-propyl-1,4-diazepan-1-yl)methyl)phenyl)-5-(1-ethyl-1H-1,2,3-triazol-4-yl)-2,2-dimethylpentanoic acid; and
3-(4-Chloro-3-((4,6,6-trimethyl-3-oxo-1,4-diazepan-1-yl)methyl)phenyl)-5-(1-ethyl-1H-1,2,3-triazol-4-yl)-2,2-dimethylpentanoic acid;
or a pharmaceutically acceptable salt thereof.

4. A pharmaceutical composition comprising a compound or a pharmaceutically acceptable salt thereof according to any one of claims 1-3 and a pharmaceutically acceptable excipient.

5. A compound or a pharmaceutically acceptable salt thereof according to any one of claims 1-3 for use in therapy.

6. A compound or a pharmaceutically acceptable salt thereof according to any one of claims 1-3 for use in treating respiratory and non-respiratory disorders, including COPD, asthma, ALI, ARDS, fibrosis, chronic asthma and acute asthma, lung disease secondary to environmental exposures, acute lung infection, chronic lung infection, α1 antitrypsin disease, cystic fibrosis, autoimmune diseases, diabetic nephropathy, chronic kidney disease, sepsis-induced acute kidney injury, acute kidney injury (AKI), kidney disease or malfunction seen during kidney transplantation, Pulmonary Arterial Hypertension, atherosclerosis, hypertension, heart failure, acute coronary syndrome, myocardial infarction, myocardial repair, cardiac remodelling, cardiac arrhythmias, Parkinson's disease (PD), Alzheimer's disease (AD), Friedreich's Ataxia (FA), amyotrophic lateral sclerosis (ALS), multiple sclerosis (MS), inflammatory bowel disease, colon cancer, neovascular (dry) AMD and neovascular (wet) AMD, eye injury, Fuchs Endothelial Corneal Dystrophy (FECD), uveitis or other inflammatory eye condtions, Non-alcoholic Steatohepatitis (NASH), toxin-induced liver disease (e.g., acetaminophen-induced hepatic disease), viral hepatitis, cirrhosis, psoriasis, dermatitis/topical effects of radiation, immunosuppression due to radiation exposure, Preeclampsia, and high altitude sickness.

7. A compound for use according to claim 6 wherein the disease is COPD.

8. A compound for use according to claim 6 wherein the disease is heart failure.

9. A compound for use according to claim 6-8 wherein the compound is administered orally.

10. A compound for use according to claims 6-8 wherein the compound is administered intravenously.

11. A compound for use according to claims 6-8 wherein the compound is administered by inhalation.

## Patentansprüche

1. Verbindung der Formel (I): 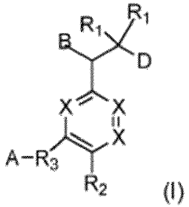 worin:
B für Benzotriazolyl, Phenyl, Triazolopyridinyl, -O(CH₂)-Triazolyl oder -(CH₂)₂-Triazolyl steht, worin jedes aus dem Benzotriazolyl, Phenyl, Triazolopyridinyl, -O(CH₂)-Triazolyl oder -(CH₂)₂-Triazolyl unsubstituiert oder mit 1, 2 oder 3 Substituenten substituiert ist, unabhängig ausgewählt aus-C₁₋₃-Alkyl, -O-C₁₋₃-Alkyl, CN, -(CH₂)₂-O-(CH₂)₂-OR₄ und Halogen;
D für -C(O)OH, -C(O)NR₆R₇, -C(O)NHSO₂CH₃, -SO₂NHC(O)CH₃, 5-(Trifluormethyl)-4H-1,2,4-triazol-2-yl, -NR₆-C(O)-R₇, -NR₆-C(O)-NR₆R₇; -NR₆-C(O)-O-R₇ oder Tetrazolyl steht;
R₁ unabhängig für Wasserstoff, -OH, -C₁₋₃-Alkyl, -C₁₋₃-AlkylOR', F, -C₃₋₆-Spirocycloalkyl, Oxetan steht oder die zwei R₁-Gruppen zusammen mit dem Kohlenstoff, an den sie gebunden sind, eine Cyclopropylgruppe bilden;
R' für Wasserstoff oder -C₁₋₃-Alkyl steht;
R₂ für Wasserstoff, -C₁₋₄-Alkyl, -CF₃ oder Halogen steht;
R₃ für -(CH₂)ₘ steht;
R₄ für Wasserstoff oder -C₁₋₃-Alkyl steht;
oder wenn R₂ für -C₁₋₄-Alkyl steht, R₃ für -(CH₂)ₘ- steht und m 2 oder 3 beträgt, R₂ und R₃ zusammen einen Cycloalkylring bilden, der an den Phenylring, an den sie gebunden sind, kondensiert ist;
A für 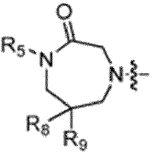 steht;
R₅ für Wasserstoff, -C₁₋₅-Alkyl oder -(CH₂)ₙ-C₃₋₅-Cycloalkyl steht;
R₆ für Wasserstoff oder -C₁₋₄-Alkyl steht;
R₇ für Wasserstoff, -C₁₋₅-Alkyl, -C₃₋₇-Cycloalkyl, -C₄₋₈-Heterocycloalkyl, -C₁₋₅-Alkoxy, -C₁₋₃-Alkyl-O-C₁₋₃-alkyl, -C₁₋₃-Alkyl-NH-C₁₋₃-alkyl, -C₁₋₃-Alkyl-SO₂C₁₋₃-alkyl, -C₁₋₃-Alkyl-C₄₋₈-heterocycloalkyl, -C₁₋₃-Alkyl-C(O)NR₄R₆ oder Heteroaryl steht, worin jedes aus -C₁₋₅-Alkyl, -C₃₋₇-Cycloalkyl, -C₄₋₇-Heterocycloalkyl, -C₁₋₅-Alkoxy, -C₁₋₃-Alkyl-O-C₁₋₃-alkyl, -C₁₋₃-Alkyl-NH-C₁₋₃-alkyl, -C₁₋₃-Alkyl-C(O)NR₄R₆ oder Heteroaryl unsubstituiert oder mit einem oder zwei Substituenten substituiert ist, ausgewählt aus -OH, -CO₂H,-C(O)NR₄R₆,-C(O)OR₄, -N-C(O)-C₁₋₃-Alkyl, F, -CN, -CH-F₂, -CF₃, -(CH₂)ₙ-O-(CH₂)ₘ-CH₃ und -C₃₋₇-Cycloalkyl, einem 5-6-gliedrigen Heteroarylring, der 1, 2 oder 3 Heteroatome enthält, ausgewählt aus O, N und S;
oder R₆ und R₇ zusammen mit dem Stickstoffatom, an das sie gebunden sind, einen 5-8-gliedrigen heterocyclischen Ring, einen 8-11-gliedrigen bicyclischen heterocyclischen Ring oder einen 9-10-gliedrigen verbrückten bicyclischen heterocyclischen Ring bilden, worin jeder 5-8-gliedrige Ring, 8-11-gliedrige bicyclische Ring oder 9-10-gliedrige verbrückte bicyclische Ring gegebenenfalls ein -C(O) oder ein -S(O)₂ aufweist, und worin jeder 5-8-gliedrige Ring, 8-11-gliedrige bicyclische Ring oder 9-10-gliedrige verbrückte bicyclische Ring gegebenenfalls ein, zwei oder drei Sauerstoffringatome, ein, zwei oder drei Schwefelringatome oder ein, zwei oder drei Stickstoffringatome enthält, und worin jeder 5-8-gliedrige Ring, 8-11-gliedrige bicyclische Ring oder 9-10-gliedrige verbrückte bicyclische Ring unsubstituiert oder mit ein, zwei oder drei Substituenten substituiert ist, unabhängig ausgewählt aus -C₁₋₅-Alkyl, -C₃₋₇-Cycloalkyl, -C₄₋₇-Heterocycloalkyl, -(CH₂)-Phenyl, Halogen,-NR₃R₄, -CHF₂, -CF₃ und -(CH₂)ₙ-O-(CH₂)ₙ-CH₃;
R₈ für Wasserstoff oder -C₁₋₄-Alkyl steht;
Rg für Wasserstoff oder -C₁₋₄-Alkyl steht;
m 1, 2 oder 3 beträgt;
n 0, 1, 2 oder 3 beträgt; und
X unabhängig für CH oder N steht;
oder ein pharmazeutisch annehmbares Salz davon.

2. Verbindung gemäß Anspruch 1, worin:
B für Benzotriazolyl steht, das unsubstituiert oder mit 1, 2 oder 3 Substituenten substituiert ist, unabhängig ausgewählt aus -C₁₋₃-Alkyl;
D für -C(O)OH steht;
R₁ unabhängig für Wasserstoff oder -C₁₋₃-Alkyl steht;
R₂ für Methyl oder Chlor steht;
R₃ für (-CH₂)ₘ steht;
A für 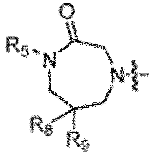 steht;
R₅ für -C₁₋₅-Alkyl oder -(CH₂)ₙ-C₃₋₅-Cycloalkyl steht;
R₈ für Wasserstoff oder Methyl steht;
Rg für Wasserstoff oder Methyl steht;
m 1 beträgt;
n 0 oder 1 beträgt; und
X für CH steht;
oder ein pharmazeutisch annehmbares Salz davon.

3. Verbindung gemäß Anspruch 1, die ausgewählt ist aus:
(S)-3-(1,4-Dimethyl-1H-benzo[d][1,2,3]triazol-5-yl)-2,2-dimethyl-3-(4-methyl-3-((4-methyl-3-oxo-1,4-(S)-3-(3-((4-cyclopropyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropansäure;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-lH-benzo[d][1,2,3]triazol-5-yl)-3-(3-((6,6-dimethyl-3-oxo-4-propyl-1,4-diazepan-1-yl)methyl)-4-methylphenyl)-2,2-dimethylpropansäure;
(S)-3-(1,4-Dimethyl-1H-benzo[d][1,2,3]triazol-5-yl)-2,2-dimethyl-3-(4-methyl-3-((4-methyl-3-oxo-1,4-diazepan-1-yl)methyl)phenyl)propansäure;
(S)-3-(1,4-Dimethyl-1H-benzo[d][1,2,3]triazol-5-yl)-2,2-dimethyl-3-(4-methyl-3-((4-methyl-3-oxo-1,4-(S)-3-(1,4-dimethyl-lH-benzo[d][1,2,3]triazol-5-yl)-3-(3-((4-ethyl-6,6-dimethyl-3-oxo-1,4-diazepan-1-yl)methyl)-4-methylphenyl)-2,2-dimethylpropansäure;
3-(1,4-Dimethyl-1H-benzo[d][1,2,3]triazol-5-yl)-3-(3-((6-ethyl-4-methyl-3-oxo-1,4-diazepan-1-yl)methyl)-4-methylphenyl)-2,2-dimethylpropansäure;
(S)-3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propansäure, 0,3 Ameisensäuresalz;
3-(1,4-Dimethyl-1H-benzo[d][1,2,3]triazol-5-yl)-2,2-dimethyl-3-(4-methyl-3-((4,6,6-trimethyl-3-oxo-1,4-diazepan-1-yl)methyl)phenyl)propansäure;
3-(3-((4-Cyclobutyl-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropansäure;
3-(3-((4-(Cyclopropylmethyl)-6,6-dimethyl-3-oxo-1,4-diazepan-1-yl)methyl)-4-methylphenyl)-3-(1,4-dimethyl-1H-benzo[d][1,2,3]triazol-5-yl)-2,2-dimethylpropansäure;
rel-3S-(1,4-Dimethyl-1H-benzo[d][1,2,3]triazol-5-yl)-2,2-dimethyl-3-(rel-(R)-3-(4,6,6-trimethyl-3-oxo-1,4-diazepan-1-yl)-2,3-dihydro-1H-inden-5-yl)propansäure;
rel-3S-(1,4-Dimethyl-1H-benzo[d][1,2,3]triazol-5-yl)-2,2-dimethyl-3-(rel-(S)-3-(4,6,6-trimethyl-3-oxo-1,4-diazepan-1-yl)-2,3-dihydro-1H-inden-5-yl)propansäure;
rel-(3R)-3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propansäure;
3-(1,4-Dimethyl-1H-benzo[d][1,2,3]triazol-5-yl)-2,2-dimethyl-3-(3-(4,6,6-trimethyl-3-oxo-1,4-diazepan-1-yl)-2,3-dihydro-1H-inden-5-yl)propansäure;
3-((1-Ethyl-1H-1,2,3-triazol-4-yl)methoxy)-2,2-dimethyl-3-(4-methyl-3-((4,6,6-trimethyl-3-oxo-1,4-diazepan-1-yl)methyl)phenyl)propansäure;
3-(4-Chlor-3-((6,6-dimethyl-3-oxo-4-propyl-1,4-diazepan-1-yl)methyl)phenyl)-5-(1-ethyl-1H-1,2,3-triazol-4-yl)-2,2-dimethylpentansäure; und
3-(4-Chlor-3-((4,6,6-trimethyl-3-oxo-1,4-diazepan-1-yl)methyl)phenyl)-5-(1-ethyl-1H-1,2,3-triazol-4-yl)-2,2-dimethylpentansäure;
oder ein pharmazeutisch annehmbares Salz davon.

4. Pharmazeutische Zusammensetzung, umfassend eine Verbindung oder ein pharmazeutisch annehmbares Salz davon gemäß einem der Ansprüche 1-3 und einen pharmazeutisch annehmbaren Hilfsstoff.

5. Verbindung oder ein pharmazeutisch annehmbares Salz davon gemäß einem der Ansprüche 1-3 zur Verwendung bei einer Heilbehandlung.

6. Verbindung oder ein pharmazeutisch annehmbares Salz davon gemäß einem der Ansprüche 1-3 zur Verwendung bei der Behandlung von Atemwegserkrankungen und Nicht-Atemwegserkrankungen, einschließlich COPD, Asthma, ALI, ARDS, Fibrose, chronischem Asthma und akutem Asthma, Lungenerkrankung infolge von Umwelteinflüssen, akuter Lungeninfektion, chronischer Lungeninfektion, α1-Antitrypsin-Krankheit, Mukoviszidose, Autoimmunerkrankungen, diabetischer Nephropathie, chronischer Nierenerkrankung, Sepsisinduzierter akuter Nierenverletzung, akuter Nierenverletzung (AKI - "acute kidney injury"), Nierenerkrankung oder Fehlfunktion, die während einer Nierentransplantation beobachtet wird, pulmonaler arterieller Hypertonie, Atherosklerose, Hypertonie, Herzinsuffizienz, akutem Koronarsyndrom, Myokardinfarkt, Myokardwiederherstellung, kardialem Remodeling, Herzrhythmusstörungen, Parkinson-Krankheit (PD - "Parkinson's disease"), Alzheimer-Krankheit (AD - "Alzheimer's disease"), Friedreich-Ataxie (FA), amyotropher Lateralsklerose (ALS), Multipler Sklerose (MS), entzündlicher Darmerkrankung, Dickdarmkrebs, neovaskulärer (trockener) AMD und neovaskulärer (feuchter) AMD, Augenverletzung, Fuchs-Endothel-Hornhautdystrophie (FECD - "Fuchs Endothelial Corneal Dystrophy"), Uveitis oder anderer entzündlicher Augenerkrankungen, nicht-alkoholischer Steatohepatitis (NASH), Toxin-induzierter Lebererkrankung (z. B. Acetaminophen-induzierter Lebererkrankung), Virushepatitis, Zirrhose, Psoriasis, Dermatitis/topischer Strahleneffekte, Immunsuppression aufgrund von Strahlenexposition, Präeklampsie und Höhenkrankheit.

7. Verbindung zur Verwendung gemäß Anspruch 6, worin die Erkrankung COPD ist.

8. Verbindung zur Verwendung gemäß Anspruch 6, worin die Erkrankung Herzinsuffizienz ist.

9. Verbindung zur Verwendung gemäß Anspruch 6-8, worin die Verbindung oral verabreicht wird.

10. Verbindung zur Verwendung gemäß Anspruch 6-8, worin die Verbindung intravenös verabreicht wird.

11. Verbindung zur Verwendung gemäß Anspruch 6-8, worin die Verbindung mittels Inhalation verabreicht wird.

## Revendications

1. Composé de formule (I) : 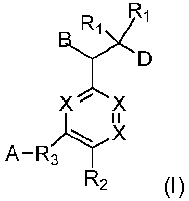 dans lequel :
B est un benzotriazolyle, un phényle, un triazolopyridinyle, un -O(CH₂)-triazolyle ou un -(CH₂)₂-triazolyle, dans lequel chacun des benzotriazolyle, phényle, triazolopyridinyle, -O(CH₂)-triazolyle ou -(CH₂)₂-triazolyle est non substitué ou substitué avec 1, 2 ou 3 substituants choisis indépendamment parmi un -alkyle en C₁₋₃, un -O-alkyle en C₁₋₃, CN, -(CH₂)₂-O-(CH₂)₂-OR₄ et un halo ;
D est -C(O)OH, -C(O)NR₆R₇, -C(O)NHSO₂CH₃, -SO₂NHC(O)CH₃, un 5-(trifluorométhyl)-4H-1,2,4-triazol-2-yle, -NR₆-C(O)-R₇, -NR₆-C(O)-NR₆R₇; -NR₆-C(O)-O-R₇ ou un tétrazolyle ;
R₁ est indépendamment un hydrogène, -OH, un -alkyle en C₁₋₃, un -alkyleOR' en C₁₋₃, F, un -spirocycloalkyle en C₃₋₆, un oxétane, ou les deux groupes R₁ ensemble avec le carbone auquel ils sont attachés forment un groupe cyclopropyle ;
R' est un hydrogène ou un -alkyle en C₁₋₃;
R₂ est un hydrogène, un -alkyle en C₁₋₄, -CF₃ ou un halo ;
R₃ est -(CH₂)ₘ ;
R₄ est un hydrogène ou un -alkyle en C₁₋₃ ;
ou, lorsque R₂ est un -alkyle en C₁₋₄, R₃ est -(CH₂)ₘ-, et m est 2 ou 3, R₂ et R₃ forment ensemble un cycle cycloalkyle fusionné au cycle phényle auquel ils sont attachés ;
A est 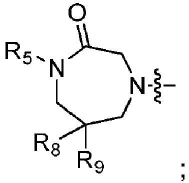
R₅ est un hydrogène, un -alkyle en C₁₋₅ ou un -(CH₂)n-cycloalkyle en C₃₋₅ ;
R₆ est un hydrogène ou un -alkyle en C₁₋₄ ;
R₇ est un hydrogène, un -alkyle en C₁₋₅, un -cycloalkyle en C₃₋₇, un -hétérocycloalkyle en C₄₋₈, un -alcoxy en C₁₋₅, un -alkyle en C₁₋₃-O-alkyle en C₁₋₃, un -alkyle en C₁₋₃-NH-alkyle en C₁₋₃, un -alkyle en C₁₋₃-SO₂alkyle en C₁₋₃, un -alkyle en C₁₋₃-hétérocycloalkyle en C₄₋₈, un -alkyle en C₁₋₃-C(O)NR₄R₆, ou un hétéroaryle, chacun parmi l'alkyle en C₁₋₅, le cycloalkyle en C₃₋₇, l'hétérocycloalkyle en C₄₋₇, l'alcoxy en C₁₋₅, l'alkyle en C₁₋₃-O-alkyle en C₁₋₃, l'alkyle en C₁₋₃-NH-alkyle en C₁₋₃, l'alkyle en C₁₋₃-C(O)NR₄R₆, ou l'hétéroaryle est non substitué ou substitué avec un ou deux substituants choisis parmi -OH, -CO₂H,
-C(O)NR₄R₆, -C(O)OR₄, un -NC(O)-alkyle en C₁₋₃, F, -CN, -CH-F₂, -CF₃, -(CH₂)ₙ-O-(CH₂)ₘ-CH₃, et un -cycloalkyle en C₃₋₇, un cycle hétéroaryle de 5 à 6 chaînons contenant 1, 2 ou 3 hétéroatomes choisis parmi O, N et S ;
ou R₆ et R₇ ensemble avec l'atome d'azote auquel ils sont attachés forment un cycle hétérocycle de 5 à 8 chaînons, un cycle hétérocycle bicyclique de 8 à 11 chaînons ou un cycle hétérocyclique bicyclique ponté de 9 à 10 chaînons, dans lequel chaque cycle de 5 à 8 chaînons, cycle bicyclique de 8 à 11 chaînons ou cycle bicyclique ponté de 9 à 10 chaînons inclut facultativement un -C(O) ou un -S(O)₂, et dans lequel chaque cycle de 5 à 8 chaînons, cycle bicyclique de 8 à 11 chaînons ou cycle bicyclique ponté de 9 à 10 chaînons contient facultativement un, deux ou trois atomes d'oxygène de cycle, un, deux ou trois atomes de soufre de cycle ou un, deux ou trois atomes d'azote de cycle, et dans lequel chaque cycle de 5 à 8 chaînons, cycle bicyclique de 8 à 11 chaînons ou cycle bicyclique ponté de 9 à 10 chaînons est non substitué ou substitué avec un, deux ou trois substituants choisis indépendamment parmi un -alkyle en C₁₋₅, un -cycloalkyle en C₃₋₇, un -hétérocycloalkyle en C₄₋₇, un -(CH₂)phényle, un halogène, -NR₃R₄, -CHF₂, -CF₃ et -(CH₂)ₙ-O-(CH₂)ₘ-CH₃ ;
R₈ est un hydrogène ou un -alkyle en C₁₋₄ ;
R₉ est un hydrogène ou un -alkyle en C₁₋₄ ;
m est 1, 2 ou 3 ;
n est 0, 1, 2 ou 3 ; et
X est indépendamment CH ou N ;
ou un sel pharmaceutiquement acceptable de celui-ci.

2. Composé selon la revendication 1, dans lequel :
B est un benzotriazolyle qui est non substitué ou substitué avec 1, 2 ou 3 substituants choisis indépendamment parmi un -alkyle en C₁₋₃ ;
D est -C(O)OH ;
R₁ est indépendamment un hydrogène ou un -alkyle en C₁₋₃ ;
R₂ est un méthyle ou un chloro ;
R₃ est (-CH₂)ₘ ;
A est 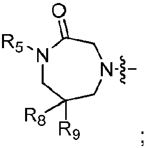 R₅ est un -alkyle en C₁₋₅ ou un -(CH₂)ₙ-cycloalkyle en C₃₋₅ ;
R₈ est un hydrogène ou un méthyle ;
R₉ est un hydrogène ou un méthyle ;
m est 1 ;
n est 0 ou 1 ; et
X est CH ;
ou un sel pharmaceutiquement acceptable de celui-ci.

3. Composé selon la revendication 1, choisi parmi :
l'acide (S)-3-(1,4-diméthyl-1H-benzo[d][1,2,3]triazol-5-yl)-2,2-diméthyl-3-(4-méthyl-3-((4-méthyl-3-oxo-1,4-(S)-3-(3-((4-cyclopropyl-6,6-diméthyl-3-oxo-1,4-diazépan-1-yl)méthyl)-4-méthylphényl)-3-(1,4-diméthyl-1H-benzo[d][1,2,3]triazol-5-yl)-2,2-diméthylpropanoïque ;
l'acide (S)-3-(1,4-diméthyl-1H-benzo[d][1,2,3]triazol-5-yl)-2,2-diméthyl-3-(4-méthyl-3-((4-méthyl-3-oxo-1,4-(S)-3-(1,4-diméthyl-1H-benzo[d][1,2,3]triazol-5-yl)-3-(3-((6,6-diméthyl-3-oxo-4-propyl-1,4-diazépan-1-yl)méthyl)-4-méthylphényl)-2,2-diméthylpropanoïque ;
l'acide (S)-3-(1,4-diméthyl-1H-benzo[d][1,2,3]triazol-5-yl)-2,2-diméthyl-3-(4-méthyl-3-((4-méthyl-3-oxo-1,4-diazépan-1-yl)méthyl)phényl)-propanoïque ;
l'acide (S)-3-(1,4-diméthyl-1H-benzo[d][1,2,3]triazol-5-yl)-2,2-diméthyl-3-(4-méthyl-3-((4-méthyl-3-oxo-1,4-(S)-3-(1,4-diméthyl-1H-benzo[d][1,2,3]triazol-5-yl)-3-(3-((4-éthyl-6,6-diméthyl-3-oxo-1,4-diazépan-1-yl)méthyl)-4-méthylphényl)-2,2-diméthylpropanoïque ;
l'acide 3-(1,4-diméthyl-1H-benzo[d][1,2,3]triazol-5-yl)-3-(3-((6-éthyl-4-méthyl-3-oxo-1,4-diazépan-1-yl)méthyl)-4-méthylphényl)-2,2-diméthyl-propanoïque ;
l'acide (S)-3-(1,4-diméthyl-1H-benzo[d][1,2,3]triazol-5-yl)-2,2-diméthyl-3-(4-méthyl-3-((4,6,6-triméthyl-3-oxo-1,4-diazépan-1-yl)méthyl)phényl) propanoïque, 0,3 sel d'acide formique ;
l'acide 3-(1,4-diméthyl-1H-benzo[d][1,2,3]triazol-5-yl)-2,2-diméthyl-3-(4-méthyl-3-((4,6,6-triméthyl-3-oxo-1,4-diazépan-1-yl)méthyl)phényl)propanoïque ;
l'acide 3-(3-((4-cyclobutyl-6,6-diméthyl-3-oxo-1,4-diazépan-1-yl)-méthyl)-4-méthylphényl)-3-(1,4-diméthyl-1H-benzo[d][1,2,3]triazol-5-yl)-2,2-diméthylpropanoïque ;
l'acide 3-(3-((4-(cyclopropylméthyl)-6,6-diméthyl-3-oxo-1,4-diazépan-1-yl)méthyl)-4-méthylphényl)-3-(1,4-diméthyl-1H-benzo[d][1,2,3]triazol-5-yl)-2,2-diméthylpropanoïque ;
l'acide rel-3S-(1,4-diméthyl-1H-benzo[d][1,2,3]triazol-5-yl)-2,2-diméthyl-3-(rel-(R)-3-(4,6,6-triméthyl-3-oxo-1,4-diazépan-1-yl)-2,3-dihydro-1H-indén-5-yl)propanoïque ;
l'acide rel-3S-(1,4-diméthyl-1H-benzo[d][1,2,3]triazol-5-yl)-2,2-diméthyl-3-(rel-(S)-3-(4,6,6-triméthyl-3-oxo-1,4-diazépan-1-yl)-2,3-dihydro-1H-indén-5-yl)propanoïque ;
l'acide rel-(3R)-3-(1,4-diméthyl-1H-benzo[d][1,2,3]triazol-5-yl)-2,2-diméthyl-3-(3-(4,6,6-triméthyl-3-oxo-1,4-diazépan-1-yl)-2,3-dihydro-1H-indén-5-yl)propanoïque ;
l'acide 3-(1,4-diméthyl-1H-benzo[d][1,2,3]triazol-5-yl)-2,2-diméthyl-3-(3-(4,6,6-triméthyl-3-oxo-1,4-diazépan-1-yl)-2,3-dihydro-1H-indén-5-yl)-propanoïque ;
l'acide 3-((1-éthyl-1H-1,2,3-triazol-4-yl)méthoxy)-2,2-diméthyl-3-(4-méthyl-3-((4,6,6-triméthyl-3-oxo-1,4-diazépan-1-yl)méthyl)phényl)propanoïque ;
l'acide 3-(4-chloro-3-((6,6-diméthyl-3-oxo-4-propyl-1,4-diazépan-1-yl)méthyl)phényl)-5-(1-éthyl-1H-1,2,3-triazol-4-yl)-2,2-diméthylpentanoïque ; et
l'acide 3-(4-chloro-3-((4,6,6-triméthyl-3-oxo-1,4-diazépan-1-yl)-méthyl)phényl)-5-(1-éthyl-1H-1,2,3-triazol-4-yl)-2,2-diméthylpentanoïque ;
ou un sel pharmaceutiquement acceptable de ceux-ci.

4. Composition pharmaceutique comprenant un composé ou un sel pharmaceutiquement acceptable de celui-ci selon l'une quelconque des revendications 1 à 3 et un excipient pharmaceutiquement acceptable.

5. Composé ou sel pharmaceutiquement acceptable de celui-ci selon l'une quelconque des revendications 1 à 3 pour une utilisation en thérapie.

6. Composé ou sel pharmaceutiquement acceptable de celui-ci selon l'une quelconque des revendications 1 à 3, destiné à être utilisé dans le traitement des troubles respiratoires et non respiratoires, incluant la BPCO, l'asthme, l'ALI, le SDRA, la fibrose, l'asthme chronique et l'asthme aigüe, les maladies pulmonaires suite à des expositions environnementales, une infection pulmonaire aiguë, une infection pulmonaire chronique, une maladie associée à l'α1-antitrypsine, une fibrose kystique, les maladies auto-immunes, une néphropathie diabétique, une maladie rénale chronique, une lésion rénale aiguë induite par une septicémie, une lésion rénale aiguë (AKI), une maladie rénale ou un dysfonctionnement des reins constaté(e) pendant une transplantation rénale, l'hypertension artérielle pulmonaire, l'athérosclérose, l'hypertension, l'insuffisance cardiaque, le syndrome coronarien aigu, l'infarctus du myocarde, une réparation du myocarde, un remodelage cardiaque, les arythmies cardiaques, la maladie de Parkinson (PD), la maladie d'Alzheimer (MA), l'ataxie de Friedreich (FA), la sclérose latérale amyotrophique (SLA), la sclérose en plaques (SEP), une maladie inflammatoire de l'intestin, le cancer du côlon, la DMLA néovasculaire (sèche) et la DMLA néovasculaire (humide), une lésion oculaire, la dystrophie cornéenne endothéliale de Fuchs (FECD), l'uvéite ou d'autres affections oculaires inflammatoires, la stéatohépatite non alcoolique (NASH), une maladie hépatique induite par une toxine (par exemple, une maladie hépatique induite par l'acétaminophène), une hépatite virale, une cirrhose, le psoriasis, une dermatite/les effets topiques d'un rayonnement, une immunosuppression due à une exposition à un rayonnement, une pré-éclampsie, et le mal de la haute altitude.

7. Composé à utiliser selon la revendication 6, dans lequel la maladie est la BPCO.

8. Composé à utiliser selon la revendication 6, dans lequel la maladie est une insuffisance cardiaque.

9. Composé à utiliser selon les revendications 6 à 8, dans lequel le composé est administré par voie orale.

10. Composé à utiliser selon les revendications 6 à 8, dans lequel le composé est administré par voie intraveineuse.

11. Composé à utiliser selon les revendications 6 à 8, dans lequel le composé est administré par inhalation.
